# EUROPEAN PATENT APPLICATION

(11) **EP 4 303 325 A1**
(43) Date of publication of application: **10.01.2024**
(21) Application number: 22183020.1
(22) Date of filing: 05.07.2022
(51) Int. Cl.: C12Q 1/689

(54) **METHOD FOR DETERMINING BACTERIAL METABOLITES FOR INDIVIDUALIZED NUTRITIONAL ADJUSTMENT**

(71) Applicant: Max-Delbrück-Centrum für Molekulare Medizin in der Helmholtz-Gemeinschaft, 13125 Berlin (DE); Charité - Universitätsmedizin Berlin, 10117 Berlin (DE)
(72) Inventor: McParland, Victoria, 13158 Berlin (DE); Wilck, Nicola, 13125 Berlin (DE); Müller, Dominik, 10405 Berlin (DE)
(74) Representative: Hertin und Partner Rechts- und Patentanwälte PartG mbB

(57) **Abstract**

The invention relates to a method for determining a production capacity of multiple bacterial metabolites in a subject by quantifying an expression level and an abundance of each of multiple bacterial genes, said genes encoding enzymes associated with the production of said bacterial metabolites. The invention further relates to a kit for quantifying an expression level and an abundancy of each of multiple bacterial genes associated with the production of multiple bacterial metabolites. The invention further relates to oligonucleotides, such as primers and probes, suitable for the inventive method and/or kit, and to sets of corresponding primers and probes.

## Description

The invention is in the field of molecular detection, quantification and/or diagnostics, in particular detection and quantification of bacterial genes. The invention further relates to the field of nutritional adjustments and/or treatment based on the detection and quantification of bacterial genes and metabolites.

The invention relates to a method for determining a production capacity of multiple bacterial metabolites in a subject by quantifying an expression level and an abundance of each of multiple bacterial genes, said genes encoding enzymes associated with the production of said bacterial metabolites, comprising performing a quantitative polymerase chain reaction (qPCR) on a sample comprising commensal bacteria obtained from the subject, with primers that hybridize to nucleic acid molecules encoding said bacterial genes, and determining an abundancy of said bacterial genes, and performing a quantitative reverse transcription polymerase chain reaction (qRT-PCR) on said sample, with primers that hybridize to said nucleic acid molecules encoding said bacterial genes, and determining expression levels of said bacterial genes.

The invention further relates to a kit for quantifying an expression level and an abundancy of each of multiple bacterial genes associated with the production of multiple bacterial metabolites, comprising preferably degenerate primers that hybridize with nucleic acid molecules encoding said bacterial genes associated with the production of said bacterial metabolites, probes that hybridize with said nucleic acid molecules encoding said bacterial genes, standards for said bacterial genes, for example for generating one or more copy number standard curves, and optionally primers that hybridize with a nucleic acid molecule encoding a bacterial 16s RNA sequence.

The invention further relates to oligonucleotides, such as primers and/or probes, suitable for the inventive method and/or kit, or to sets of corresponding primers and/or probes.

### BACKGROUND OF THE INVENTION

The composition and function of the microbiome of a human is unique and depends on the microenvironment of said microbiome, whereby genetics, age, nutrition, medication, lifestyle, living environment and circadian rhythm can play a role. An imbalance of commensal bacteria of the microbiome (microbiota) is termed dysbiosis and can include changes in the functional composition, in metabolic activity and a shift in the local distribution of the microbiota.

It has been shown that dysbiosis, especially dysbiosis of the gut microbiome, termed intestinal dysbiosis, alters the metabolism and production of microbiota-derived metabolites (bacterial metabolites). Bacterial metabolites are resorbed across the intestinal barrier into host circulation where they can have profound impact on human physiology and especially host immunity and inflammation. Bacterial production of these key metabolites is dependent on the presence of certain dietary precursors further underlying the importance of human nutrition. Some of these metabolites enhance inflammatory processes and accelerate chronic and/or inflammatory diseases and are thus associated with the development, onset, progression, and complications of chronic and/or inflammatory diseases. Whereas other bacterially produced metabolites are beneficial to the host and contribute to chronic disease prevention. Inflammatory and/or chronic diseases for which a correlation to intestinal dysbiosis has been identified to date include e.g., cardiovascular disease (CVD), chronic kidney disease (CKD), inflammatory bowel diseases (IBD) such as Crohn's disease or ulcerative colitis, obesity, diabetes type 2, autoimmune diseases including rheumatic diseases, multiple sclerosis, diabetes type 1, allergic asthma, cancer, and mental health disorders.

Chronic kidney disease (CKD) is a major cause of premature morbidity and mortality, with approx. 10-13% of the worldwide adult population having signs of CKD *(Hill et al.,* 2016). The incidence of CKD has increased 89% and associated deaths have doubled from 1990 to 2016 (Jha *etal.,* 2018). Thus, CKD is a global health problem with a high economic burden for the health systems. The increase in incidence is associated with an increasing age of the population and with the increase in non-communicable diseases such as obesity, diabetes and high blood pressure, all of which cause significant burden on the kidneys.

CKD patients have an increased risk of cardiovascular events (*e.g.*, non-fatal myocardial infarction, stroke, cardiovascular death) which increases with kidney disease progression and is only partially resolved after kidney transplantation. It is estimated that cardiovascular events account for more than 50% of deaths in patients suffering from CKD (Pfann, 2020). The underlying mechanisms responsible for the increased cardiovascular risk are not entirely clear. Besides known risk factors such as arterial hypertension and proteinuria, recent studies suggest enhanced immune activation and chronic inflammation in part mediated through pathological changes in the function, composition and metabolite production of the intestinal microbiome, also play an important role (Schaefer *et al.,* 2017; Claro *et al.,* 2018, Holle *et al.,* 2019, Velasquez et *al.,* 2018; Vanholder *et al.,* 2014; Castillo-Rodriguez *et al.,* 2018). Thus, treatments targeting the microbial composition and metabolite production in CKD patients, that are known to be influenced by the patient's diet and can be modulated by nutritional intervention, could reduce the associated cardiovascular risk in this vulnerable and difficult to treat patient group.

Nutritional therapy is an important component of CKD treatment. However general dietary recommendations are often ineffective, partly due to low adherence rates of the patients varying between 20% and 70% (Beto *et al.,* 2016). Personalized dietary recommendations based on the composition and function of a patient's individual gut microbiome would be more effective as it can individually alter microbial composition and metabolite production, and also improve patient adherence. Microbiome-targeted personalized nutrition has been shown to more effectively manage glycemic response to food and algorithms that use parameters such as microbiome composition to predict an individual's physiological response to particular foods/nutrients have been effective in research settings (Zeevi *etal.,* 2015; Zhao *etal.,* 2018). However, such strategies have not yet been translated into clinical practice, due to the complexities of the prediction algorithms that require multiple patient input parameters. A simpler microbiome-based diagnostic tool which does not require expensive specialized equipment and complex interpretation would enable translation of personalized nutritional approaches into clinical practice, this is however not yet available.

Although the relationship between microbial dysbiosis, nutrition and the progression of CKD and other chronic diseases is well described in the art, it is not yet fully understood (Bartolomaeus *et al*., 2019; Schlender *et al.,* 2021). Furthermore, personalized nutritional approaches that take into account the composition, function and metabolite production of the gut microbiome by customizing the intake of the metabolite's dietary precursors have great potential and could be more effective than standard nutritional guidelines in treating and preventing the progression of chronic disease, especially when systemic inflammation is driving disease progression.

Despite the rapid advances and the potential for microbiome based personalized nutrition as an integrated component of medical treatments, individualized nutrition has not yet been translated into clinical practice. One reason for this is that there are no established and reliable tests or analytical methods that can be applied in a clinical setting to study a patient's gut microbiome in terms of its composition and associated metabolite production. Such tests and analytic methods would be necessary to help nutritionists and doctors provide individualized nutritional advice for their patients taking into consideration the individual patient's intestinal microbiome and metabolite production capacity thereof.

A standard method to measure metabolite concentrations in patient plasma and urine samples involved highly specialized and expensive mass spectrometry methods. Furthermore, the measured values are highly variable and sensitive to differences in measurement methods, study populations and environmental factors. For example, measurements in plasma or urine vary by +403% to -80% in a test-retest study of the metabolite trimethylamine N-oxide (TMAO) in obese subjects with type 2 diabetes (McEntyre *et al.,* 2015). Blood and urine metabolite measurements are especially complex in CKD. Typically, CKD patients have drastically increased levels of TMAO and the metabolite indoxyl sulphate (IS) in the serum. This is due to CKD-related changes in the gut microbiota but also influenced to a variable and undefinable extent by the kidney disease and impaired renal clearance itself.

Besides measurements of metabolites in the blood such as in serum and/or plasma samples and in the urine the composition and metabolite production in a patient's microbiome can be determined by analysis of the bacterial genome or more specifically the bacterial genes associated with the production of key metabolites. Such methods require extraction of bacterial DNA or RNA from patient samples, *e.g.*, stool samples. In contrast to measurements in blood and urine samples, these measurements of microbiome function are not influenced by the above mentioned methodological or environmental factors that impair serum measurements.

Analysis of bacterial DNA by nucleic acid amplification techniques such as quantitative polymerase chain reaction (qPCR) and next generation sequencing (NGS) including genome shotgun sequencing or amplicon 16s sequencing techniques provide information on the abundancy of bacterial genes and the composition of the gut microbiome (taxonomy). Analysis of bacterial DNA by qPCR is disclosed in Daskova et *al*., 2021. The publication by Daskova *et al.* describes the development of a qPCR-based assay to detect the butyryl-CoA transferase gene in bacterial DNA extracted from human stool samples and using a spike-in DNA from C. *elegans* as standard to quantify gene abundancies. Although useful for potentially guiding microbiome based personalized nutritional strategies this study does not provide a full picture of the expression levels of the metabolite-associated genes, a parameter that is especially important, being more dynamic and responsive to dietary adjustments. Such gene expression analyses require analysis of RNA levels by whole transcriptome shot gun sequencing followed by meta transcriptome analysis as for example disclosed in WO2020051559A1 and Schirmer *et al.*

In general, the analysis of mRNA levels allows the detection of gene expression and thus metabolically active cells with clear advantages over an analysis of DNA. Analyses of mRNA provides an extremely useful snapshot of health, disease, disease progression and even disease risk. In the context of the diet-gut microbiome-disease axis, commensal bacterial mRNA analyses to report on the current metabolite production of a person's microbiome is an important readout for the constantly changing microbiome during the course of a disease. Thus, monitoring mRNA levels in the microbiome as a snapshot proxy for metabolite expression is a useful diagnostic tool to guide individualized nutritional therapy and also to monitor if a patient is having the desired response to their prescribed nutrition. DNA, on the other hand, provides information on the presence of bacterial genes with no information on the activity or expression of the said gene. Furthermore, abundancies of genes at the DNA level do not or only marginally change during disease development and progression and have been shown to contribute much less to chronic diseases than changes in gene expression.

Prior art techniques including sequencing technologies to analyze bacterial gene expression are typically expensive, require extensive specialist and bioinformatic expertise to interpret the results and are therefore not suitable for general or bed-side use in a clinical setting.

In view of the medical need and significant potential of individualized nutritional treatments for the prevention and/or treatment of chronic and/or inflammatory disease, a straightforward, quick and reliable method not requiring specialized equipment and/or knowledge is urgently needed to enable an effective analysis of a patient's microbiome composition and metabolite production. Novel approaches suitable for a clinical setting to provide individualized nutritional adjustment and/or treatment are therefore still needed.

### SUMMARY OF THE INVENTION

In light of the prior art the technical problem underlying the present invention is to provide alternative or improved means for determining the production capacity of multiple bacterial metabolites in a subject by detection and quantification of the abundance and expression of multiple bacterial genes that overcome the disadvantages of the prior art.

A further object of the present invention is to provide simple, quick, and reliable methods to determine the production capacity of multiple bacterial metabolites that do not require specialized equipment and/or expertise and are applicable in a clinical setting using standard equipment.

A further object of the present invention is to provide means for determining the production capacity of multiple bacterial metabolites in a subject to provide individualized nutritional adjustment and/or treatment of the subject. Another problem underlying the invention is to provide means for monitoring a subject's response to individualized nutritional adjustment and monitor and/or adjust the individual nutrition by repetitively determining the production capacity of multiple bacterial metabolites.

This problem is solved by the features of the independent claims. Preferred embodiments of the present invention are provided by the dependent claims.

In one aspect the invention relates to a method for determining a production capacity of multiple bacterial metabolites in a subject by quantifying an expression level and an abundance of each of multiple bacterial genes, said genes encoding enzymes associated with the production of said bacterial metabolites, comprising:
- performing a quantitative polymerase chain reaction (qPCR) on a sample comprising commensal bacteria obtained from the subject, with primers that hybridize to nucleic acid molecules encoding said bacterial genes, and determining an abundancy of said bacterial genes, and
- performing a quantitative reverse transcription polymerase chain reaction (qRT-PCR) on said sample, with primers that hybridize to said nucleic acid molecules encoding said bacterial genes, and determining expression levels of said bacterial genes.

Surprisingly, the method in the present invention provides a simple, rapid, and robust approach for determining the abundancy and expression levels of bacterial genes associated with the production of bacterial metabolites. By analyzing both abundancy and gene expression, the method provides information on both the composition and function of the microbiome and thus a current metabolite production capacity of the microbiome.

It has been shown that the composition and function of a subject's microbiome may play a significant role in the development and progression of chronic disease, and dysbiosis is associated with a plethora of chronic diseases. However, methods known in the prior art either provide information solely on the abundancy of bacterial genes and/or require expensive equipment and specialized expertise and are thus not applicable in a bedside or point of care clinical setting. In contrast, the method of the present invention can be performed with standard equipment present in clinical and research labs and provides robust information on both abundancy and expression of bacterial genes and thus metabolite production capacity, of a subject's microbiome. Furthermore, multiple genes are analyzed using the method of the present invention, which may therefore be considered in embodiments to be a medium- to high-throughput method.

In embodiments of the invention, the method is used to indicate an individualized nutritional adjustment for and/or therapy of the subject. In embodiments, the method is used to indicate an individualized nutritional adjustment for and/or therapy of the subject when the determined expression and/or abundance levels deviate from a control value, for example a healthy subject and/or a population average.

In embodiments of the invention deviation of gene expression and/or abundance from a control value indicates the presence and/or risk of a medical condition, for example an inflammatory and/or chronic disease, such as a cardiovascular disease including atherosclerosis, arterial hypertension, peripheral arterial occlusive disease (pAVK) and coronary heart disease (CHD), a chronic kidney disease, irritable bowel syndrome, an inflammatory bowel disease including Crohn's disease or ulcerative colitis, obesity, insulin resistance, obesity, diabetes type 2, an autoimmune disease including a rheumatic disease, multiple sclerosis and diabetes type 1, allergic asthma, non-alcoholic fatty liver disease (NAFLD/NASH), cancer, a mental health disorder including Alzheimer's disease, dementia, Parkinson, autism spectrum disorders, generalized anxiety disorder, depression and posttraumatic stress disorder (PTSD), and/or cancer and/or a skin disease including eczema and psoriasis.

In embodiments of the invention the indicated nutritional adjustment comprises an adjustment in diet and/or administration of commensal bacteria to the subject.

Individualized nutrition that takes into consideration the composition and function and thus metabolite production capacity of the microbiome has been shown to be more effective in the prevention and therapy of chronic and/or inflammatory diseases than standard nutritional guidelines, particularly in cases where low grade systemic inflammation is driving disease progression. However, individualized nutrition has not yet been translated to clinical practice, due to a lack of diagnostic and monitoring tools of metabolite production capacity of the microbiome that are applicable in a clinical setting.

The method of the present invention provides a simple and reliable tool for analyzing and monitoring the metabolite production capacity of a subject's microbiome in a clinical setting and thus provides relevant diagnostic information for individualized adjustments of a subject's diet. Thereby a deviation of gene expression and/or abundance from a control value indicates that the current nutritional intake of the subject is not the most appropriate diet for their personal health. Identification of this and appropriate individualized dietary adjustments help to prevent inflammation driven chronic diseases.

In embodiments, the gene expression of a bacterial gene associated with the production of a bacterial metabolite provides information on the quality and impact of a subject's nutrition such as information on a nutritional deficit in a subject's current diet and resulting prospective risk for development and progression of a chronic and/or inflammatory disease. Therefore, the expression of the bacterial gene by itself and/or in combination with its abundancy can be used as a biomarker for monitoring the effect and impact of a subject's nutrition for prevention and/or treatment of chronic and/or inflammatory diseases.

For example, an increase in the dietary sources of metabolite precursors can be advised if a deficiency of metabolite production is revealed and likewise a reduced intake of a dietary source can be advised if overproduction of a metabolite is revealed. The method of the present invention provides not only a tool for analyzing the metabolite production capacity of a subject's microbiome but also provides means for monitoring the production capacity over time, such as the course of a chronic disease and allows constant individual adjustments of a subject's diet and intake of nutrients.

In embodiments of the invention the sample is, without limitation, a stool sample, fecal water, a biopsy sample (*e.g.*, of the gastrointestinal tract and/or skin), interstitial fluid sample, mucosa sample of an oral, respiratory or intestinal compartment, a mucosal swab and/or skin swab of the subject.

Surprisingly, various sample types are suitable for use within the method of the present invention. Thereby, DNA and RNA can be extracted from samples, comprising commensal bacteria, from a subject by using suitable methods known to a person skilled in the art. Independent of the sample type, the extracted DNA and RNA can be analyzed by the method of the present invention in order to determine the abundance and expression level of a bacterial gene associated with the production of a bacterial metabolite.

In embodiments of the invention, the method includes preparing a sample and isolating nucleic acids from the sample.

In embodiments the sample is a biopsy sample, comprising tissue of a subject.

In embodiments preparing the sample and isolation of nucleic acids comprises lysing tissue, cells, and/or cellular compartments in the sample by mechanical, thermal, chemical and/or enzymatic methods to release nucleic acids from the sample, and/or methods to remove non-nucleic acid material from the sample to isolate the nucleic acids.

In embodiments of the invention mechanical methods of lysing tissue, cells and/or cellular compartments include homogenizing, grinding, ultrasonicating and/or freezing of the sample.

In embodiments of the invention chemical methods of lysing tissue, cells and/or cellular compartments alkaline lysis, detergent lysis, solvent lysis, use of a chaotropic agent such as guanidinium isothiocyanate, guanidinium chloride, urea, thiourea, lithium perchlorate, lithium acetate, sodium iodide, phenol and others.

In embodiments of the invention enzymatic methods of lysing tissue, cells and/or cellular compartments include treatment of the sample with protease, lipase, glycoside hydrolases, proteinase K, keratinase, trypsin, subtilisin, lyticase, lysozyme, collagenase, cellulase, glucanase, chitinase, pectinase, and/or amylase.

In embodiments of the invention thermal methods of lysing tissue, cells and/or cellular compartments include subjecting the sample to a temperature of more than or equal to 50° C or more, preferably 60° to 100 °C.

In embodiments of the invention methods to non-nucleic-acid material from the sample include extraction, precipitation and/or precipitation by absorption to ta DNA- or RNA-binding matrix such as columns and magnetic beads followed by centrifugation.

In embodiments methods to remove impurities by an extraction step includes the use of phenol, chloroform and or mixtures thereof (phenol/chloroform extraction), Triton X-100 and/or Trizol.

In embodiments of the invention the method comprises additionally a quantitative nucleic acid amplification reaction of a standard for gene expression and/or for gene abundancy. In embodiments, the standard comprises, without limitation, amplification of a gene specific copy number standard and/or amplifying a 16s ribosomal RNA (16s rRNA) sequence as a quantitative control for bacterial gene abundancy.

In embodiments, for accurate measurement and absolute quantification of gene expression, copy number standard curves for each specific bacterial gene are established, either prior to or as part of the method of the present invention. In embodiments, for normalization of abundance of a gene, bacterial 16s rRNA gene is amplified. In contrast to relative quantification methods, the use of a copy number standard curve provides absolute values of the number of amplicons generated during quantitative PCR and thus exact information on the expression of a bacterial gene in a sample from the subject. The use of 16s rRNA as an internal control further allows normalization of gene abundance by normalizing the amplicon copy number to the total amount of DNA per sample, thereby taking variations in sample preparation and/or loading into account.

In embodiments of the invention the primers are degenerate and can hybridize to nucleic acid molecules from multiple bacteria with distinct coding sequences for the bacterial gene encoding an enzyme associated with the production of said bacterial metabolite.

Genes encoding for enzymes contributing to the production of bacterial metabolites can be present in several bacterial species within the microbiome of a subject. The use of degenerate primers in the context of the present invention allows detection and quantification of the expression and abundancy of specific bacterial genes across the entire microbiome independently of the source bacterial species, thus providing an efficient approach for determining overall expression of a bacterial gene in a subject's microbiome.

In embodiments of the invention the bacterial metabolite is a short chain fatty acid (SCFA) or indole derivative.

In embodiments of the invention the bacterial metabolite is butyrate, propionate, trimethylamine-N-oxide (TMAO) and/or indoxyl sulphate (IS).

In embodiments of the invention the bacterial gene comprises, *e.g.*, butyrate-CoA-dehydrogenase *(Bcd),* methylmalonyl-CoA-mutase (spcA), choline trimethylamine lyase *(cutC),* carnitine monooxygenase *(cntA),* and/or tryptophanase enzyme *(tnaA).*

In further embodiments of the invention the bacterial gene comprises, *e.g.*, bile acid inducible operon *(baiCD)* and/or sulphite reductase *(dsrA).*

As described in more detail below, SCFA as a class of compounds provides a suitable target for determining microbiome composition and/or function. A skilled person is capable of determining suitable SCFA molecules for interrogation via the method of the invention. Surprisingly, determining the presence and/or expression levels of genes associated with production of SCFA represents an improvement over techniques that determine SCFA levels directly, which can be plagued by the disadvantages mentioned above. As also shown in more detail below, by way of example, the specific metabolites and the genes associated with their production surprisingly enable accurate determination of microbial function and composition in any given sample and enable accurate and therapeutically relevant statements regarding potential nutritional adjustment and/or other medical treatments.

In embodiments, the method of the invention may be characterized by the particular primer, probe or target sequences of the amplification method described herein. The particular sequences disclosed herein are intended as examples of preferred embodiments and may be elected and potentially adjusted in order to perform the method of the invention.

In embodiments of the invention, the primers hybridize with a region of a bacterial butyrate-CoA-dehydrogenase gene *(Bcd)* according to SEQ ID NO 1 to 7, or to sequences with an 80%, preferably 90% identity thereto.

As used herein, any designation of multiple sequences relates to any one of those sequences within the designates SEQ ID NOs. For example, reference to SEQ ID NO 1 to 7 relates to any one or more of SEQ ID NO 1 to 7, such as for example SEQ ID NO 1, 2, 3, 4, 5, 6 and/or 7.

In embodiments of the invention, the primers hybridize with a region of a bacterial butyrate-CoA-dehydrogenase gene *(Bcd)* according to SEQ ID NO 1 to 7, wherein the primers may be according to SEQ ID NO 8 to 13, or sequences with an 80%, preferably 90% identity thereto. In embodiments, the method may optionally comprise a probe according to SEQ ID NO 14 to 16 or sequences with an 80%, preferably 90% identity thereto.

In embodiments of the invention the primers hybridize with a region of a methyl-malonyl-CoA-mutase *(scpA)* gene according to SEQ ID NO 17 to 23 and/or 26 to 28, or to sequences with an 80%, preferably 90% identity thereto.

In embodiments of the invention, where the primers hybridize with a region of a methyl-malonyl-CoA-mutase *(scpA)* gene according to SEQ ID NO 17 to 23 and/or 26 to 28, and/or the method may comprise primers according to SEQ ID NO 24, 25, 29 and/or 30 and/or SEQ ID NO 31, or sequences with an 80%, preferably 90% identity thereto, and optionally the method may comprise a probe according to SEQ ID NO 31 or sequences with an 80%, preferably 90% identity thereto.

In embodiments of the invention the primers hybridize with a region of a bacterial choline trimethylamine lyase *(cutC)* gene according to SEQ ID NO 32, 35 to 39 and/or 51 to 54, or to sequences with an 80%, preferably 90% identity thereto.

In embodiments of the invention, where the primers hybridize with a region of a bacterial choline trimethylamine lyase *(cutC)* gene according to SEQ ID NO 32, 35 to 39 and/or 51 to 54, and/or the method comprises primers according to SEQ ID NO 33, 34, 40 to 50, and/or 55 to 61 or sequences with an 80%, preferably 90% identity thereto.

In embodiments of the invention the primers hybridize with a region of a bacterial carnitine monooxygenase *(cntA)* according to SEQ ID NO 62 to 64, or to sequences with an 80%, preferably 90% identity thereto.

In embodiments of the invention, where the primers hybridize with a region of a bacterial carnitine monooxygenase *(cntA)* gene according to SEQ ID NO 62 to 64, and/or the method comprises primers according to SEQ ID NO 65 and/or 66 or sequences with an 80%, preferably 90% identity thereto.

In embodiments of the invention the primers hybridize with a region of a bacterial tryptophanase enzyme *(tnaA)* gene according to SEQ ID NO 67, 68 and/or 71 to 77, or to sequences with an 80%, preferably 90% identity thereto.

In embodiments of the invention, where the primers hybridize with a region of a bacterial tryptophanase enzyme *(tnaA)* gene according to SEQ ID NO 67, 68 and/or 71 to 77, and/or the method comprises primers according to SEQ ID NO 69, 70, 78 and/or 79, or sequences with an 80%, preferably 90% identity thereto.

In embodiments of the invention the primers hybridize with a region of a bile acid inducible operon *(baiCD)* gene according to SEQ ID NO 91, or to sequences with an 80%, preferably 90% identity thereto. In embodiments, the method comprises primers according to SEQ ID NO 92 and 93 or sequences with an 80%, preferably 90% identity thereto.

In embodiments of the invention the primers hybridize with a region of a sulphite reductase *(dsrA)* gene according to SEQ ID NO 96, or to sequences with an 80%, preferably 90% identity thereto. In embodiments, the method comprises primers according to SEQ ID NO 97 and 98, or sequences with an 80%, preferably 90% identity thereto.

There is evidence that several bacterial metabolites play a significant role in the immune system and inflammatory status of a subject and are related to the development and progression of chronic and/or inflammatory diseases. Furthermore, the production of bacterial metabolites is determined by the diet of the subject making personalized microbiome nutrition actionable via individualized nutritional adjustments targeting of microbial metabolite production. Central metabolites associated with inflammation and the development and/or progression of chronic and/or inflammatory diseases include short chain fatty acids (SCFA) such as butyrate and propionate, indole derivatives such as indoxyl sulphate (IS) and trimethylamine-N-oxide (TMAO) and its precursor trimethylamine (TMA).

By using degenerate primers and optionally probes specifically designed for genes encoding enzymes associated with the production of these metabolites, the expression of these genes can be accurately determined, quantified, and monitored overtime, and thus in embodiments indicating required individual nutritional changes within a subject to optimize metabolite production.

For example, in embodiments, overexpression of *cutC* or *cntA* resulting in high TMA and TMAO production in a subject indicates dietary sources of choline (found in eggs, meat and soya) or carnitine (found in meat) should be reduced, respectively. Both approaches reduce microbiota TMA production and thus TMAO production in a targeted manner.

In embodiments, overexpression of *tnaA* resulting in high IS production can be targeted by restricting dietary sources of tryptophan to reduce IS production. In subjects with low *Bed* and/or *spcA* expression, resulting in low butyrate or propionate production respectively, dietary sources of indigestible carbohydrates (*e.g.*, resistant starch) can be increased to effectively boost butyrate and/or propionate production.

In one aspect, the present invention relates to a kit for quantifying an expression level and an abundancy of one or more bacterial genes associated with the production of one or more bacterial metabolites, comprising one or more degenerate primers that hybridize to a nucleic acid molecule encoding a bacterial gene associated with the production of a bacterial metabolite, one or more standards for said bacterial gene, for example for generating one or more copy number standard curves, optionally one or more probes that hybridize to said nucleic acid molecule encoding said bacterial genes, and optionally primers that hybridize with a nucleic acid molecule encoding a bacterial 16s rRNA sequence.

In one aspect, the kit, comprises optionally one or more reagents for preparing a sample and isolating nucleic acids from the sample, and optionally one or more reagents for performing a quantitative polymerase chain reaction (qPCR) and/or quantitative reverse transcription polymerase chain reaction (qRT-PCR).

In one aspect, the present invention relates to a set of oligonucleotides, for example suitable in a method and/or kit of the invention.

In one embodiment, the set of oligonucleotides comprises primers to detect a bacterial butyrate-CoA-dehydrogenase gene *(Bcd)* according to SEQ ID NO 1 to 7, wherein the primers comprise or consist of a sequence according to SEQ ID NO 8 to 13, or sequences with an 80%, preferably 90% identity thereto.

In one embodiment, the set of oligonucleotides comprises primers to detect a methyl-malonyl-CoA-mutase *(scpA)* gene according to SEQ ID NO 17 to 23 and/or 26 to 28, wherein the primers comprise or consist of a sequence according to SEQ ID NO 24, 25, 29 and/or 30 and/or SEQ ID NO 31, or sequences with an 80%, preferably 90% identity thereto.

In one embodiment, the set of oligonucleotides comprises primers to detect a bacterial choline trimethylamine lyase *(cutC)* gene according to SEQ ID NO 32, 35 to 39 and/or 51 to 54, wherein the primers comprise or consist of a sequence according to SEQ ID NO 33, 34, 40 to 50, and/or 55 to 61 or sequences with an 80%, preferably 90% identity thereto.

In one embodiment, the set of oligonucleotides comprises primers to detect a bacterial carnitine monooxygenase *(cntA)* gene according to SEQ ID NO 62 to 64, wherein the primers comprise or consist of a sequence according to SEQ ID NO 65 and/or 66 or sequences with an 80%, preferably 90% identity thereto.

In one embodiment, the set of oligonucleotides comprises primers to detect a bacterial tryptophanase enzyme (taA) gene according to SEQ ID NO 67, 68 and/or 71 to 77, wherein the primers comprise or consist of a sequence according to SEQ ID NO 69, 70, 78 and/or 79, or sequences with an 80%, preferably 90% identity thereto.

In one embodiment, the set of oligonucleotides comprises primers to detect a bile acid inducible operon *(baiCD)* gene according to SEQ ID NO 91, wherein the primers comprise or consist of a sequence according to SEQ ID NO 92 and 93 or sequences with an 80%, preferably 90% identity thereto.

In one embodiment, the set of oligonucleotides comprises primers to detect a sulphite reductase *(dsrA)* gene according to SEQ ID NO 96, wherein the primers comprise or consist of a sequence according to SEQ ID NO 97 and 98, or sequences with an 80%, preferably 90% identity thereto.

In one embodiment, the set of oligonucleotides comprises primers, as described herein, to detect one or more, or preferably two or more, or preferably all genes selected from the group consisting of butyrate-CoA-dehydrogenase *(Bcd),* methylmalonyl-CoA-mutase *(spcA),* choline trimethylamine lyase *(cutC),* carnitine monooxygenase *(cntA)* and tryptophanase enzyme *(tnaA).*

In one embodiment, the set of oligonucleotides comprises probes suitable to detect one or more, or preferably two or more, or preferably all genes selected from the group consisting of butyrate-CoA-dehydrogenase *(Bcd),* methylmalonyl-CoA-mutase *(spcA),* choline trimethylamine lyase *(cutC),* carnitine monooxygenase *(cntA)* and tryptophanase enzyme *(tnaA).*

In one embodiment, the set of oligonucleotides comprises probes suitable to detect one or more, or preferably two or more, or preferably all genes selected from the group consisting of bile acid inducible operon *(baiCD)* and/or sulphite reductase *(dsrA).*

In embodiments, the probes are suitable to hybridize to one or more of:
a region of a bacterial butyrate-CoA-dehydrogenase gene *(Bcd)* according to SEQ ID NO 1 to 7, or to sequences with an 80%, preferably 90% identity thereto,
a region of a methyl-malonyl-CoA-mutase *(scpA)* gene according to SEQ ID NO 17 to 23 and/or 26 to 28, or to sequences with an 80%, preferably 90% identity thereto,
a region of a bacterial choline trimethylamine lyase *(cutC)* gene according to SEQ ID NO 32, 35 to 39 and/or 51 to 54, or to sequences with an 80%, preferably 90% identity thereto,
a region of a bacterial carnitine monooxygenase *(cntA)* according to SEQ ID NO 62 to 64, or to sequences with an 80%, preferably 90% identity thereto, and/or
a region of a bacterial tryptophanase enzyme *(tnaA)* gene according to SEQ ID NO 67, 68 and/or 71 to 77, or to sequences with an 80%, preferably 90% identity thereto.
a region of a bacterial bile acid inducible operon (baiCD) gene according to SEQ ID NO 91 or to sequences with an 80%, preferably 90% identity thereto
a region of a bacterial sulfite reductase (dsrA) gene according to SEQ ID NO 96 or to sequences with an 80%, preferably 90% identity thereto

In embodiments of the invention, either in the context of the method, kit and/or set of oligonucleotides, the degenerated primers, probes and/or standards are provided in a dried form within a multi-well plate, preferably a 96-, 192 or 384-well plate.

Surprisingly, the components of the kit can be applied to 96- or 384-well plates, dried, sealed and stored. The DNA or RNA isolated from a subject can then be added directly to the plate with the appropriate PCR reagents and excipients to determine the subjects' bacterial abundance and current gene expression profile. Sensitivity and robustness of the measurements are not affected by the drying and storage process. This makes the method and kit medium- to high-throughput and ideal for use in everyday clinical practice.

Embodiments and features of the invention described with respect to the method, the oligonucleotides, including e.g., primers, probes and standards, and kits, are considered to be disclosed with respect to each and every other aspect of the disclosure, such that features characterizing the methods, may be employed to characterize the oligonucleotides, or kit, and vice-versa.

### DETAILED DESCRIPTION OF THE INVENTION

All cited documents of the patent and non-patent literature are hereby incorporated by reference in their entirety.

A "microbiome", also termed "microflora", is the set or a subset of microorganisms associated with a macro-organism, such as a human, also termed "host" or "human host". Thereby the microbiome is a community of commensal and potentially pathogenic microorganisms that are associated with the organism and are termed "microbiota". In a human, the microbiota mainly colonizes the intestine and the skin, but also the mouth, nasopharynx, the lungs and the urogenital tract.

The intestinal microbiome, also termed "gut microbiome", mainly comprises bacteria, which predominantly, but without limitation, belong to the groups of *Bacteroidetes* including the genera Bacteroides, Prevotella and Xylanibacter, *Firmicutes* including the genera Clostridium, Eubacterium, Faecalibacterium, Lactobacillus, Roseburia and Ruminococcus, *Actinobacteria* including the genera Bifidobacterium, *Proteobacteria* including the genera Desulfovibrio and Escherichia and *Verrucomicrobia* including the genus Akkermansia.

As is commonly understood, the intestinal microbiome establishes itself within the first years of life, whereby the colonization density is overall increasing with higher age. The composition is individual for each human and is influenced by several factors including genetics, age, nutrition, medication, lifestyle, living environment and circadian rhythm. The microbiome influences various processes of the human body, including the immune system, inflammatory processes, and metabolism. Furthermore, the microbiome has an impact on a subject's susceptibility to infection, the response to vaccination and can influence the therapy effectiveness of drugs.

The term "commensal bacteria" refers to bacteria of the human microbiota, that live in the human as host organism in a non-harmful and/or beneficial coexistence, and preferably feed on the food residues of the host organism without harming it. An example of commensal bacteria of the human intestinal microbiome is the genus Bacteroides. The host provides the living environment (intestinal surface, temperature, pH, food, etc.) for these bacteria. Commensal bacteria are typically present in large numbers in a human host.

The term "pathogenic bacteria" refers to bacteria which are harmful for the human host and can cause infectious diseases, damage of host cells and tissues or interference with their function. The pathogenic bacteria can damage host cells directly or indirectly by provoking an immune response that inadvertently damages host cells, or by releasing endotoxins and/or exotoxins. Endotoxins are the lipid portions of lipopolysaccharides that are part of the outer membrane of the cell wall of gram-negative bacteria. Exotoxins may be secreted into the surrounding medium or released when the bacteria are lysed, and the cell wall breaks apart.

An imbalance of the microbiome is termed dysbiosis and can include changes in the functional composition, in metabolic activity and a shift in the local distribution of the microbiota. A dysbiosis of the microbiome can alter the metabolism and production of microbiota-derived metabolites (bacterial metabolites), which can enhance inflammatory processes and accelerate the development, onset, progression and occurrence of chronic and/or inflammatory diseases in the host, including, without limitation, cardiovascular diseases such as atherosclerosis, arterial hypertension, peripheral arterial occlusive disease (pAVK) and coronary heart disease (CHD), chronic kidney disease, irritable bowel syndrome, inflammatory bowel diseases such as Crohn's disease or ulcerative colitis, obesity, insulin resistance, diabetes type 2, a chronic kidney disease, autoimmune diseases such as rheumatic disease, multiple sclerosis and diabetes type 1, allergic asthma, non-alcoholic fatty liver disease (NAFLD/NASH), cancer, mental health disorders including Alzheimer's disease, dementia, Parkinson, autism spectrum disorders, generalized anxiety disorder, depression and posttraumatic stress disorder (PTSD), obesity, cancer and /or skin diseases such as eczema and psoriasis.

The term "bacterial metabolite" refers to chemical substances that are formed in and/or by a bacterium for example as intermediates or degradation products of precursors, such as dietary precursors in metabolic processes, also termed "biochemical processes", taking place in commensal bacteria. A precursor is a substance from which another substance is formed by a chemical and/or biochemical reaction. A dietary precursor is a substance present in nutrients such as food products, that undergoes a chemical and/or biochemical reaction producing another compound e.g., by metabolic processes taking place in commensal and/or pathogenic bacteria of the gut microbiome. Many bacterial metabolites and the biochemical processes leading to the formation of bacterial metabolites are known in the art and can be found in the literature. Thereby, the biochemical processes resulting in the formation of a certain bacterial metabolite can include several dietary precursors and enzymes encoded by bacterial genes. Bacterial metabolites produced from dietary precursors by commensal bacteria of the gut microbiome include for example short chain fatty acids (SCFA) or salts thereof including butyrate, propionate, trimethylamine-N-oxide (TMAO) and indole derivates including indoxyl sulphate (IS).

Short chain fatty acids (SCFA) are a subgroup of fatty acids with two to six carbon atoms. Due to their low molecular weight, they are liquid and volatile at room temperature. Short chain fatty acids are formed in the intestine from dietary precursors such as indigestible carbohydrates (fibre and digestion-resistant starch), Acetyl-coenzyme A (acetyl-CoA) and pyruvate by the intestinal microbiota and are subsequently absorbed by the gut mucosa. SCFA and salts thereof include acetate (C2), propionate (C3), butyrate (C4), isobutyrate (C4), valerate (C5), isovalerate (C5) and capronate (C6). The microbiota-derived SCFAs, butyrate, acetate and propionate have been extensively studied and shown to directly communicate with host immune cells (Smith *et al.,* 2013) as well as interact with G protein-coupled receptors throughout diverse organs of the body (Brown *et al.,* 2003) and thereby regulate immune cell development and suppress inflammation. The microbial production of propionate responds to dietary changes, whereby increased consumption of indigestible fiber leads to higher plasma concentrations of SCFA (Baxter *et al.,* 2019, Vaziri *et al.,* 2014) and high fat diets reduce the formation of SCFA (Jakobsdottir *et al.,* 2013).

Trimethylamine (TMA) is a bacterial metabolite produced by the intestinal microbiome from its dietary precursors phosphatidylcholine, choline and carnitine, which are found particularly in animal food products such as eggs and meat. Trimethylamine is well absorbed and metabolized in the liver by flavin-containing monooxygenases (FMO3) to trimethylamine N-oxide (TMAO). TMAO levels are directly linked to the development of atherosclerotic heart disease in humans by enhancing the concentration of macrophage-specific cholesterol and the formation of foam cells in the blood vessels (Tang *et al.,* 2014). This is influenced by the diet of a subject since regular consumption of choline- or carnitine-rich food promotes atherosclerosis by increasing bacterial production of the TMAO precursor TMA (Koeth *et al.,* 2013). Dietary habits that increase TMAO also directly contribute to progressive renal fibrosis and elevated levels of plasma TMAO correlate with poorer long-term survival in chronic kidney disease (CKD) (Tang *et al.,* 2015).

Indoxyl sulphate (IS), also termed 3-indoxylsulfate and 3-indoxylsulfiric acid, is a bacterial metabolite derived from its dietary precursor L-tryptophan present in most protein-based food products such as milk, cheese, poultry, red meat, eggs, fish, nuts and peas. IS has been shown to directly interact with the transcription factor aryl hydrocarbon receptor (AhR) in the heart and aorta and to induce a coagulation phenotype which accelerates chronic cardiovascular diseases (CVD) (Dou *et al.,* 2018). Moreover, IS promotes pro-inflammatory macrophage activation and atherosclerosis (Nakano *et al.,* 2019), suggesting a potential mechanism for the deleterious cardiovascular effects observed in CKD. Microbiome production of the indole precursors to IS responds to dietary changes and can be induced with a tryptophan rich diet (Liang *et al.,* 2018).

Generally, TMAO and IS are considered pro-inflammatory, pro-atherosclerotic metabolites and dramatically increased for example in patients with chronic kidney disease (CKD). For example, TMAO has been reported to be >20 fold higher in CKD patients compared to healthy controls (Hai *et al.,* 2015) and plasma IS levels have been shown to increase as CKD progresses from stage 1 to 4 (Lin *et al.,* 2019). In contrast, propionate and butyrate have anti-inflammatory, anti-fibrotic effects, lower blood pressure and may be protective against CVD (Tang *et al.,* 2015; Marzocco et al., 2018; Anders *et al.,* 2013; Bartolomaeus *et al.,* 2019).

As used herein, the term "bacterial gene", refers generally to a region (the transcribed region) of bacterial DNA which encodes a protein, which can be an enzyme. A gene is transcribed into RNA and subsequently translated into a polypeptide forming a protein. A gene may comprise several sections, such as a promoter, a 5'-regulatory sequence, a coding sequence and a 3'-non-translated sequence, comprising e.g., a polyadenylation site.

The phrase "expression of a gene" or "gene expression" refers to the process wherein a gene is transcribed into RNA and/or translated into protein. The term "expression level" thereby relates to the amount of transcribed RNA and/or translated protein. Methods to determine the expression level of genes by measuring the amount of transcribed RNA require isolation of RNA or protein followed by analysis techniques, such as whole transcriptome shot gun sequencing followed by bioinformatic meta transcriptome analysis of RNA (RNA-Seq), Northern-Blot, RNAse protection assay, nuclear run-on assay, DNA-microarrays, serial analysis of gene expression (SAGE), superSAGE, differential display (DDRT-PCR or DD-PCR), qPCR and qRT-PCR. Methods for determination of the amount of translated protein include western-blot, 2D-gel electrophoresis, protein-arrays and mass spectrometric analysis (MS).

The terms "abundance", "gene abundance" and "abundance of a gene" refer to the amount of a bacterial gene in a sample. The abundance provides information about the presence and amount of certain genes and thus bacterial species comprising the gene in a sample. However, the abundance does not inherently provide information about the expression and thus the activity of the genes. The abundance of a gene can be determined by isolation and analysis of genomic DNA (gDNA) in a sample using methods known to a person skilled in the art, including but not limited to nucleic acid amplification techniques such as quantitative polymerase chain reaction (qPCR) and next generation sequencing (NGS) including genome shotgun sequencing or amplicon sequencing techniques.

As used herein, the term "production capacity" of a bacterial metabolite relates to the (preferably quantitative) potential and/or ability and/or capability of production of any given metabolite by the microbiome of a subject. As described herein, the production capacity is preferably based on the determination by quantification of the abundancy and expression levels of a bacterial gene encoding for an enzyme associated with the production of a bacterial metabolite in a sample. The expression level thereby provides information on the present activity of the bacterial gene and thus metabolite production, whereas the abundancy provides information on the amount of bacterial genes in the sample that can potentially be expressed leading to production of the metabolite.

An enzyme is a biochemical catalyst that supports the chemical conversion of a substrate specific to it but is not itself chemically altered. Most enzymes belong to the substance group of proteins, with the exception of ribozymes, which are built from RNA. Enzymes lower the activation energy required for the chemical reaction and thus enable a reaction that would not be possible or would be much slower without them. They specifically bind one or a few substrates by forming a reversible enzyme-substrate complex. In the reaction, the substrate binding site, the active site, is responsible for the specific substrate binding and the catalytic activity. It consists of specially folded parts of the polypeptide chain or reactive non-protein parts of the enzyme. Enzymes can only convert a specific substrate or a few substrates, which is called substrate specificity. Furthermore, an enzyme is only able to catalyze a certain reaction, which is called reaction specificity. A substrate, on the other hand, can be converted by different enzymes.

As used herein, the term "enzyme associated with the production of a bacterial metabolite" relates to an enzyme either directly producing a bacterial metabolite or indirectly enhancing or inhibiting production of a bacterial metabolite. Alternatively, the term "enzyme involved in the production of a bacterial metabolite" may be used. A skilled person is capable, based on the guidance provided herein and on common knowledge in the art, of determining whether any given bacterial gene is associated with the production of a bacterial metabolite. In preferred embodiments, the enzyme associated with the production of a bacterial metabolite directly produces said metabolite.

Several enzymes are involved in any given metabolic pathway leading to formation of bacterial metabolites from dietary precursors as substrates. Bacterial genes involved in the formation of butyrate encode enzymes including butyryl-CoA-acetate-CoA-transferase *(But)* and butyrate-CoA-dehydrogenase *(Bcd).* Bacterial genes involved in the formation of propionate encode enzymes including methylmalonyl-CoA-mutase *(spcA)* and methylmalonyl-CoA-decarboxylase *(mmdA)* (Reichardt *et al.,* 2014; Rath *et al.,* 2017; Devlin *et al.,* 2016). Bacterial genes involved in the formation of TMA encode enzymes including choline trimethylamine lyase *(cutC)* and carnitine monooxygenase *(cntA).* Bacterial genes involved in the formation of IS encode enzymes including tryptophanase enzyme *(tnaA).*

Bacterial genes encoding for enzymes involved in the formation of secondary bile acids from primary bile acids include the bile acid inducible operon *(baiCD).* Bacterial genes encoding for enzymes involved in the sulphate reduction pathway in the formation of the secondary bile acids, deoxycholic acid and lithocholic acid, include sulphite reductase alpha subunit *(dsrA).*

As used herein, the terms "nutrients" and "dietary nutrients" may be used interchangeably and are considered any substance used by an organism to survive, grow, and/or reproduce, that is typically obtained by the subject through their diet. The requirement for dietary nutrient intake is fundamental to life and applies without limitation to animals, plants, fungi, and protists. The diet of a subject refers to the total kind and quantities of nutrients taken in by a subject over a defined period of time e.g., a day, a week, a month or a year, in the form of e.g., food products, supplements and/or probiotics. Nutrients may comprise carbohydrates such as monosaccharides, disaccharides, oligosaccharides and polysaccharides, proteins, fats, water, vitamins, minerals, trace elements, secondary plant substances, derivatives and/or chemical precursors thereof, probiotic substances and/or commensal bacteria. In the context of the present invention nutrients may be dietary precursors and/or a source for dietary precursors also termed "dietary source" undergoing a chemical and/or biochemical reaction thus preferably producing bacterial metabolites.

In the context of the invention, nutritional adjustments for and/or nutritional therapy of a subject comprise adjustments in diet and/or administration of commensal bacteria to the subject. Such nutritional adjustments can be based on standard nutrition guidelines for e.g., healthy adults, children and patients with chronic diseases known in the art and/or be individualized for a subject. Individualized nutritional adjustments are typically based on the determination of abundance and expression of bacterial genes encoding for enzymes associated with the production of one or more bacterial metabolites in a subject. A deviation of gene expression and/or abundance of one or more bacterial genes in a subject from a control value indicates individualized nutritional adjustment. A control value for the expression and/or abundance of a bacterial gene can be a value measured in a healthy subject, an average value measured across a population (population average) such as a healthy population, a population of a specific age, a population of a specific sex, with one or more specific diseases, undergoing specific medical treatment, undergoing a specific diet and/or having a specific lifestyle and/or a subject-specific value e.g., measured before a medical treatment, measured during the course of a disease and/or measured before, during or after an nutritional adjustment. A skilled person is capable of identifying and/or determining such a control value without undue effort.

Adjustments in diet include, in embodiments, an addition of, increase in, decrease in and/or omission of nutrients as dietary precursors of bacterial metabolites and/or source of such dietary precursors, an addition of and/or increase in nutrients promoting the growth of commensal bacteria producing one or more specific bacterial metabolites, decrease in and/or omission of nutrients promoting the growth of commensal bacteria producing one or more specific bacterial metabolites. Administration of commensal bacteria includes administration of one or more commensal bacteria species producing one or more specific bacterial metabolites and/or of bacteria not producing one or more specific bacterial metabolites. Commensal bacteria can be administered by oral, rectal, and/or cutaneous application. Commensal bacteria suitable for administration during nutritional adjustment or therapy include, without limitation, bacteria belonging to the groups of *Bacteroidetes* including the genera Bacteroides, Prevotella and Xylanibacter, *Firmicutes* including the genera Clostridium, Eubacterium, Faecalibacterium, Lactobacillus, Roseburia and Ruminococcus, *Actinobacteria* including the genera Bifidobacterium, *Proteobacteria* including the genera Desulfovibrio and Escherichia and *Verrucomicrobia* including the genus Akkermansia. The particular commensal bacteria described herein are by way of example only. The method is not limited to the administration of the listed commensal bacteria in nutritional adjustment, nor to the detection of the specific bacteria or bacterial genes mentioned in the embodiments of the invention described herein.

In the context of the present invention individual nutritional adjustments aim to decrease and/or increase in the production of one or more bacterial metabolites in a subject. For example, an increase in the dietary sources of metabolite precursors can be advised if a deficiency of metabolite production is revealed and likewise a reduced intake of a dietary source can be advised if overproduction of a metabolite is revealed. For example, overexpression of *cutC* or *cntA* resulting in high TMA and TMAO production in a subject indicates dietary sources of choline (found in eggs, meat and soya) or carnitine (found in meat) should be reduced, respectively. Both approaches reduce TMA production and thus TMAO production. In a similar way, overexpression of *tnaA* can be reduced by restricting dietary sources of tryptophan to reduce IS production. In subjects with low *spcA* or *Bed* expression dietary sources of indigestible carbohydrates (*e.g.*, resistant starch) would be increased to increase propionate or butyrate production, respectively.

Herein the terms "subject", "patient" or "host" may be used interchangeably. A subject, patient or host may be an organism, a cell culture of patient cells or cell lines, an animal, or a cell culture of animal cells or cell lines. Herein a subject or patient refers preferably to a species from which a sample is taken, and/or whose biological material makes up the majority of the biological material of a sample. A subject or patient can be selected from the group comprising vertebrae, animals, livestock, mammals, humans, preferably mammal or human.

In embodiments of the present invention, deviation of gene expression and/or abundance of bacterial genes associated with the production of bacterial metabolites may indicate the presence and/or risk of a medical condition such as an inflammatory disease. The presence and/or risk of a medical condition includes the development, onset, progression and occurrence of inflammatory and/or chronic diseases including, without limitation, cardiovascular diseases such as atherosclerosis, arterial hypertension, peripheral arterial occlusive disease (pAVK) and coronary heart disease (CHD), chronic kidney disease (CDK), irritable bowel syndrome, inflammatory bowel diseases (IBD) such as Crohn's disease or ulcerative colitis, obesity, insulin resistance, diabetes type 2, autoimmune diseases including rheumatic diseases, multiple sclerosis and diabetes type 1, allergic asthma, non-alcoholic fatty liver disease (NAFLD/NASH), cancer, mental health disorders such as Alzheimer's disease, dementia, Parkinson, autism spectrum disorders, generalized anxiety disorder, depression and posttraumatic stress disorder (PTSD) and skin diseases such as eczema and psoriasis.

These indications represent preferred embodiments of the use of the invention as a dietary treatment or intervention in preventing and/or treating a medical condition as described herein.

The terms "nucleic acid", "nucleotide sequence", "polynucleotide", "nucleic acid molecule", "oligonucleotide" may be used interchangeably and refer to a polymeric form of nucleotides (nt) of any length, wherein the nucleotides can be either deoxyribonucleotides (DNA) or ribonucleotides (RNA), or analogs thereof. The terms include, without limitation, DNA, single stranded DNA (ssDNA), double stranded DNA (dsDNA), genomic DNA (gDNA), RNA, messenger RNA (mRNA), transfer RNA (tRNA), ribosomal RNA (rRNA), microRNA (miRNA), small interfering RNA (siRNA), single guide RNA (sgRNA), piwi-interacting RNA (piRNA), small nuclear RNA (snRNA), cDNA, recombinant polynucleotides, branched polynucleotides, plasmids, vectors, viral RNA, nucleic acid probes and primers.

Herein the term "oligonucleotide" may refer to or comprise a primer or a probe of a certain length of consecutive nucleotides. In the context of the present invention the terms "oligonucleotide" and "primer", or "oligonucleotide" and "probe" may be used interchangeably. Oligonucleotides are short DNA or RNA molecules (oligomers). Oligonucleotides are generally characterized by the sequence of nucleotide residues that make up the molecule. Oligonucleotides usually bind in a sequence-specific manner to a complementary oligonucleotide, DNA or RNA sequences thereby forming duplexes.

In embodiments, the present invention relates to polymerase chain reaction (PCR)-based methods, when referring to quantifying an expression level and an abundance of a bacterial gene. A PCR is an enzyme-based nucleic acid amplification reaction in which one or more copies of a specific region ("target region") of a DNA molecule also termed "template" are generated. The product of this amplification reaction is termed "amplicon" which may be interchangeably used with common laboratory terms, *e.g.*, "PCR product". The PCR relies on thermal cycling, comprising cycles of repeated heating and cooling of the reaction for the different stages of the reaction to occur, which include DNA melting also termed "denaturation" (separation of the double strand), binding of sequence-specific primers to the template DNA (annealing), and enzymatic amplification of the DNA molecule by extension of the primers. In case of the analysis of RNA, said RNA is first transcribed into DNA (cDNA) using a reverse-transcriptase enzyme (RT-polymerase). The transcription of RNA into cDNA can be performed before or in the context of a PCR reaction (reverse transcription PCR, RT-PCR).

PCR relies on the use of primers, which are short DNA oligonucleotides that contain sequences complementary to the target region and the application of a DNA polymerase that facilitates selective and repeated enzymatic amplification also termed "replication" of the target region by extension of the primers also termed "elongation". In the progress of PCR, the DNA amplicons generated are used as a template for replication, enabling a chain reaction in which the DNA template is exponentially amplified. Upon completion of the reaction post-PCR processing also termed "end-point analysis" including separation and detection of the amplification product is performed, by using methods including agarose gel electrophoresis and capillary electrophoresis. By an end point analysis, the amount of amplified product at the end of the PCR is measured, whereby the results are considered to be qualitative and indicate the presence or absence of a specific target sequence in a sample without the determination of the product concentration.

The term "DNA polymerase" describes an enzyme catalyzing the transfer of deoxyribonucleotides to a free hydroxyl group at the 3'-terminus of DNA frequently used in PCR. Such enzymes include *e.g.*, Taq polymerase and Pfu DNA polymerase.

The present invention further relates to quantitative PCR methods including "real-time PCR", such as quantitative real-time PCR (qPCR) and quantitative real time reverse transcription PCR (qRT-PCR) and digital PCR. Real-time PCR allows monitoring of the amplification process and detection and quantification of the amplified target region in real time as the reaction proceeds. Data is therefore collected during the exponential phase of the PCR reaction. In digital PCR target molecules are diluted and partitioned into numerous PCR reactions in smaller micro-volume compartments, so that either no, only one or several target molecules are present in each individual reaction. Each partition is analyzed for the presence (positive reaction) or absence (negative reaction) of a fluorescence signal following the endpoint of PCR, from which the absolute number of molecules present in the sample is calculated. In this way a single molecule of the gene target can be detected for gene expression to enable an absolute measurement of gene expression. In conventional PCR methods data is also collected at the end point of the reaction and analyzed by post-PCR processing using methods including agarose gel electrophoresis and capillary electrophoresis. For qPCR no post-PCR processing is necessary as the quantification occurs in real-time during the reaction.

In real-time PCR usually a fluorescent reporter dye also termed "fluorophore" or "reporter dye" is used as an indirect measure of the amount of nucleic acid present during each amplification cycle. A fluorescent reporter dye is designed such that changes in its fluorescent intensity are in direct relationship to the reaction progress, *i.e.*, how much DNA is synthesized. The increase in the fluorescent signal is directly proportional to the amount of exponentially increasing amplicons. Thereby, the cycle threshold (Ct) is the number of cycles in real-time PCR that are necessary to exceed a previously defined threshold value in the measurement signal, *e.g.*, fluorescence signal. The threshold value is usually defined by using a non-template control, which is a sample comprising no template. The Ct-value is related to the initial concentration of the template, whereby a higher initial concentration results in fewer amplification cycles necessary to reach the Ct-value. Reporter dyes can be in the form of double-stranded DNA (dsDNA) binding dyes, dyes conjugated to primers, or dye-conjugated oligonucleotides termed "probes".

A "fluorophore" (or fluorochrome, similarly to a chromophore) is a fluorescent compound that can re-emit light upon light excitation. A double-stranded DNA (dsDNA) binding dye also termed "intercalating dye" emits light with low signal intensity in solution or in the presence of single stranded oligonucleotides. As the PCR progresses and the amount of double-stranded DNA (dsDNA) is increasing, the dye binds to the amplicons by intercalating into the dsDNA, resulting in an increase of light emission (fluorescence) with increasing number of amplicons generated. Intercalating dyes commonly used include but are not limited to propidium iodide (PI), ethidium bromide (EtBr), crystal violet, DAPI, 7-AAD, Hoechst 33258, Hoechst 33342, Hoechst 34580, PicoGreen^{®}, Helixyte^{™}, YOYO-1, DiYO-1, TOTO-1, Dito-1, BOBO-1 and SYBR Green^{®}.

Reverse transcription polymerase chain reaction (RT-PCR) is a laboratory technique combining reverse transcription of RNA into DNA and amplification of specific DNA targets using polymerase chain reaction (PCR). It is an established technique primarily used to measure the amount of a specific RNA, thus enabling, for example, an analysis of gene expression. This is achieved by monitoring the PCR amplification reaction using fluorescence, a technique called real-time PCR or quantitative PCR (qPCR). Combined RT-PCR and qPCR are routinely used for analysis of gene expression and quantification of viral RNA in research and clinical settings.

Modifications of, or particular variations on, known PCR and quantitative PCR reactions or methods are included in the scope of the invention when used to carry out the method described herein. Such variations include for example a digital PCR modification or a multiplex PCR with more than one pair of primers and/or detection probes, whereby two or more target regions can be simultaneously amplified and detected.

The term "primer" refers to an oligonucleotide whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of nucleotides and an inducing agent such as DNA polymerase and at a suitable temperature and pH value. A primer thus can serve as a starting point for DNA-replicating enzymes such as DNA polymerase. The primer or oligonucleotide is preferably single stranded for maximum efficiency in amplification but may alternatively be double stranded. If double stranded, the primer is first treated to separate its strands before being used to prepare extension products. Preferably, the primer is an oligonucleotide, more preferably an oligodeoxyribonucleotide. The primer or oligonucleotide must be sufficiently long to prime the synthesis of extension products in the presence of the inducing agent. The exact lengths of the primers will depend on many factors, including temperature, source of primer and use of the method. For example, for diagnostics applications, depending on the complexity of the target sequence, the oligonucleotide primer typically contains 15-25 or more nucleotides, although it may contain fewer nucleotides.

Hybridization is the process of establishing a non-covalent, sequence-specific interaction between two or more complementary strands of nucleic acids into a single hybrid, which in the case of two strands is referred to as a duplex or DNA double strand in the case of DNA. The terms "binding" or "annealing" may be used in place of hybridization in the present invention. The hybrids may be dissociated by thermal denaturation, also referred to as melting. Here, the solution of hybrids is heated to break the hydrogen bonds between nucleic bases, after which the two strands separate. In the absence of external negative factors, the processes of hybridization and melting may be repeated in succession indefinitely, which lays the ground for PCR.

In the context of the present invention the detection of a certain nucleic acid sequence (target), such as a specific amplicon and/or specific nucleic acid sequence, is in preferred embodiments achieved by using hybridization probes specific for a nucleic acid sequence, or an amplicon. Such a hybridization probe, or short "probe" might be used in embodiments in a nucleic acid amplification reaction. In the context of the present invention, the term "probe" or "hydrolysis probe" refers to a hybridization probe, which is a molecule for the detection of a nucleic acid sequence, such as an amplicon. It can be used in samples containing nucleic acids to detect the presence of a nucleotide or nucleic acid sequence that is at least partially complementary to the sequence in the probe, for example by quantitative PCR, including real-time PCR or digital PCR. The probe thereby hybridizes to single-stranded nucleic acid whose base sequence allows probe-target base pairing due to complementarity between the probe and the target. For detection the probe might be labeled (or "tagged") with one or more molecular markers of either radioactive and/or preferably fluorescent and/or a quencher molecule. Commonly used radioactive markers are 32P (a radioactive isotope of phosphorus incorporated into the phosphodiester bond in the probe DNA) or Digoxigenin, which is a non-radioactive, antibody-based marker.

Detection of sequences with moderate or high similarity depends on how stringent the hybridization conditions are applied. High stringency, such as high hybridization temperature and low salt in hybridization buffers, permits only hybridization between nucleic acid sequences that are highly similar, whereas low stringency, such as lower temperature and high salt, allows hybridization when the sequences are less similar. During real-time or qPCR, hydrolysis probes comprising a fluorescent marker and a corresponding quencher can be used for detection of a nucleic acid sequence. During amplification of a nucleic acid sequence to which a hydrolysis probe is binding by means of primers that lie outside the target sequence, the amplifying polymerase can degrade or hydrolyze the probe from the 5'-end. This leads to the separation of the fluorescence marker and the corresponding quencher that were initially localized in close proximity to each other attached to the probe. Through separation of the fluorescent marker and the quencher, a detectable change in a signal can be generated. Hydrolysis probes are also called 5'-exonuclease probes or TaqMan probes and are well established molecular biology tools that are well known to the person skilled in the art. Further techniques such as molecular beacon probe technology, Scorpion probe technology, nanoparticle probe technology or Amplifluor probe technology may also be used herein for the detection of an amplicon or nucleic acid sequence of interest, such as an MRSA-specific sequence.

As used herein, the term "hybridization" is used in reference to the pairing of complementary nucleic acids. Hybridization and the strength of hybridization (e.g., the strength of the association between the nucleic acids) is impacted by such factors as the degree of complementary between the nucleic acids, stringency of the conditions involved, the Tm (melting temperature) of the formed hybrid, and the G:C ratio within the nucleic acids. As used herein the term "stringency" is used in reference to the conditions of temperature, ionic strength, and the presence of other compounds such as organic solvents, under which nucleic acid hybridizations are conducted. In an illustrative example, hybridization under "highly stringent condition" can mean hybridization at 65 °C in 5X SSPE and 50% formamide and washing at 65 °C in 0.5X SSPE. In another illustrative example,"highly stringent condition" can mean hybridization at 55°C in a hybridization buffer consisting of 50% formamide (vol/vol); 10% dextran sulfate; 1 x Denhard"s solution; 20 mM sodium phosphate, pH 6.5; 5 x SSC; and 200 µg of salmon sperm DNA per ml of hybridization buffer for 18 to 24 hours, and washing four times (5 min each time) with 2 x SSC; 1% SDS at room temperature and then washing for 15 min at 50- 55°C with 0.1 x SSC. In another illustrative example Conditions for high stringency hybridization are described in Sambrook et al., "Molecular Cloning: A Laboratory Manual", 3rd Edition, Cold Spring Harbor Laboratory Press, (2001), incorporated herein by reference. In some illustrative aspects, hybridization occurs along the full-length of the nucleic acid. Detection of highly stringent hybridization in the context of the present invention indicates strong structural similarity or structural homology (e.g., nucleotide structure, base composition, arrangement or order) to, e.g., the nucleic acids provided herein.

In embodiments of the invention, the "target sequences" are those genes described herein associated with the production of a bacterial metabolite. The term "target sequence" refers to the nucleic acid sequence that is to be detected by the probe or a set of primers. The nucleic acid target sequence comprises a nucleic acid sequence, which is at least partially complementary to the nucleic acid sequence comprised in the probe. A probe according to the present invention comprises an oligonucleotide sequence complementary to the region of the nucleic target acid sequence. Said oligonucleotide sequence of the probe comprises, consists or essentially consists of 5 to 100 bases, preferably 5 to 50, 10 to 40, 12 to 38, 13 to 35, 14 to 32, 15 to 28, 16 to 26, 17 to 25, 18 to 24, 19 to 23, 20 to 22, 21 to 22, or most preferably 22 bases. The length of the probe is therefore preferably between 10 and 40 nucleotides in length, more preferably 10, 11 12, 13, 14, 15, 16, 25 17, 18, 19, 20, 21, 22, 23, 24, 25, 26, 27, 28, 29, 30, 31, 32, 34, 3,5 36, 37, 38, 39, or 40 nt.

In embodiments of the invention, primer, probe and/or target sequences may exhibit some variation in sequence and/or length compared to the specific sequences described herein. In embodiments, a nucleic acid molecule, such as an oligonucleotide, primer or probe may have, a 0 to 10 nucleotides addition or deletion at the 5' or 3' terminus of a sequence, with reference to the specific sequences provided herein. As used herein the term "a 0 to 10 nucleotides addition or deletion at the 5' or 3' terminus of a sequence" means that the nucleic acid may have a) 0, 1 , 2, 3, 4, 5, 6, 7, 8, 9 or 10 additional nucleotides at its 5' terminus and 0, 1 , 2, 3, 4, 5, 6, 7, 8, 9 or 10 nucleotides deleted at its 3' terminus or b) 0, 1 , 2, 3, 4, 5, 6, 7, 8, 9 or 10 additional nucleotides at its 3' terminus and 0, 1 , 2, 3, 4, 5, 6, 7, 8, 9 or 10 nucleotides deleted at its 5' terminus, c) 0, 1 , 2, 3, 4, 5, 6, 7, 8, 9 or 10 additional nucleotides at its 5' terminus and 0, 1 , 2, 3, 4, 5, 6, 7, 8, 9 or 10 additional nucleotides at its 3' terminus or d) 0, 1 , 2, 3, 4, 5, 6, 7, 8, 9 or 10 nucleotides deleted at its 5' terminus and 0, 1 , 2, 3, 4, 5, 6, 7, 8, 9 or 10 nucleotides deleted at its 3' terminus. Such additional nucleotides, for example in the context of primers and/or probes, may for example also be designed to be complementary to a target sequence.

In embodiments, the method, kit or oligonucleotides of the present invention are characterized in that the one or more primer or probe or target sequence comprises a nucleotide sequence with 80% or more, 85% or more or preferably 90%, 95% or more sequence identity, to the sequences provided herein, for example, 80, 81, 82, 83, 84, 85, 86, 87, 88, 89, 90, 91, 92, 93, 94, 95, 96, 97, 98, 99% sequence identity. A sequence variant with between 80% and 99% sequence identity is preferably functionally analogous, *i.e.*, sequences that although exhibiting differences in DNA sequence, so that for example the same, fewer or more mismatches may occur between primer/probe and target, exhibit a similar specificity for the target to be detected, so that the function of the primer/probe in the context of the present invention is maintained. Functionally analogous sequences can be tested by one skilled in the art without inventive effort in light of the information provided within the application, for example by testing hybridization properties in the context of the PCR or other amplification methods described.

As used herein, the term "complementary" refers to the ability of purine and pyrimidine nucleotide sequences to associate through hydrogen bonding to form double- stranded nucleic acid molecules by hybridization. The nucleic acid bases guanine (G) and cytosine (C), adenine (A) and thymine (T), and adenine (A) and uracil (U) are complementary and can associate through hydrogen bonding resulting in the formation of double-stranded nucleic acid molecules when two nucleic acid molecules have "complementary" sequences. The complementary sequences can be DNA or RNA sequences. The complementary DNA or RNA sequences are referred to as a "complement". Complementary may be "partial" in which only some of the nucleic acid bases are matched according to the base pairing rules, or there may be "completely" or "total" complementary between the nucleic acids.

The oligonucleotides, probes or primers herein are selected to be "substantially" complementary to the different strands of each specific sequence to be amplified. This means that the oligonucleotides, probes or primers must be sufficiently complementary to hybridize with their respective strands. Therefore, the sequences do not need to have a 100% sequence identity, some mismatches are acceptable and the nucleic acid strands will still hybridize. Thereby, non-complementary bases or longer sequences can be interspersed into a primer, provided that a primer sequence has sufficient complementarity with the sequence of the strand to hybridize therewith and thereby form a point of initiation for synthesis of the extension product within a PCR reaction.

As used herein, "sequence identity", "identity", "sequence homology" or "homology" in the context of two nucleic acid sequences makes reference to a specified percentage of residues in the two sequences that are the same when aligned for maximum correspondence over a specified comparison window, as measured by sequence comparison algorithms or by visual inspection.

As used herein, "percent (%) sequence identity", "sequences with % identity", "percent (%) sequence homology" or "sequences with % homology" to a specific "reference sequence" (*e.g*., to SEQ ID No 1) means the percentage of nucleotides in a certain sequence that are identical with the nucleotides of the reference sequence and is determined by comparing the two optimally aligned sequences over a comparison window wherein the portion of the polynucleotide sequences in the comparison window may include additions or deletions (*i.e.*, gaps) as compared to the reference sequence (which does not include additions or deletions) for optimal alignment of the two or more sequences. The percentage is calculated by determining the number of positions at which the identical nucleic acid base or amino acid residue occurs in the two or more sequences to yield the number of matched positions, dividing the number of matched positions by the total number of positions in the window of comparison, and multiplying the result by 100 to yield the percentage of sequence identity.

Any suitable methods of alignment of sequences for determination the percent sequence identity may be employed and are known to a person skilled in the art. The determination of percent identity between any two or more sequences can be accomplished using a publicly available mathematical algorithm. Computer software implementations of these mathematical algorithms include but are not limited to: ClustalW algorithm (VNTI software, InforMax Inc.), ALIGN (Version 2.0), GAP (Genetics Computer Group, software; now available via Accelrys on http://www.accelrys.com), BESTFIT, BLAST^{®}, FASTA, and TFASTA in the Wisconsin Genetics Software Package, Version 8 (available from Genetics Computer Group (GCG), Madison, Wis., USA) and Multiple sequence alignments MUSCLE (EMBL's European Bioinformatics Institute (EMBL-EBI), Cambridgeshire, UK). Alignments using these programs can be performed using the default parameters. Software for performing BLAST^{®} analyses is publicly available through the National Center for Biotechnology Information. A sequence database can be searched using the nucleic acid sequence of interest. Algorithms for database searching are typically based on the BLAST software (Altschul *et al.,* 1990). In some embodiments, the percent homology or identity can be determined along the full-length of the nucleic acid.

The term "degenerate primer" refers to a mixture of primers with similar sequence, whereby nucleic acid bases differ at defined positions termed "degenerate regions" while the rest of the sequence is identical. The sequence of degenerate primers is summarized in one sequence code termed "degenerate code". The degeneracy of a degenerate primer is the number of unique sequence combinations it contains. Using the International Union of Pure and Applied Chemistry (IUPAC) system the degenerate regions within the degenerate code are encoded by a single letter code, whereby N encodes for adenine (A), guanine (G), cytosine (C) or thymine (T); S encodes for G or C; R encodes to A or G; Y encodes for C or T; W encodes for A or T; K encodes for G or T; M encodes for A or C; B encodes for C, G or T; D encodes for A, G or T; H encodes for A, C or T and V encodes for A, C or G.

Degenerate primers are used to amplify multiple homologous genes (other termed genes with a degree of sequence identity) with distinct coding sequence (e*.g.,* in different bacterial species) or paralogous genes (within a species) with a degenerate primer pair. The use of degenerate primers in the context of the present invention allows detection and quantification of the expression and abundancy of specific bacterial genes encoding for enzymes associated with the production of bacterial metabolites across the microbiome independently of the source bacterial species. Methods for designing degenerate primers are known to a person skilled in the art and usually involve steps of alignment of multiple protein sequences or nucleic acid sequences, identification of regions with high sequence similarity, identification of primer sequences hybridizing with these regions, testing of degenerate primer pairs with regards to primer-primer interactions, pairing with other regions leading to unspecific amplification products (mispriming, specificity of the primer) and pairing with the target region (primer efficiency). Degenerate primer design can generate a large number of potential primers, which is often too large to test experimentally. *In silico* PCR can be used to simultaneously pre-screen a large number of potential degenerate primer sets before narrowing down to a set of primers that is experimentally tested and validated. Software used for degenerate primer design is known to a person skilled in the art and includes common sequence alignment software and primer design software including iCODEHOP (Consensus-Degenerate Hybrid Oligonucleotide Primers), NCBI Primer-BLAST or HYDEN (Highly Degenerate primers).

In the context of the present invention the detection and quantification of a certain nucleic acid sequence (target), such as a bacterial gene encoding for an enzyme associated with the production of bacterial metabolites, is in preferred embodiments achieved by using hybridization probes specific for a nucleic acid sequence. Such a hybridization probe, or short "probe" might be used in embodiments in a nucleic acid amplification reaction. In the context of the present invention, the term "probe" or "hydrolysis probe" refers to a hybridization probe, which is a molecule for the detection of a nucleic acid sequence, such as an amplicon. It can be used in samples containing nucleic acids to detect the presence of a nucleotide or nucleic acid sequence that is at least partially complementary to the sequence in the probe, for example by real-time PCR. The probe thereby hybridizes to a single-stranded nucleic acid whose base sequence allows probe-target base pairing due to complementarity between the probe and the target. A probe according to the present invention comprises an oligonucleotide sequence complementary to the region of the nucleic target acid sequence. A probe is thereby designed to bind to the target sequence in between the primers.

For detection the probe might be labeled (or "tagged") by covalent attachment of one or more molecular markers of either radioactive and/or preferably fluorescent and/or a quencher molecule.

Commonly used radioactive markers are 32P (a radioactive isotope of phosphorus incorporated into the phosphodiester bond in the probe DNA) or Digoxigenin, which is a non-radioactive, antibody-based marker. Fluorophores for use as covalently-attached markers for oligonucleotides of the present invention can be selected from, without being limited to, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3- 25 coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 30 488, Rhodamine Green, Oregon Green 514, Cal Gold, BODIPY R6Gj, Yakima Yellow, JOE, HEX, Cal Orange, BODIPY TMR-X, Quasar-570 /Cy3, TAMRA, Rhodamine Red-X, Redmond Red, BODIPY 581/591, Cy3.5, Cal Red/Texas Red, BODIPY TR-X, BODIPY 630/665-X, Pulsar-650, Quasar-670/Cy5.

During real-time PCR, hydrolysis probes comprising a fluorescent marker and a corresponding quencher can be used for detection of a nucleic acid sequence. The probe is usually modified such that it comprises a fluorescent marker at the 5' end and a quencher at the 3' end, whereby the fluorescent marker is quenched due to the close proximity to the quencher. During amplification of a nucleic acid sequence to which a hydrolysis probe is binding, the amplifying polymerase can degrade or hydrolyze the probe from the 5'-end. This leads to the separation of the fluorescence marker and the corresponding quencher and results in a detectable change in fluorescence signal. As the target is amplified, an increasing amount of probe is hydrolyzed, resulting in an exponential increase or the fluorescence signal, being proportional to the amplicon concentration. As the probes are sequence-specific, the fluorescence will only increase as a result of amplification of the target DNA sequence. Hydrolysis probes are also called 5'-exonuclease probes or TaqMan probes and are well established molecular biology tools that are well known to the person skilled in the art. Further techniques such as molecular beacon probe technology, HyBeacons^{®} technology, Scorpion probe technology, nanoparticle probe technology or Amplifluor probe technology may also be used herein for the detection of an amplicon or nucleic acid sequence of interest, such as a bacterial gene encoding for an enzyme associated with the production of a bacterial metabolite.

In the context of the present invention in preferred embodiments the specific detection of amplification during a real-time PCR reaction can be achieved by using fluorescent reporter probes, which are sequence-specific and detect only the DNA comprising the complementary sequence to the probe. Therefore, use of the reporter probe significantly increases specificity, and enables performing the technique even in the presence of other DNA molecules with distinct sequences. By using fluorescence markers with distinct excitation and/or emission wavelengths, hybridization probes can be used in multiplex assays for simultaneously amplifying and monitoring several target sequences in the same reaction. Within multiplex assays the excitation and/or emission wavelengths of the fluorescent probe should be as distinct as possible from each other to minimize optical cross talk.

In the context of the present invention, an oligonucleotide, primer or probe might be labelled with a fluorophore and optionally a quencher that interacts with said fluorophore. Herein "quenching" refers to any process which decreases the fluorescence intensity of a given substance. Quenching is the basis for Förster resonance energy transfer (FRET) assays. FRET is a dynamic quenching mechanism wherein energy transfer occurs while the donor is in an excited state. A "quencher" is a molecule, which quenches the fluorescence emitted by the fluorophore when excited by a light source via FRET. Quenchers that might be used in the context of the present invention can be selected from the group comprising, but without being limited to, DDQ-I, 22 Dabcyl, Eclipse, Iowa Black FQ, BHQ-1, QSY-7, BHQ-2, DDQ-II, Iowa Black RQ, QSY-21, BHQ-3, QSY-35, BHQ-0, QSY-9, ElleQuencher, Iowa Black, MGB. The one skilled in the art can routinely choose suitable reporter-quencher combinations.

The amount of the amplification product in a quantitative PCR reaction can be determined by absolute and relative quantification methods. Absolute quantification methods include digital PCR and quantification by a copy number standard curve in real time PCR. Digital PCR comprises partitioning of the sample into multiple individual reactions, resulting in random distribution of the target molecules into the reactions, whereby some of which contain the target molecule (positive) and some of which do not (negative). The ratio of positive to negative reactions is subsequently used to determine the number of target molecules. Since the target molecule is randomly distributed across the available partitions, the average number of molecules per partition (none, one or more) is estimated by statistical analysis and the copies of the target molecule per positive partition is calculated. The statistical analysis of the number of positive and negative reactions provides a precise absolute quantification of the target sequence. Absolute quantification by using a copy number standard curve (standard curve quantification method) relies on the determination of the copy number of target molecules also by reference to a standard curve generated by serial- dilution of a standard with defined, absolute concentration, *i.e.*, copy number. The term "copy number" of a standard or a target refers to the number of amplicons generated during a PCR reaction and thus to the amount or the concentration of amplicons. A standard with defined concentration can be generated by various methods including the use of plasmid DNA and *in vitro* transcribed RNA, subsequent determination of the concentration by measuring the absorption at 260 nm (A₂₆₀) and conversion to the copy number using the molecular weight of the DNA or RNA molecule. A standard for absolute quantification is usually a target-specific nucleic acid, *i.e.*, gene-specific for a gene encoding for an enzyme associated with the production of a bacterial metabolite, which comprises the target sequence or a fragment thereof. Standard curves are generated by serial dilution of the standard and correlation of the Ct-values determined in real-time PCR, which are related to the initial template concentration used in the reaction, to the concentration of the standard. The standard and dilutions thereof can be amplified simultaneously with the target within a multiplex PCR or in separate tubes.

Relative quantification also termed "comparative quantification" determines relative changes in expression levels of a gene between samples that were treated differently or underwent different experimental conditions, whereby one of the samples is the control sample. The quantification value is expressed as ratio relative to the control sample. Relative quantification methods include the use of a standard curve, wherein only the relative dilution and not the absolute concentration of the standard need to be known, and the comparative Ct-method, which are known to a person skilled in the art.

In the context of the present invention the terms "standard" and "control" refer to nucleic acids molecules that are used for quantification and/or normalization of the amplicon copy number within a real-time PCR. Standards can be internal (endogenous) or external (exogenous), whereby an internal standard is inherently present in each sample and is amplified simultaneously with the target gene. The use of an internal control allows normalization of the amplicon copy number to the total amount of RNA or DNA per sample, thereby taking variations in sample preparation and/or loading into account. The choice of internal control is dependent on the sample and target, whereby commonly used internal controls include but are not limited to β-actin, Glycerinaldehyd-3-phosphat-Dehydrogenase (GAPDH), ribosomal RNA (rRNA) such as 18s or 16s rRNA. An exogenous control is a pre-characterized nucleic acid that is added to each sample at a defined concentration (spiked) and/or measured separately, e.g., in serial dilution within a standard curve. The exogenous control can be target-specific, i.e., comprising the target sequence or a fragment thereof in the form of an oligonucleotide or another pre-characterized nucleic acid that is not target-specific. Exogenous controls can be used for normalizing for differences in the efficiency of sample extraction or synthesis of complementary DNA (cDNA) by reverse transcriptase. Target-specific exogenous control can be further used to distinguish true target-negative samples from PCR inhibition and for absolute quantification of the target as described above, whereas

In the context of the present invention a sample may be any suitable tissue, excremental or fluid/liquid sample comprising commensal and/or pathogenic bacteria of a subject. The sample may be obtained from a human or animal subject. In embodiments of the present invention the sample is a stool sample, a biopsy sample, *e.g.*, of the gastrointestinal tract and/or skin), interstitial fluid sample of an oral, respiratory and/or intestinal compartment, a mucosal swab and/or skin swab.

A biopsy sample is a sample comprising body material obtained through a biopsy. A biopsy is a procedure to remove and obtain a small amount of tissue from a subject. Different types of biopsy include without limitation punch biopsy, needle biopsy, endoscopic biopsy, excision biopsy and perioperative biopsy and are known to a person skilled in the art. Biopsy samples include without limitation samples of the intestinal mucosa, the skin, the liver, the kidney, the lung, the brain and a tumor.

The sample may be prepared to lyse tissue and/or cells such as bacteria and release the nucleic acids within cells into a solution using any suitable method, as described below. In some embodiments, the sample contains a suitable buffer for lysing cells, for stabilizing nucleic acids in the sample and/or for carrying out PCR such as real-time PCR.

In certain embodiments, the present method includes preparing a sample, *e.g.*, a stool sample, intestinal mucosal biopsy sample, etc. for determination of the production capacity of a metabolite by the method described herein. Preparing the sample may include treating the sample with mechanical, thermal, chemical and/or enzymatic methods of lysing tissue, cells, and/or cellular compartments (*e.g.*, plasma membrane, cell wall, nucleus, mitochondria, etc.) in the sample to release nucleic acids, *e.g.*, DNA and/or RNA, into the bulk of the sample and remove impurities.

Any suitable method of mechanically lysing tissue and/or cells may be used including but not limited to homogenizing, grinding, ultrasonicating or freezing the sample. Any suitable method of chemically lysing tissue and/or cells may be used including but not limited to alkaline lysis, detergent lysis (*e.g.*, sodium dodecyl sulfate (SDS)), solvent lysis (*e.g.*, chloroform), use of a chaotropic agent, *e.g.*, a chaotropic salt such as guanidinium isothiocyanate, guanidinium chloride, urea, thiourea, lithium perchlorate, lithium acetate, sodium iodide, phenol and others. Any suitable method of enzymatically lysing tissue and/or cells may be used. In some embodiments including but not limited to treatment of the sample with protease, lipase, glycoside hydrolases, proteinase K, keratinase, trypsin, subtilisin, lyticase, lysozyme, collagenase, cellulase, glucanase, chitinase, pectinase, or amylase. Any suitable method of thermally lysing tissue and/or cells may be used, e.g., by subjecting the sample to a temperature of 50° C or more, e.g., 60° C, 70° C, 80° C, 90° C, 95° C or 100° C.

Preparing the sample may further include removal of non-nucleic acid material after lysis of tissue and/or cells. Removal of non-nucleic acid material may include an extraction step, precipitation and/or precipitation by absorption to ta DNA- or RNA-binding matrix. These methods include but are not limited to the combined use of phenol and chloroform (phenol/chloroform extraction), Triton X-100, trizol-extraction and absorption to solid phases such as columns and magnetic beads followed by centrifugation. These methods are known to a person skilled in the art and have been employed within various kits from a variety of suppliers.

In certain embodiments of the present invention sample preparation may further include the removal of DNA from a sample comprising RNA by enzymatic treatment of the sample by DNAses such as DNAse I.

The present invention also includes a kit, package and multi-container unit, comprising degenerated primers, probes and standards for the determination of the expression level and/or abundance of a bacterial gene associated with the production of a bacterial metabolite. In a preferred embodiment within the kit, package and/or multi-container unit the primers, probes and standards are provided in a multi-well plate, preferably a 96-, 192- or 384-well plate. In another preferred embodiments of the present invention within the kit, package and/or multi-container unit the primers, probes and standards are provided in a dried form. In another preferred embodiment the kit, package and/or multi-component unit comprises primers, probes, and standards for the determination of the expression level and/or abundancy one or more bacterial genes associated with the production of a bacterial metabolite. Sensitivity of the assay is not affected by the drying process. Providing the primers, probes and standards within a multi-well plate further makes the assay medium- to high-throughput and ideal for use in everyday clinical practice.

In another preferred embodiment the kit, package or multi-container unit further comprises standard excipients for carrying out a real-time PCR, including without limitation suitable buffers, PCR nucleotides (dNTPs), a polymerase and optionally a reverse-transcriptase (RT). The kit, package and/or multi-component unit may further comprise necessary materials and chemicals for sample acquisition and/or preparation, including without limitation a container for sample acquisition, a swab for acquisition of a mucosal or skin swab, buffers, chemicals for cell lysis and/or a nucleic acid-binding matrix for extraction of nucleic acids from the sample. In a preferred embodiment of the invention the prepared sample of a subject can be directly added to the multi-well plate comprising the primers, probes and standards and the real-time PCR reaction can be carried out upon addition of standard PCR excipients.

### Table 1: Sequences of the invention:

Notes: Binding sites of the primers to the respective nucleic acid sequence are underlined within the nucleic acid sequence.
[1] Rath, S., et al., Uncovering the trimethylamine-producing bacteria of the human gut microbiota. Microbiome, 2017. 5(1): p. 54.
[2] Solbach, P., et al., BaiCD gene cluster abundance is negatively correlated with Clostridium difficile infection. PLoS One, 2018. 13(5): p. e0196977.
[3] David, L.A., et al., Diet rapidly and reproducibly alters the human gut microbiome. Nature, 2014. 505(7484): p. 559-63.
   * *in silico* designed
   ** identified target sequence for primer design
   *** by product of bacterial bile acid transformation

Any of the sequences provided in the table below, any combination of sequences, or any marked sub-regions of the sequence, for example those underlined, may represent an embodiment of the invention and/or be employed in any of the aspects or embodiments of the invention described herein.

| **SEQ ID NO** | **Sequence 5'-3'** | **Name** | **Gene** | **Metabolite / function** |
|---|---|---|---|---|
| 1 | TTATTCGGTGGATACGGATATATCCGTGAGTAT GATGTAGAGCGTATGATGCGTGATGCTAAGATT ACAGAGATCTACGAGGGAACTTCAGAGGTTCAG CGTATGGTTATTTC | Eubacterium rectale (cluster 2) | Butyryl-CoA-dehydrogenas e (*Bcd*) | Butyrate |
| 2 | ACAGGCTAAGCTGATCGCAGCCCGCACTGCAAG CGATGTGACCCGCCGCTGCCTGCAGCTGTTCGG TGGTTACGGCTACACCCGTGACTACCCCATCGA GCGCATGATGCGTGATGCCAAGATCACTGAGAT CTACGAGGGCACCAGCGAAGTGCAGATGATGGT CATTTCCGGCGCGCTGCTGAAGTAA | Faecalibacter ium prausnitzii (cluster 3) | Butyryl-CoA-dehydrogenas e (*Bcd*) | Butyrate |
| 3 | TTATATGGTGGATATGGATATACAAAAGATTAT CCAATCGAGAGAATGATGCGTGACGCTAAGATT ACAGAAATCTATGAAGGAACTTCTGAAGTACAG ATGATGGTTATCTCT | Anaerobutyri cum hallii (cluster 3) | Butyryl-CoA-dehydrogenas e (*Bcd*) | Butyrate |
| 4 | TTATTCGGTGGATACGGATATATCCGTGAGTAC GACGTAGAGCGTATGATGCGCGATGCCAAGATC ACAGAGATCTACGAAGGAACTTCCGAAGTTCAG CGTATGGTTATTTCT | Roseburia faecis (cluster 2) | Butyryl-CoA-dehydrogenas e (*Bcd*) | Butyrate |
| 5 | TTATTCGGTGGATATGGATACATCAGAGAGTAC GATGTAGAGCGTATGATGCGTGATGCTAAGATC ACAGAGATCTATGAGGGAACAAGCGAAGTACAG CGCATGGTTATCTCA | Roseburia inulinivorans (cluster 2) | Butyryl-CoA-dehydrogenas e (*Bcd*) | Butyrate |
| 6 | TTACACGGTGGTTACGGTTACATCAAGGAGTAC GATGTTGAGCGTATGATGCGTGATGCTAAGATT ACAGAGATCTACGAGGGAACTTCAGAGGTTCAG CGTATGGTTATTTCT | Coprococcus eutactus (cluster 2) | Butyryl-CoA-dehydrogenas e (*Bcd*) | Butyrate |
| 7 | CTATTCGGCGGCTATGGCTACACCCGTGAATAT CCCGTGGAGCGGATGATGCGAGACGCCAAGATC ACTGAGATCTACGAGGGTACCAGCGAGGTCCAG CGCATGGTGATCTC | Oscillibacter sp. (cluster 1) | Butyryl-CoA-dehydrogenas e (*Bcd*) | Butyrate |
| 8 | GGYGGHTAYGGHTAYAY | BCD_Primer _degenerate _forward_1 | Butyryl-CoA-dehydrogenas e (*Bcd*) | Butyrate |
| 9 | TGGATCTCAAGAGAATAT | BCD_Primer _degenerate _forward_2 | Butyryl-CoA-dehydrogenas e (*Bcd*) | Butyrate |
| 10 | CGGCTACACCCGTGACTA | BCD_Primer _degenerate _forward_3 | Butyryl-CoA-dehydrogenas e (*Bcd*) | Butyrate |
| 11 | ACCATVMKSTGNACYTC | BCD_Primer _degenerate _reverse_1 | Butyryl-CoA-dehydrogenas e (*Bcd*) | Butyrate |
| 12 | ATAGCTTCGGATGTACC | BCD_Primer _degenerate _reverse_2 | Butyryl-CoA-dehydrogenas e (*Bcd*) | Butyrate |
| 13 | ACCGGAAATGACCATCATCTG | BCD_Primer _degenerate _reverse_3 | Butyryl-CoA-dehydrogenas e (*Bcd*) | Butyrate |
| 14 | TGATGCGHGAYGCYAAGATYACWG | BCD_Probe_ 1 | Butyryl-CoA-dehydrogenas *e* (*Bed*) | Butyrate |
| 15 | TGTATCGTGATGCAAAGATCAC | BCD_Probe_ 2 | Butyryl-CoA-dehydrogenas *e* (*Bed*) | Butyrate |
| 16 | ATGATGCGYGATGCYAAGAT | BCD_Probe_ 3 | Butyryl-CoA-dehydrogenas e (*Bcd*) | Butyrate |
| 17 | ACGAACTGGCTCACAAGGCTTGGGAACATATTC AGGAAATTGAAAGCTTGGGCGGTATGGCAAAAG CTATCGAAACCGGAGTACCCAAAATGCGTATTG AAGAAGCTGCGGCACGTACTCAGGCCCGTATCG ACTCCGGTGCCCAGACAATTGTCGGCACTAACA AATATCGTTTGGAAAAAGAGGATCCTATCGATA TCCTTGAAGTAGACAATACAGCCGTACGTTTGG AACAAATTGAAAATCTGAAGCGTTTGAAAGAAG GCCGCAATCAAGCAGAAGTAGACAAAGCTTTGG CAGCCATCACCGAATGTGTAAAGACAGGTAAAG GCAATTTGCTGGAACTGGCTGTAGAGGCAGCTC | Bacteroides vulgatus (cluster 1) | Methylmalonyl -CoA mutase (*spcA*) | Propionate |
| | ATGTCCGTGCTACCTTGGGCGAGATTTCGGATG CTTGTGAAACAATTGTAGGACGTTATAAAGCAG TAATCAGAACTATATCAGGCGTGTACTCATCAG AA | | | |
| 18 | ACGAACTGGCTCATAAGGCTTGGGAACACATTC AGGAAATCGAAAAACTGGGCGGTATGGCGAAAG CTATCGAAACCGGAATTCCGAAAATGCGTATCG AAGAAGCTGCCGCACGTACACAGGCTCGTATCG ACAGTGGTCAGCAGACTATTGTGGGTGTGAACA AGTATCGTCTGGATAAAGAAGCTCCGATTGATA TTCTTGAAATTGATAATACTGCCGTTCGTCTTG AACAGATTGAAAACCTGAAACGTTTGAAAGAAG GACGCAACCAGGCTGAAGTGGACAAGGCATTGG CTGCAATTACCGAATGTGTGAAAACCGGTAAGG GCAACTTGCTGGAACTGGCTGTGGAAGCGGCTC GTGTTCGTGCGACATTGGGTGAAATCTCATCTG CTTGCGAACAGATTGTAGGACGTTATAAAGCAA TAATTAGAACTATATCAGGCGTGTATTCATCAG AA | Bacteroides acidifaciens (cluster 1) | Methylmalonyl -CoA mutase (*spcA*) | Propionate |
| 19 | ACGAACTGGCTCACAAGGCTTGGGAACGTATCG AGGAAGTAGAAAAACTGGGTGGTATGGCGAAAG CTATCGAGACCGGTATTCCTAAGATGCGTATCG AAGAGGCTGCTGCTCGTACGCAGGCCCGTATCG ACAGCGGTTCTCAGACTATTGTTGGTGTGAACA AATATCGTCTGGAAAAAGAAGCTCCGATTGATA TCCTTGAGATTGATAACACGGCAGTTCGTCTTG AACAGATCGAGAACCTGAAACGTTTGAAAGAAG GACGTAACCAGGCAGAAGTGGACAAAGCACTGG CAGCCATCACCGAATGCGTGGAGACCGGTAAGG GTAACTTGCTGGAACTGGCTGTAGAAGCTGCTC GTGTTCGTGCAACGCTGGGTGAAATCTCCTATG CTTGCGAAAAGGTTGTAGGACGTTATAAAGCAA TAATTAGAACAATATCAGGCGTGTATTCATCAG AA | Bacteroides thetaiotaomic ron (cluster 1) | Methylmalonyl -CoA mutase (*spcA*) | Propionate |
| 20 | ACGAACTGGCACACAAGGCTTGGGAGCATATTC AGGAAATTGAAAAACTGGGTGGTATGGCGAAAG CTATCGAAACAGGTATTCCAAAGATGCGTATCG AAGAAGCTGCTGCCCGTACACAGGCTCGTATCG ACAGTGGTCAGCAAACAATTGTGGGTGTGAACA AGTATCGTTTGGAAAAAGAAGCTCCGATTGATA TTCTTGAAATTGATAATACTGCAGTCCGTCTTG AACAGATCGAAAACCTGAAACGTTTGAAAGAAG GACGTAATCAGGCTGAAGTGGACAAAGCATTGG CAGCTATCACTGAATGTGTGAAGACCGGCAAGG GTAACTTGCTTGAACTGGCAGTTGAAGCAGCTC GTGTTCGCGCAACATTGGGTGAGATTTCTTATG CTTGCGAACAGATAGTAGGACGTTATAAAGCAA TAATTAGAACGATATCAGGCGTGTATTCATCAG AA | Bacteroides ovatus (cluster 1) | Methylmalonyl -CoA mutase (*spcA*) | Propionate |
| 21 | ATGACTTGGCTCACAAGGCTTGGGAGCTTATTC AGGAAGTAGAAAAACTGGGTGGTATGGCAAAGG CTATCGAAACCGGTATTCCTAAGATGCGCATCG AAGAAGCTGCTGCACGTACACAGGCTCGTATCG ACTCCGGTTCTCAGACTATTGTTGGTGTGAATA AATATCGTCTGGAGAAAGAAGCTCCGATTGATA TCCTCGAAATCGACAATACGGCTGTTCGTCTGG AACAGATTGAGAACCTGAAGCGCTTGAAAGAAG GAAGAAATGAGGCTGAAGTGCAGAAAGCATTGG AAGCTATTACCAAATGCGTGGAAACCAAGGAAG GCAATTTGCTGGAGCTGGCAGTAGAAGCTGCCC | Bacteroides intestinalis (cluster 1) | Methylmalonyl -CoA mutase (*spcA*) | Propionate |
| | GTGTTCGCGCTACGTTGGGAGAAATCTCTTATG CTTGCGAAAAGATTGTAGGACGTTATAAGGCAA TAATCAGAACTATATCAGGCGTGTATTCATCAG AA | | | |
| 22 | ATGAGTTGGCTCACAAGGCTTGGGAGTTGATTC AGGAAGTAGAGAAACTGGGCGGTATGGCAAAAG CCATCGAAACCGGCATTCCCAAAATGCGTATCG AAGAGGCTGCTGCGCGTACACAAGCCCGTATCG ACAGCGGTTCACAGACCATTGTCGGTGTGAACA AGTATCGTCTGGAGAAAGAAGCTCCGATTGATA TTCTTGAAATTGACAATACGGCAGTCCGTCAGG AACAGATTGAGAACCTGAAGCGTCTGAAGGAAG GACGTAACCAGGCGGAAGTGGACAAGGCATTGG AGGCTATTACTGAATGTGTAAAGACCGGTAAGG GAAACTTGCTGGAACTGGCAGTCGAGGCAGCCC GTGTCCGTGCCACTTTGGGGGAAATCTCTTATG CTTGCGAAAAGATTGTAGGACGTTATAAAGCAG TAATTAGAACTATATCAGGCGTGTATTCATCAG AA | Bacteroides uniformis (cluster 1) | Methylmalonyl -CoA mutase (*spcA*) | Propionate |
| 23 | AATGAATTGGCTCACAGTGCTTGGGAGCATATT CAGGAAATCGAGAAGTTGGGAGGTATGGCAAAG GCTATCGAGACCGGTATTCCGAAAATGCGTATC GAAGAAGCGGCTGCCCGTGCACAAGCACGTATC GACAGCGGATCGCAGACCATTGTCGGTGTAAAT AAGTATCGTCTTGAAAAAGAAGATCCGATTGAT ATTCTTGAAGTAGACAATACTGCTGTTCGTAAA GAGCAGATCGAGAACCTGAAACGTTTGAAAGAA GGTCGTAATCAGGCCGAAGTGGACAAGGCACTG GCCGCTATCACTGAATGTGTGAAAACCGGTAAG GGCAATTTGCTCGAGTTGGCGGTAGAAGCTGCC CGTGTTCGTGCTACATTGGGTGAAATCTCGTAT GCTTGCGAACAAATAGTAGGACGTTATAAAGCA ATAATCAGAACTATATCAGGCGTGTATTCATCA GAA | Bacteroides fragilis (cluster 1) | Methylmalonyl -CoA mutase (spcA) | Propionate |
| 24 | TGGCWCAYARKGCTTGGGAR | ScpA_clust 1_Primer_de generate_for ward | Methylmalonyl -CoA mutase (spcA) | Propionate |
| 25 | TGARTACACGCCTGATATHGTTCTRATT | ScpA_clust 1_Primer_de generate_rev erse | Methylmalonyl -CoA mutase (spcA) | Propionate |
| 26 | GGCCGTGGACTCCATCCTGGACATGGAAATCCT CTTCAAGGGCATTCCCCTGGACAAAATGTCCGT TTCCATGACCATGAACGGCGCCGTGCTGCCCGT GCTGGCTTTCTACATCGTCGCCGCCCAGGAACA GGG | Akkermansia muciniphila (cluster 2) | Methylmalonyl -CoA mutase (spcA) | Propionate |
| 27 | CGCTATCGACACCGTGGAAGATATGAAAGTCCT GTTCGACCAGATCCCGCTGGATAAAATGTCGGT TTCGATGACCATGAATGGCGCAGTGCTACCAGT ACTGGCGTTTTATATCGTCGCCGCAGAAGAGCA AGG | Escherichia coli (cluster 2) | Methylmalonyl -CoA mutase (spcA) | Propionate |
| 28 | TGCAGTAGACTCCATCCTCGACATGGAAATTCT ATTCTCCGGTATCCCTCTTGATCAAATGTCCGT ATCCATGACAATGAACGGCGCCGTATTGCCAGT | Veillonella parvula (cluster 2) | Methylmalonyl -CoA mutase (*spcA*) | Propionate |
| | AATGGCGTTCTACATCTTGGCTGGTGAAGAACA AGG | | | |
| 29 | VGACWCCRTSSWVGAYATGGA | scpA_clust 2_Primer-degenerate_f orward | Methylmalonyl -CoA mutase (*spcA*) | Propionate |
| 30 | TCYTSDSCRGCGAMGATRTAR | scpA_clust 2_Primer_de generate_rev erse | Methylmalonyl -CoA mutase (*spcA*) | Propionate |
| 31 | GAAGARGCKGCTGCNCGTGCACARGCHCGTATC GACW | scpA_Probe | Methylmalonyl -CoA mutase (*spcA*) | Propionate |
| 32 | AAGTTCTTTGCCGGATACCAGCCCTTCGTGAAC ATGTGCGTGGGCGGCGTTACCCGTGAAGGACGC GACGCCACCAACGAGCTGACCTACCTGCTCATG GACGCAGTGCGCCACGTGCGTATTTACCAGCCC TCGCTGGCAACCCGCGTGCACAATTCCTCGCCG CAGGAATACCTCAAGAAAATCGTGGCCGTCATC CGTTCCGGCATGGGCTTCCCCGCCGTGCACTTT GACGACACCCACATCAAGATGATGCTGGCCAAG 32GGCGTGAGCATTGAAGACGCCCGCGACTACT GCCTCATGGGTTGCGTGGAACCGCAGAAAGC | Desulfovibrio desulfuricans | choline trimethylamine -lyase (*cutC*) | trimethylamine-N-oxide (TMAO) |
| 33[1] | TTYGCIGGITAYCARCCNTT | cutC_Primer _degenerate _forward | choline trimethylamine -lyase (*cutC*) | trimethylamine-N-oxide (TMAO) |
| 34[1] | TGNGGYTCIACRCAICGCAT | cutC_Primer _degenerate _reverse | choline trimethylamine -lyase (*cutC*) | trimethylamine-N-oxide (TMAO) |
| 35 | CTGTGCACTTTGACGACACCCACATCAAGATGA TGCTGGCCAAGGGCGTCGACATTGAAGACGCCC GCGACTACTGCCTCATGGGTTGCGTGGAACCGC AAAAATCCGGCCGCCTGTATCAGTGGACGTCCA CGGCATACACCCAGTGGCCCATCTGCATCGAGC TTGTACTCAACCGCGGCGTGCCCCTCTGGTACG GCAAGCAGGTCTGCCCTGACCTCGGCGATCTGA GCCAGTTCGACACTTTTGAAAAGTTTGATGCCG CCGTCAAGGAACAGATCAGGTACATCACCAAAT GGTCAAGCGTGGCTACGGTTATCTCCCAGCGTG TCCACCGCGACCTGGCGCCCAAGCCGCTGATGT CCATCATGTACGAAGGCTGCATGGAAAAAGGCC GCGATGTAGCCGCCGGCGGTGCCATGTACAACT TCGGTCCCGGCGTGATCTGGAGCGGCCTTGCAA CCTACGCCGACTCGATGGCCGCTATCAAGAAGC TCGTGTATGACGACAGGAAGTACACCCTGCAGC AACTTAACGAGGCGCTCAAAGCCAACTTTGAAG GCTATGACGCCATTCTGGCCGACTGCCTGGCCG CGCCCAAATACGGCAACGATGATGACTATGCCG ACGATATCGCCGCCGACCTCATCGCCTTTACCG AGCGTGAGCACCGCAAGTACCGGACGCTCTACT CCATCCTGAGCCACGGTACACTGTCCATTTCCA ACAACACGCCCTTTGGTCAGCTCCTGGGCGCTT CGGCCGGCGGCCGCAAGGCATGGCAGCCGCTTT CCGACGGTATCAGCCCCACGCAGGGCGCGGACT ACAAGGGTCCCACGGCCATCATCAAGAGCGT | Desulfovibrio desulfuricans (cluster 1) | choline trimethylamine -lyase (*cutC*) | trimethylamine-N-oxide (TMAO) |
| | GTCCAAGATGTCCAACGACAACATGAACATCGG CATGGTGCATAACTTCAAGCTCATGCCCGGCCT GCTGGATACCCCCGAAGGCGAACAGGGCATTAT CACCCTCATTCGCTCTGCCAGCATACTTGGCAA CGGTGAAATGCAGTTCAACTATCTGGACAACAA GACCATGCTTGAAGCCCAGCAGAACCCCAAAGA CTATCGCGACCTTGTGGTCCGTGTGGCAGGCTA TAGCGCCTTCTTTGTGGAACTTTGCAAGGACGT GCAGGACGAAATCATCAGCAGAACTGTGC | | | |
| 36 | CCTGCCACTTCGATGACTCCCACATCAAGATGA TGCTGCGCAAAGGCTTCGATTTTGAAGACGCCC GCGATTACTGCCTGATGGGCTGCGTTGAGCCGC AGAAATCCGGGCGCATCTACCAGTGGACCTCCA CCGGCTACACCCAGTGGCCTATTGCTATCGAGT TCGTGCTCAACCGCGGACGGATGGTGCTGTTCG ATAGCTATCAGGGGCTGGATACCGGCGACCTGC GCGACCTGCGCACCTTCGACGAATTCGACGCGG CGGTGAAACAGCAGATCGCCCATATCGTGCGCC TGTCGGCCATCGGCACCGTCATCAGCCAGCGCG TCCACCGCGATGTGGCGCCGAAACCGCTGATGT CGCTGCTGGTGGAAGGCTGCATGGAGAGCGGCA AAGATGTCGCCGCCGGCGGCGCGATGGTCAACC ACGGTCCGGGGCTGATTTTCTCCGGCCTGGCGA CCTACGTCGACTCGATGGCGGCGATCCGCAAGC TGGTGTTCGAAGAGAAGAAATACACCCTTGAGC AGATCCGCGATGCGCTGCTGGCCAACTTTGAAG GTTACGAAGCGCTGCGCCGCGACTGCCTGAACG CGCCGAAATACGGTAACGACGATAACTACGTCG ACCAGTACGCGCTGGATATTACCGAATGGACCG AGAAAGAGTGCCGTAAGTACAAAATGCTCTACT CGACCCTCAGCCACGGCACCCTGTCTATCTCCA ACAACACGCCAATCGGCGAGCTGACCAACGCCA CGCCGAACGGCCGCCTGGCGTGGATGCCGCTCT CCGACGGCATCAGCCCGACTCAGGGCGCGGATA AACAAGGCCCGACGGCGATTATCAAGTCGGTCA GCAAGATGAACGTGGAGACCATGAACATCGGTA TGGTGCACAACTTCAAATTCCTCAAAGGGCTGC TGGATACCCCGGAGGGGCGCCATGGACTGATTA CCTTGCTGCGTACCGCCTCGATTCTCGGCAACG GCCAGATGCAGTTCAGCTATGTCGATAACGAAG TGCTGAAGAAGGCGCAGCAGGAGCCGGAGAAAT ACCGCGACCTGATCGTCCGCGTCGCCGGCTACA GCGCTTACTTCGTCGAGCTGTGCAAAGAGGTCC AGGACGAGATCATCAGCCGTACGGTG | Klebsiella variicola (cluster 1) | choline trimethylamine -lyase (*cutC*) | trimethylamine-N-oxide (TMAO) |
| 37* | CGTGCCACTTTGACGACACGCACATCAAGATGA TGCTCGCCAAGGGCGTGAGCGTCGAGGATGCCC GCGACTACTGCATGATGGGCTGCGTCGAGCCCC AGAAGTCTGGTCGCCTGTATCAGTGGACCTCCA CCTCATACACCCAGTGGCCCATCTGCATCGAGC TCACCCTCAACCACGGCATCCCCCTGTGGTACG GCAAGCAGGTCACGCCTGACTACGGTGACCCTG CGCAGTACAAGACCTACGAGGAGTTTGACGCCG CGGTCAAGAAGACCATCTACTACGTGACCAAGT GGACTGATGTCGCAACCGTGATCTCGCAGCGCG TTGCCCGCGACGTCGCCCCCAAGCCTCTCATGT CCATCATGTACGAGGGGTGCATGGAGCACGGCA AGGACGTCTCGGCCGGTGGCGCTATGTACAACT ACGGCCCCGGCGTCGTGTGGACGGGCCTTGCGA CCTACGCCGACTCCATGGCCGCCATCAAGAAGC TCGTCTTCGACGAGCACAAGTACACCCTCACCC AGCTTGCCGAGGGTCTCAAGGCCGACTTCGAGG GCTACGAGCAGATGCAGCAGGACTGCCTCAACG | Olsenella porci (cluster 1) | choline trimethylamine -lyase (*cutC*) | trimethylamine-N-oxide (TMAO) |
| | CCCCCAAGTATGGCAACGACGATGACTACGCCG ACAGCATCGCGGCAGACATCATCGACTACACCG AGCACATCCACCTGGACTTCAAGACGCTGTACT CGCACATGTCCACCGGTACGCTCTCCATCTCCA ACAACACGCCCTTCGGCGAGATGACGGGCGCCA CGGCGAACGGCCGCAAGGCCTGGACGCCGCTGT CCGACGGCATCAGCCCCACCCAGGGCGCCGACC ACAGGGGACCCACTGCCATCATCAAGTCCGTCT CCAAGCTGTCGAACGATTCCATGAACATCGGCA TGGTTCATAACTTCAAGCTCATGAGCGGCCTGC TCGACACCCCCGAGGGCGAGAACGGCCTCATCA CGCTGCTGCGCACCGCCTCGATGTTCGGCAATG GCGAGATGCAGTTCAACTACCTGGACAACAAGA CGCTCATCGATGCCCAGAAGCACCCCGAGAAGC ACCGCGACCTGGTCGTGCGCGTCGCCGGCTACT CCGCGTTCTTCAACGAGCTCTGCAAGGACGTCC AGGACGAGATCATCAGCCGCACCATG | | | |
| 38* | CCTGCCACTTCGACGACGCCCACATCAAGATGA TGCTGGCAAAGGGCGTGAGCATCGAGGATGCCA GAGACTACTGTCTGATGGGCTGCGTGGAGCCGC AGAAATCGGGCCGTCTCTATCAGTGGACCTCCA CCGCATATACCCAGTGGCCGGTCTGCATCGAAC TGGTGTTAAACCATGGCGTTCCGCTCTGGTACG GCAAACAGGTCTGCCCGGACATGGGAGATTTGA GCCGGTTTAAGACCTTTGAGGAATTTGACGCAG CCGTAAAGGAAGAGATTAAATACGTGACAAAAT GGACCGACGTGGCTACGGTGATTTCCCAGAGAG TCCATAAGGAACTGGCTCCGAAGCCGCTGATGT CGATCATGTACGAGGGCTGTATGGAGAAAGGAA AAGACGTTTCCTCCGGCGGTGCGATGTATAATT ACGGCCCGGGTGTCGTATGGTCGGGTCTCGCCA CCTACACCGACTCCATGGCCGCCATCAAAAAGC TGGTATTTGATGATAAGAAGTACACACTGGAGC AGCTAAACGAAGCGTTAAAAGCGGATTTCGTTG GATACGACCAGATCAAAGCAGACTGCTTAAACG CTCCGAAGTACGGCAACGATGATGATTACGCAG ATCTCATCGCAGCGGATTTAGTTGATTTTACGG AGGCGGAGCACAGAAAATACAAGACGCTGTATT CCCATTTAAGCCACGGCACGCTGTCCATCTCTA ACAATACACCGTTTGGGCAGATGACAGGGGCTT CGGCCAACGGCCGGGGCGCATGGCTCCCGTTAT CCGACGGCATCAGCCCAACCCAGGGTGCGGACT ACAAGGGGCCGACGGCTATCATTAAGTCGGTGT CCAAGATGGCAAACGACAGCATGAACATCGGTC TGGTACATAACTTTAAGATCATGGCAGGCCTCT TAGACACTCCGGAAGGGGAAGAGAGCCTGATCA CCCTGCTCCGTACCGCCAGCATTTTCGGAAACG GCGAGATGCAGTTTAACTATCTGGATAACAATA CGCTCCTGGAAGCTCAGAAGCATCCGGAGAAAT ACCGTGACTTAATCGTCCGGGTAGCCGGATACA GCGCCTTTTTCGTGGAACTCTGCAAGGAGGTTC AGGATGAGATTATCAGCAGAACCATG | Hungatella hathewayi (cluster 1) | choline trimethylamine -lyase (*cutC*) | trimethylamine-N-oxide (TMAO) |
| 39 | CCTGTCACTTCGATGATTCTCATATCAAGATGA TGTTGCGCAAAGGCTTCGACTTTGAAGATGCAC GCGATTACTGCTTGATGGGCTGTGTTGAACCAC AAAAATCAGGTCGTATCTATCAATGGACATCAA CGGGTTATACCCAGTGGCCAATTGCCATTGAAT TTGTATTAAATCGTGGTCGTATGGTGCTGTTTG ATAGCTACCAAGGCTTAGACACCGGCGATTTAC GTGATTTACGTACTTATGAGGATTTCGATCGCG CGGTTAAAGAGCAAGTCGCACATATTATTCGTT TATCAGCAATTGGTACGGTTATCAGCCAGCGCG | Proteus Mirabilis (cluster 1) | choline trimethylamine -lyase (*cutC*) | trimethylamine-N-oxide (TMAO) |
| | TTCACCGTGACATTGCCCCAAAACCACTCATGT CGCTATTGGTTGAAGGTTGTATGGAGCAAGGTA AAGACGTGACAGCGGGTGGTGCGATGGTTAACC ACGGACCGGGTCTGATCTTCTCTGGTTTAGCCA CTTATGTCGATTCTATGGCGGCAATTCGCAAAC TGGTTTATGAAGACAAAAAATATACCTTAGAGC AAATACGTGACGGCTTATTAGCTAATTTTGAAG GGCACGAAGAGTTACTGCGTGACTGTTTAAATG CACCGAAATTTGGTAACGACGATGATGTTGTTG ACCAATATGCGTTAGACATTACCGAATGGACTG AACGTGAATGCCGTAAATATAAAATGCTCTACT CCACATTTAGTCACGGTACGTTATCTATCTCTA ATAACACGCCAATTGGTGAATTAACCGCAGCAA CACCAAACGGTCGTTTAGCATGGAAACCATTAT CAGATGGGATCAGCCCTACACAGGGTGCCGATA AACATGGCCCAACTGCCATCATTAAATCCATCA GCAAGATGAATGTTGAAACCATGAATATCGGTA TGGTTCATAACTTTAAATTCCTCAAAGGCTTGC TAGATACCAATGAAGGACGCCAAGGATTAATTA CCTTATTAAGAACTGCCTCTATTTTGGGTAATG GTCAGATGCAATTTAGCTATGTCGATAATGAAG TACTGAAAAAAGCCCAATTAGAGCCTGAAAAGT ACCGCGATTTAATTGTCCGGGTTGCTGGCTACA GTGCGTACTTCGTTGAATTATGTAAAGAAGTTC AGGATGAAATTATTAGTCGTACAGTGATTGAGA A | | | |
| 40 | ACTTYGAYGAYDCBCACATC | cutC_cluster 1_primer 1_degenerat e_forward | choline trimethylamine -lyase (*cutC*) | trimethylamine-N-oxide (TMAO) |
| 41 | GAYTACTGYHTSATGGGYTG | cutC_cluster 1_primer 2_degenerat e_forward | choline trimethylamine -lyase (*cutC*) | trimethylamine-N-oxide (TMAO) |
| 42* | CACSKSMTAYACCCAGTG | cutC_cluster 1_primer 3_degenerat e_forward | choline trimethylamine -lyase (*cutC*) | trimethylamine-N-oxide (TMAO) |
| 43* | CSAARTWYGGYAACGAYG | cutC_cluster 1_primer 4_degenerat e_forward | choline trimethylamine -lyase (*cutC*) | trimethylamine-N-oxide (TMAO) |
| 44* | TAYGGYAACGAYGATRAYTAYG | cutC_cluster 1_primer 5_degenerat e_forward | choline trimethylamine -lyase (*cutC*) | trimethylamine-N-oxide (TMAO) |
| 45* | CGYTNTCCGACGGYATCAG | cutC_cluster 1_primer 6_degenerat e_forward | choline trimethylamine -lyase (*cutC*) | trimethylamine-N-oxide (TMAO) |
| 46* | CAGGAYGARATYATYAGYMGH | cutC_cluster 1_primer 7_degenerat e_forward | choline trimethylamine -lyase (*cutC*) | trimethylamine-N-oxide (TMAO) |
| 47* | CARCCCATSADRCAGTARTC | cutC_cluster 1_primer 2_degenerat e_reverse | choline trimethylamine -lyase (*cutC*) | trimethylamine-N-oxide (TMAO) |
| 48* | CRTCGTTRCCRWAYTTSG | cutC_cluster 1_primer 4_degenerat e reverse | choline trimethylamine -lyase (*cutC*) | trimethylamine-N-oxide (TMAO) |
| 49* | CRTARTYATCRTCGTTRCCRTA | cutC_cluster 1_primer 5_degenerat e reverse | choline trimethylamine -lyase (*cutC*) | trimethylamine-N-oxide (TMAO) |
| 50* | CTGATRCCGTCGGANARCG | cutC_cluster 1_primer 6_degenerat e reverse | choline trimethylamine -lyase (*cutC*) | trimethylamine-N-oxide (TMAO) |
| 51* | GAAAATCGGTTGATGAATATTGTGAGGACCAGT ACCGGGAAGCCGGGGTATGGGAATTATCCGGGG AATCCTTTGTATCTGACTGCTCCTACCATGCCA TCAACGGCGGCGGTGACTCCAACCCGGGCTATG ATGTGATTCTTATGAAGAAGGGCATGCTGGACA TTCAGCAGGAAGCCAAGGATCATTTAAAAGAAC TGGATTATGAGAATCCGGATGATATTGAAAAGA TTTATTTTTACAAATCCATTATCGATACCACAG AAGGCGTTATGATCTATGCAAAAAGGCTTTCTG AGTATGCAGCGGAGCTTGCGGCAAAGGAAACCA ATCCAAAGCGTAAGGAAGAGCTGTTAAAGATCT CAGAAGTAAATGCTTACGTTCCTGCTCACAAGC CCAGAACCTTCTGGGAAGCGATCCAGGCCGTAT GGACCATTGAATCCCTCCTTGTAGTGGAAGAGA ACCAGACCGGTATGTCCATCGGACGTGTGGATC AGTATATGTATCCATTCTACAAAGACGATATCG AATCCGGCCGGATGAATGATTACCAGGCATTTG AGCTTGCCGGCTGCATGCTGATCAAAATGTCTG AGATGATGTGGATCACCAGCGAAGGCGGTTCCA AATTCTTTGCAGGCTACCAGCCGTTCGTAAACA TGTGCGTAGGCGGTGTGACCCGCAGCGGACATG ATGCCACCAATGATTTAACTTACTTGCTTATGG ATGCCGTCCGTCATGTAAAGATTTACCAGCCGT CTCTTGCATGCCGTATCCACAACAAGTCCCCGA AAAAGTACATGAAAAAGATCGTGGATGTGGTTC GTTCCGGTATGGGATTCCCGGCTTGCCATTTTG ACGACGCCCACATCAAGATGATGCTTGCAAAAG GCGTATCCATTGAAGATGCACGTGATTACTGCC TCATGGGCTGCGTAGAGCCTCAGAAGTCCGGCC GACTGTATCAGTGGACCTCCACTGCTTATACCC AGTGGCCCATCTGCATCGAGCTTGTTTTAAACC ACGGCGCTCCTTTATGGTATGGCAAGCAGGTTT | Clostridium saccharolytic um (cluster 2) | choline trimethylamine -lyase (*cutC*) | trimethylamine-N-oxide (TMAO) |
| | GCCCGGATATGGGTGACTTAAGCAATTTCAAAA CTTATGAAGAGTTTGAAGCTGCCGTAAAAGAAG AGATCAAATACATTACAAAGTGGACCGATGTTG CCACCGTAATTTCCCAGCGGGTTCACAGAGACT TAGCTCCAAAGCCGCTCATGTCCATTATGTACG AAGGCTGTATGGAAAAGGCAAAGGATGTATCTG CAGGCGGCGCCATGTACAACTTCGGACCTGGTG TGGTATGGTCTGG | | | |
| 52* | GCAAATCTGTTGACGAGTACTGCGAGGATCAGT ATCGCGAGGCTGGTGTCTGGGAGCTGTCCGGCG AGTCCTTCGTATCAGATTGTTCCTACCATGCAA TCAACGGCGGTGGAGATTCCAACCCGGGTTACG ACGTGATCCTGATGAAGAAGGGCATGCTTGACA TCCAGAACGAGGCGAAGGAGCATCTGGTAGCAC TTCACTATGACAATCCGGAGGATATCGACAAGA TCTATTTCTATAAGTCGATCATCGAGACGACCG AGGGCGTTATGATCTATGCGAAAAGAATGTCTG AGTACGCAAGAGAGCTTGCTGAGAAAGAGACTG ATCCGAAGAGAAAGGCAGAGCTGTACAAGATCT CTGAGGTCAACGCAAAGGTTCCGGCACACAAAC CGGAGACGTTCTGGGAGGCGATTCAGGCAGTGT GGACAATCGAGTCTCTGCTTGTTGTTGAGGAGA ACCAGACCGGTATGTCCATCGGACGTGTCGATC AGTACATGTATCCGTTCTACAAGGCAGATATCG AGTCCGGACGCATGAACAACTTTGAGGCATTTG AGCTTGCTGGATGTATGCTGATCAAGATGTCTG AGATGATGTGGATTACAAGTGAGGGCGGTTCCA AGTTCTTCGCAGGTTATCAGCCGTTCGTCAATA TGTGCGTCGGCGGTGTGACGAGAGCCGGTCACG ATGCGACAAACGACCTGACCTACCTGCTGATGG ATGCGGTTCGTCACGTAAAGATTTACCAGCCGT CTCTGGCTTGCCGTATTCACAACAAGTCCCCGC GCCGTTATCTGAAGAAGATTGTTGATGTCGTAC GCGCAGGAATGGGATTCCCGGCATGTCACTTCG ATGACACTCACATCAAGATGATGCTCGCAAAGG GCGTTTCGCTCGAGGATGCAAGAGATTACTGCC TGATGGGATGCGTAGAACCGCAGAAGTCCGGTC GTCTGTATCAGTGGACTTCCACCGCTTACACCC AGTGGCCGATCTGTATCGAGCTTGTGCTCAACC ACGGTGTTCCGCTGTGGTACGGCAAGCAGGTTT GCCCGGATATGGGAGATTTAAGCCAGTTCAAGA CTTTCGAGCAGTTTGATGCAGCAGTCAAGGAAG AGATCAAGTACATCACAAAGTGGACGGATGTTG CAACCGTGATCTCCCAGAGAGTTCACCGCGATT TAGCTCCGAAGCCTTTAATGTCCGTTATGTACG AGGGATGTATGGAGCACGGAAAAGACGTATCTG CAGGCGGAGCCATGTATAACTTCGGACCGGGTG TTGT | Enterocloster clostridioform is (cluster 2) | choline trimethylamine -lyase (*cutC*) | trimethylamine-N-oxide (TMAO) |
| 53* | GAAAATCAGTTGATGAATACTGTGAAGATCAGT ATCGTGAAGCTGGAGTATGGGAACTTTCAGGAG AATCCTTTGTTTCAGATTGTTCATACCACGCAG TAAATGGTGGAGGAGACTCTAACCCAGGATATG ACGTTATCTTAATGAAAAAAGGTATGCTTGACA TAAAGAGAGAAGCAGAGGATAAATTAGCTGAGC TTAATTATGAAAATCCAGAAGACATAGACAAAA TCTATTTTTATAAATCATTAATTGATACTGCTG AAGGTGTTATGATATATGCTAAACGTATGTCAG ACTATGCAGCTGAACTTGCTGCAAAGGAAACCA ACCCTAAGCGTAAAGCAGAACTTCAAAAGATTT CTGAAGTAAATGCTAGAGTTCCGGCACATAAAC CAAGTACTTACTGGGAAGCAATTCAATCAGTTT GGACTATAGAATCATTACTTGTAGTTGAAGAAA | Clostridium carboxidivora ns (cluster 2) | choline trimethylamine -lyase (*cutC*) | trimethylamine-N-oxide (TMAO) |
| | ATCAAACAGGTATGTCTATAGGACGTGTTGACC AATACATGTATCCATTCTATAAAGCTGATATTG AAGCTGGACGTATGACTGATTTTGAAGCATTTG AACTATCAGGTTGTATGCTTATAAAAATGTCTG AAATGATGTGGATAACAAGTGAAGGTGGCTCTA AATTCTTTGCAGGTTATCAGCCATTTGTTAACA TGTGTTTAGGTGGTGTTACTAGAGAAGGACGTG ATGCTACAAACGACTTAACTTATCTTTTAATGG ATGCAGTTCGTCATGTTAAAATATATCAACCAT CTTTAGCATGTCGTATACACAAAGCATCACCAC AGAAATACCTTAAGAAAATAGTGGATGTTATCC GTGCAGGTATGGGATTCCCAGCATGTCACTTTG ATGATGTTCATATAAAAATAATGTTAGCTAAAG GTGTTTCTATAGAAGATGCAAGAGATTACTGCT TAATGGGATGCGTTGAACCTCAGAAGGCAGGAA GATTATATCAATGGACTTCTACAGGATATACTC AATGGCCTATATGTATAGAACTTGTTCTTAACC ATGGTGTACCGCTATGGTATGGTAAGCAAGTGT GCCCAAATTTAGGAGACTTAAGCAACTTTAAAA CTTATGAACAGTTTGAAGCTGCTGTTAAAGAGC AAATTAAATACATCACTAAATGGACAAGTGTTG CTACAGTAATTTCTCAACGTGTTCACAAAGATC TTGCACCAAAACCACTTATGTCTATGATGTATG AAGGCTGTATGGAAAAGGGTAGAGGAGTAGAAG CAGGCGGAGCTATGTATAACTTCGGACCTGGAG TAGT | | | |
| 54* | GAAAATCTGTTGATGAATACTGTGAAGACCAGT ACCGTGAAGCAGGTGTTTGGGAACTTTCTGGTG AATCATTTGTTTCAGATTGCTCTTACCATGCAA CTAATGGTGGTGGAGATTCAAACCCTGGATATG ATGTTATCTTAATGAAAAAAGGTATGGCAGATA TTCAGCAGGAAGCAAAGGATCATTTAGCAAAAT TAGATTATGAAAATCCTGAAGATATCGAAAAGA TTTATTTCTATAAATCCGTTATTGATACTACTG AAGGAGTTATGATTTACGCTAAACGTATGTCTG AATATGCTGCTGAATTAGCTGCAAAGGAGACAA ACCCTAAACGTAAGGCAGAATTACAAAAGATTT CAGAAGTTAATGCTCATGTTCCTGCACATAAGC CTAGCAATTTCTGGGAAGCAATTCAATCAGTTT GGACTATTGAGTCATTACTCGTAGTTGAAGAAA ACCAAACTGGTATGTCAATCGGCCGTGTTGACC AATATATGTATCCATTCTATAAGGCAGATATAG AATCAGGACGTATGAATAATTATGAAGCATTTG AATTAGCTGGTTGTATGTTAATCAAAATGTCTG AAATGATGTGGGTTACAAGCGAAGGTGCTTCTA AGTTCTTTGCAGGTTACCAACCATTCGTAAATA TGTGTGTTGGTGGTGTTACTCGTGACGGTCTTG ATGCAACAAATGACTTGACTTATCTTTTGATGG ATGCTGTTCGTCACGTTAAGATTTATCAGCCAT CTTTAGCTTGTCGTATTCACAACAAATCACCTA AGAAGTACTTGAAGAAGATTGTTGATGTTGTTC GTGCTGGTATGGGATTCCCAGCTTGCCATTTTG ATGATGCACATATTAAGATGATGCTTGCAAAAG GTGTTTCCATAGAAGATGCTAGAGATTATTGTA TAATGGGTTGTGTTGAGCCTCAGAAAGCTGGAC GTTTATATCAGTGGACTTCTACAGCTTATACTC AATGGCCTATATGTATAGAATTAGTTCTAAACC ATGGTGTTCCTTTATGGTATGGAAAACAAGTTT GTCCTGATATGGGAGATTTAAATAGATTTAAGA CTTATGAAGAGTTTGATGCTGCTGTTAAGGAAG AAATCAAATATATTACAAAATGGACTGACGTTG CTACAGTTATTTCTCAGCGTGTTCACAGAGATT | Clostridium ljungdahlii (cluster 2) | choline trimethylamine -lyase (*cutC*) | trimethylamine-N-oxide (TMAO) |
| | TAGCTCCAAAACCTTTGATGTCCATCATGTATG AAGGCTGTATGGAACACGGTAAAGATGTTTCTG CCGGTGGTGCTATGTACAATTTCGGACCTGGTG TTG | | | |
| 55* | CDGTTGAYGARTAYTGYGARGAYCAG | cutC_cluster 2_primer 1_degenerat e forward | choline trimethylamine -lyase (*cutC*) | trimethylamine-N-oxide (TMAO) |
| 56* | GTCHATMGGMCGTGTBGAYCARTAYAT | cutC_cluster 2_primer 2_degenerat e forward | choline trimethylamine -lyase (*cutC*) | trimethylamine-N-oxide (TMAO) |
| 57* | ATGTAYGARGGMTGTATGGAR | cutC_cluster 2_primer 3_degenerat e forward | choline trimethylamine -lyase (*cutC*) | trimethylamine-N-oxide (TMAO) |
| 58* | ATGTAYAAYTTCGGACCKGG | cutC_cluster 2_primer 4_degenerat e forward | choline trimethylamine -lyase (*cutC*) | trimethylamine-N-oxide (TMAO) |
| 59* | ATRTAYTGRTCVACACGMCCMATDGAC | cutC_cluster 2_primer 2_degenerat e reverse | choline trimethylamine -lyase (*cutC*) | trimethylamine-N-oxide (TMAO) |
| 60* | YTCCATACAKCCYTCRTACAT | cutC_cluster 2_primer 3_degenerat e reverse | choline trimethylamine -lyase (*cutC*) | trimethylamine-N-oxide (TMAO) |
| 61* | CCMGGTCCGAARTTRTACAT | cutC_cluster 2_primer 4_degenerat e reverse | choline trimethylamine -lyase (*cutC*) | trimethylamine-N-oxide (TMAO) |
| 62 | TGCCCATATCATGCCTGGGCATTCAAACTCGAT GGCAATCTGGCCCATGCACGTAACTGCGAAAAC GTCGCCAATTTCGATAGCGACAAAGCGCAACTG GTTCCGGTGCGTCTGGAAGAATATGCCGGATTC GTCTTCATCAACATGGACCCTAACGCCACCAGC GTAGAAGATCAGTTACCCGGTCTGGGGGCGAAA GTGCTGGAAGCCTGCCCGGAAGTCCACGACCTG AAACTGGCGGCCCGCTTTACCACCCGCACGCCT GCCAACTGGAAGAACATTGTCGATAACTATCTC GAGTGCTATCACTGTGGTCCG | Escherichia coli strain MEI003 | carnitine monooxygena se (*cntA*) | trimethylamine-N-oxide (TMAO) |
| 63 | TGCCCTTACCACGCATGGGCGTTCAAACTTGAC GGCAATCTGGCGCATGCGCGTAACTGCGAAAAT GTCGCCAACTTCGACAGCGAAAAAGCGCACCTT ACACCTGTACGTCTGGAAGAGTATGCCGGATTC GTGTTTATCAACATGGATCCGAACGCGGGCAGC GTGGAAGAACAACTCCCAGGTCTTGCAGACAAA GTTATCGAAGCCTGCCCTGATGTGCATGACCTG AAGCTGGCGGCGCGCTTTACGACCCGAACTCCA GCCAACTGGAAGAATATTGTCGATAACTATCTG GAGTGCTACCACTGTGGCCC | Citrobacter portucalensis | carnitine monooxygena se (*cntA*) | trimethylamine-N-oxide (TMAO) |
| 64 | TGCCCCTATCACGCCTGGGCCTTCAAGCTGGAC GGCAATCTCGCCCACGCCCGCAACTGCGAAAAC GTGGCGAATTTCGATAGCGAAAAGGCAACGCTG GTGCCGGTTCGCCTGGAAGAGTATGCCGGCTTT GTGTTTATCAATATGAATCCGGAAGCGGAGAGT GTGGAAACGCAGCTACCGGGGCTGCAGGACAAA GTGCTGGAAGCCTGTCCGGACGTTCATGATCTG AAACTGGCGGCGCGCTTTACCACCCTCACCCCG GCGAACTGGAAAAATATCGTCGATAACTATCTG GAATGTTACCACTGCGGTCCG | Klebsiella pneumoniae | carnitine monooxygena se (*cntA*) | trimethylamine-N-oxide (TMAO) |
| 65[1] | TAYCAYGCITGGRCITTYAARCT | cntA_primer_ degenerate_f orward | carnitine monooxygena se (*cntA*) | trimethylamine-N-oxide (TMAO) |
| 66[1] | RCAGTGRTARCAYTCSAKRTAGTTRTCRAC | cntA_primer_ degenerate_r everse | carnitine monooxygena se (*cntA*) | trimethylamine-N-oxide (TMAO) |
| 67 | CGAAAAAGTTTTTGCCACATATCCCAATGAATG AGTATCCAGCAGGAGCATTAACGGCTGCTTTAT ATTTGTGTAGCGGTATACGTGCAATGGAGAGAG GTACAATTTCAGAAGGAAGAAATGAAGATGGTA GCGACAAATTGGCTGATATGGAATTAGTGCGTA TAGCAATACCTAGACGAGTGTATTCATTATCTC ATGTTAAATATGCAATTGATAGATTAAAATGGT TGTTTGATAATAGAGAGTTGATACAGGGATTGG AATTTGTTGAAGAACCAGAAAAAT | Eubacterium rectale (cluster 1) | tryptophanase enzyme (*tnaA*) | indoxyl sulphate (IS) |
| 68 | GGGCGTTTCCTGCCCCATATCCCGCAGGAGGAC TATCCCGCCGGGGCTTTGGCGGCGGCTTTCTTC ATCGCCTCCGGCGTGCGCGGCATGGAACGCGGC ACGCTTTCCAGCGTCCGCGACGAAAACGGCAAG GACATTCTGGCGGATGTGGAACTGCTCCGCCTG GCCTTCCCGCGCCGTGTCTTCACATTATCCCAG GTGAAATACGTGGCGGACCGCATGAAGTGGCTC TATGACAACCGCGATTTGATTGGCGGCCTGGAA TTTGTGGAGGAACCGCCCGT | Akkermansia muciniphila (cluster 1) | tryptophanase enzyme (*tnaA*) | indoxyl sulphate (IS) |
| 69 | TTYYTGCCMCATATCCC | tnaA_cluster 1_Primer_de generate_for ward | | |
| 70 | GGTTCYTCMACAAATTCCA | tnaA_cluster 1_Primer_de generate_rev erse | | |
| 71 ** | ATGGAATTACCTTTTGCTGAATCATGGAAAATC AAGATGGTAGAACCCATCAGAAAAAGTACCCGC GAAGAACGCGAGCAATGGATTAAAGAAGCACAT TATAACGTCTTTCAACTCAAATCAGAGCAAGTT TATATTGATCTTATCACCGACTCCGGAACGGGA GCCATGAGCGACCGCCAATGGGCAGGCATGATG CTGGGTGATGAAAGTTATGCCGGTGCCACCTCC TTCTTCAAACTAAAAGATACCATTACAAGAATA ACAGGATTCGAATACATCATTCCTACCCATCAA | Bacteroides thetaiotaomic ron (cluster 2) | tryptophanase enzyme (*tnaA*) | indoxyl sulphate (IS) |
| | GGACGGGCAGCAGAAAATGTTCTTTTCTCTTAT CTGGTACATGAAGGCAATAT | | | |
| 72** | ATGGAATTACCTTTTGCTGAATCATGGAAAATC AAGATGGTAGAACCCATCCGGAAAAGCACACGT GAAGAGCGTGAGCAATGGATTAAAGAAGCACAT TATAATGTCTTTCAACTCAAATCAGAACAAGTT TATATCGATCTCATCACCGACTCCGGCACCGGA GCGATGAGTGACCGTCAATGGGCGGGAATGATG CTAGGTGATGAAAGCTATGCCGGAGCGACCTCA TTCTTCAAGTTGAAAGAGATGATTACGAAACTA ACAGGATTTGAATACATCATACCAACCCATCAG GGACGTGCCGCTGAAAATGTTCTTTTCTCTTAT TTGGTACATGAAGGAGACAT | Bacteroides ovatus (cluster 2) | tryptophanase enzyme (*tnaA*) | indoxyl sulphate (IS) |
| 73** | ATGGAATTACCTTTTGCTGAATCATGGAAAATA AAGATGGTAGAACCCATCCGGAAAAGCACACGT GAAGAACGCGAGCAATGGATTAAAGAAGCACAT TATAATGTCTTTCAACTCAAATCAGAACAAGTT TATATCGATCTCATCACCGATTCCGGAACCGGA GCGATGAGCGACCGTCAATGGGCGGGAATGATG CTAGGCGATGAAAGCTACGCCGGAGCAACCTCT TTCTTTAAGTTGAAAGAGATGATTACGAAACTG ACAGGATTTGAATATATCATACCTACCCATCAA GGACGTGCCGCAGAAAATGTGCTTTTCTCTTAT CTCGTGCATGAAGGAGACAT | Bacteroides xylanisolvens (cluster 2) | tryptophanase enzyme (*tnaA*) | indoxyl sulphate (IS) |
| 74** | CTCAAGGAGGGCGACATCGTACCGGGTAACTCG CACTTTGACACGACAAAGGGGCATATCGAGTTT CGCAAGGCTGTAGCCGTCGACTGCACGATCGAT GCAGCGAGCAACACGGAGCTAGAGCTACCCTTC AAGGGTGAGATGGATCTGAACAAGCTGGAGGAT TGCTTCAAGAAGTATCCCAAGGAGCAGATCCCT ATGTGCGTCCTGACGGTGACCAACAACACGGCT GGCGGTCAGCCTGTCTCTATGAAGAATGTACGT GAGACGAGTGAGCTGTGCCACAAGTACGGCATA CCGCTACAGATCGATGGTGCTCGCTTTGCTGAG AATGCTTACTTCATCAAGATGCGTGAGGAGGGC TATGCGGACAAGTCGATCAAGGAGATCGTCCGT GAGATGCTCAGCTACGCTGACATCATGACCATG TCTAGCAAGAAGGACGCTGTGGTCAACATGGGT GGCTTCCTCGCTATGCGTGACGAGGAGCTCTAT CACAAGTGCTCTATGTACGCTGTGATGAATGAG GGCTTCCTGACCTACGGCGGTATGAACGGTCGT GATATGAACGCGCTCGCTATTGGTCTGGATGAG AACACGGAGTTTGACATGCTCGACACGCGTATC CGTCAGGTGGCTTACCTCGGTAGCAAGCTAGAC GAGTACG | Porphyromon as asaccharolyti ca (cluster 3) | tryptophanase enzyme (*tnaA*) | indoxyl sulphate (IS) |
| 75** | TGCTCAAAGAGGGCGACATCGTGCCGGGCAATT CCCATTTCGACACCACGAAGGGACACATCGAGT TCCGCCGCTGCCACGCCGTGGACTGCACCATCG ACGAGGCGGCCGACACCCAGAAGGAGCTGCCGT TCAAAGGCGAAATGGACATCGCCAAGCTCGAAA AGCTCCTGCGCGAGAACCCGCGCGAGAAAGTTC CGTGCGTGGTGCTGACCATCACCAACAACACGG CGGGCGGACAGCCC | Alistipes finegoldii (cluster 3) | tryptophanase enzyme (*tnaA*) | indoxyl sulphate (IS) |
| 76 | TATTCCGGAACCGTTCAGAATTAAAATGGTAGA ACGTATTAAAATGTTAACACAAGAAGAACGTAT TGAAAAAATCAAAGAGGCTAAGTATAATTTGTT TAATCTTAGGGGAGAAGATGTCTATATTGACCT TCTAACCGACAGCGGAACCAATGCTATGAGCGA TGGCCAATGGGCAGGCGTCATGAGGGGAGATGA AGCATACGCCGGTTCATCCAGTTACTTTAAATT AATTGATGCAGGTAAAGATATATTCGGTTATGA TTATATACAGCCGGTTCATC | Clostridium saccharolytic um (cluster 4) | tryptophanase enzyme (*tnaA*) | indoxyl sulphate (IS) |
| 77 | ATGAGTAAAAAATACGTAGCAGAACCATTCAAA ATCAAAATGGTAGAACCAATCAAAATGACAACA AAAGAGGAAAGAGAAGTAAAGATAGCAAATGCA AATTATAACTTATTTTCATTAAAAGCAGATGAT GTGTACATAGACTTATTAACTGATAGTGGGACT GGTGCTATGAGTTCAAAGCAATGGGCAGCAGTT ATGTTAGGAGACGAGTCATATTCAGGGGCTAGT AGCTACTTTAAATTAGTAGATGCAGCTAAAGAT ATTTTTGGATATAAATATATACAACCAGTACAT CAAGGTAGAGCGGCTGAAAAGGTTTTAATTCCT TTGTTTGCTGAGACTGGGAA | Clostridium sordellii (cluster 4) | tryptophanase enzyme (*tnaA*) | indoxyl sulphate (IS) |
| 78 | RGAACCRTTCARAATYAAA | tnaA_cluster 4_Primer_de generate_for ward | tryptophanase enzyme (*tnaA*) | indoxyl sulphate (IS) |
| 79 | CYGGYTGTATATAWRYATAWCCRA | tnaA_cluster 4_Primer_de generate_rev erse | tryptophanase enzyme (*tnaA*) | indoxyl sulphate (IS) |
| 80 | GCTCAAGATTTCCGAGGTCAA | Cut C_ standard fragment_Pri mer 1 For | Desulvibrio desulficans | 665 bp standard fragment |
| 81 | GCACCAGTTCGATGCAGAT | Cut C_ standard fragment_Pri mer 1_Rev | Desulvibrio desulficans | 665 bp standard fragment (for copy number standard curve) |
| 82 | CAATCTGAGCCCTGACTTTGTA | CntA_ standard fragment_Pri mer 1_For | Desulvibrio desulficans | 700 bp standard fragment (for copy number standard curve) |
| 83 | ACATCGCGATACCACTCAATC | CntA_ standard fragment_Pri mer 1_Rev | Desulvibrio desulficans | 700 bp standard fragment (for copy number standard curve) |
| 84 | AACGTACCACTCGCGCTTAT | tnaA_ standard fragment_Pri mer 1_For | Escherichia Coli | 1348 bp standard fragment (for copy number standard curve) |
| 85 | AGTTTTGCGGTGAAGTGACG | tnaA_ standard fragment_Pri mer 1_Rev | Escherichia Coli | 1348 bp standard fragment (for copy number standard curve) |
| 86 | ACTGAGACACGGCCCA | 16s rRNA_Primer 1_For | Universal | Position 281 (16s rRNA) |
| 87 | TTACCGCGGCMGCTGGCAC | 16s rRNA_Primer 1_Rev | Universal | Position 515 (16s rRNA) |
| 88 | ACTCCTACGGGAGGCAGC | 16s rRNA_Probe | Universal | (16s rRNA) |
| 89 | TCGTCGGCAGCGTCAGATGTGTATAAGAGACAG CCTACGGGNGGCWGCAG | MiSeq_overh ang-D-Bact-0341-b-S-17 MiSeq_overh ang-S-D-Bact-0785-a-A-21 | | |
| 90 | GTCTCGTGGGCTCGGAGATGTGTATAAGAGACA GGACTACHVGGGTATCTAATCC | MiSeq_overh ang-D-Bact-0341-b-S-17 MiSeq_overh ang-S-D-Bact-0785-a-A-21 | | |
| 91 | AGGATTCAGCCCGCAGATGTTCTTTGACGAGAC CAACACCCTGGAAACTCCGGACACTCTTACGGT AGAGAGGATTCATGAGATCGTAGAAGAATTCGG ACGCGGCGCAAGGATGGCTGTTCAGGCTGGATT TGACGCAGTAGAATTCCATGCGGCTCA | Clostridium scindens | Bile acid inducible operon (*baiCD*) | deoxycholic acid |
| 92[2] | CAGCCCRCAGATGTTCTTTG | BaiCD_Prim er degenerate_f orward | Bile acid inducible operon (*baiCD*) | deoxycholic acid |
| 93[2] | GCATGGAATTCHACTGCRTC | BaiCD_Prim er degenerate_r everse | Bile acid inducible operon (*baiCD*) | secondary bile acid deoxycholic acid |
| 94 | GGGCATCCAGCTGTGGCATGGAG | BaiCD_stand ards fragment_pri mer forward | | |
| 95 | CGTGAGGCAGATAACTGTGA | BaiCD_stand ards fragment_pri mer reverse | | |
| 96 | GTCTGACTAACATGCACGGTTCCACCGGCGATA TCGTGCTGCTCGGTACCCAGACTGCCCAGCTGG AAGAAATCTTCCACGAACTGACCCACAACATGA ATGTTGACCTGGGTGGTTCCGGCTCCAACCTGC GTACGCCCGAATCCTGCCTTGGCCAGTCGCGCT GCGAATATGCCTGCTACAACACGCAGGACATGT GCTACACGCTGACCATGGACTATCAGGACGAAC TGCACCGTCCCGCCTTCCCCTACAAGTTCAAGT TCAAGTTCGACGGTTGCCC | Desulfovibrio desulfuricans | dissimilatory-type sulfite reductase subunit alpha (*dsrA*) | Hydrogen sulfide*** |
| 97[3] | CCAACATGCACGGYTCCA | dsrA_Primer _degenerate _forward | dissimilatory-type sulfite reductase subunit alpha (*dsrA*) | Hydrogen sulfide |
| 98[3] | CGTCGAATTGAACTTGAACTTGTAGG | dsrA_Primer _degenerate _reverse | dissimilatory-type sulfite reductase subunit alpha (*dsrA*) | Hydrogen sulfide |
| 99 | CGTGGTTCTGGTCTGACTAAC | dsrA_standar ds fragment primer _forward | dissimilatory-type sulfite reductase subunit alpha (*dsrA*) | |
| 100 | GAAGGACGACATCAAGATCGAC | dsrA_standar ds fragment primer _reverse | dissimilatory-type sulfite reductase subunit alpha (*dsrA*) | |

### FIGURES

The invention is further described by the following figures. These are not intended to limit the scope of the invention but represent preferred embodiments of aspects of the invention provided for greater illustration.

### Brief Description of the Figures

**Figure 1****:** Serum levels of TMAO in subjects before and 24 h after a choline-rich egg meal challenge measured by mass spectrometry (MS).
**Figure 2****:** Abundance and expression of the TMAO-associated gene choline trimethylamine lyase *(cutC)* in subjects before and 24 h after a choline-rich egg meal challenge measured by qPCR using gDNA and by qRT-PCR using RNA.
**Figure 3****:** Abundancy of the SCFA associated *Bcd* gene and of the TMAO associated *CutC* gene in the microbiome of subjects following a vegan diet compared to omnivorous diet.
**Figure 4****:** Schematic representation of decision making for nutritional recommendations based on a patients determined production capacity of a bacterial metabolite in the clinical management of chronic kidney disease (CKD) patients at increased risk of chronic cardiovascular diseases (CVD).
**Figure 5****:** Exemplary application scheme of the method of the present invention for individualized nutritional adjustment in a subject.
**Figure 6****:** Arterial hypertension and systemic inflammation are linked to impaired intestinal barrier function in pediatric chronic kidney disease.
**Figure 7****:** Characteristics of gut microbiota in a cohort of pediatric patients with chronic kidney disease compared to healthy controls.
**Figure 8****:** Stage-dependent activation of plasma tryptophan metabolism activates the Aryl-hydrocarbon receptor.
**Figure 9****:** Correlation network of gut microbiome, clinical parameters, plasma tryptophan metabolites and cytokines.
**Figure 10****:** Monocyte subtypes promote inflammation in chronic kidney disease.
**Figure 11****:** Pro-inflammatory T cell subtypes in chronic kidney disease.
**Figure 12****:** Dendritic cell differentiation in human peripheral blood mononuclear cells
**Figure 13****:** Potential for short chain fatty acid (SCFA) production and its serum levels in children with chronic kidney disease.
**Figure 14****:** Hierarchical gating of mucosa-associated invariant T cells and regulatory T cells.
**Figure 15****:** Systemic levels of TMAO in pediatric CKD.
**Figure 16****:** Microbiome analysis on phylogenetic phylum level.
**Figure 17****:** Phylogenetic tree analysis of 16S amplicon sequencing.
**Figure 18****:** Activity of the aryl hydrocarbon receptor in response to different concentrations of indoxyl sulfate.
**Figure 19****:** Schematic description of CKD-associated alterations of the microbiome, serum metabolome and blood immunome of children with CKD.
**Figure 20****:** Description of the study analyzing metabolite production capacity in patients with CKD.

### Detailed Description of the Figures

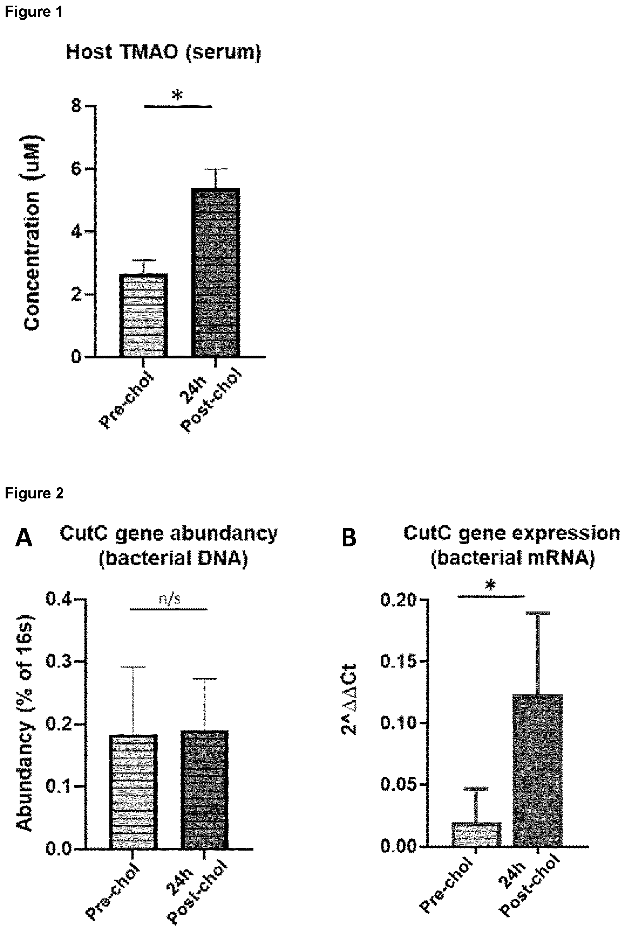

**Figure 1****:** Serum levels of TMAO in subjects before and 24 h after subjection to a choline-rich diet measured by mass spectrometry (MS). Morning fasted blood samples were taken from 3 subjects (pre-choline challenge samples). Subjects then ate a breakfast containing 4 eggs, equivalent to approx. 550mg of dietary choline (a choline-rich egg challenge). Subjects continued with their normal meals but were instructed not to consume any, meat, fish eggs or soya, so that after the egg challenge, subjects consumed no further sources of dietary choline or carnitine for the next 24h. The following morning, after an overnight fast and approx. 24h after the choline-rich egg challenge a blood sample was taken (post-choline challenge sample). All blood samples were centrifuged to obtain serum. The concentration of TMAO in each of the serum samples was measured by mass spectrometry.

**Figure 2:** (A) Abundancy of the TMAO-associated bacterial gene choline trimethylamine lyase *(cutC)* in subjects gut microbiome before and 24 h after the choline-rich egg challenge measured by qPCR using gDNA extracted from stool samples. Bacterial gDNA and RNA were simultaneously extracted from stool samples collected prior to and 24h after consuming the choline-rich egg challenge. The abundancy of the *cutC* gene in the gDNA samples was measured using degenerate primers specific for the *cutC* gene and normalized to the 16s rRNA gene as a readout of the total copies of bacterial genomes in the sample, i.e., the bacterial richness. (B) Expression levels of the TMAO-associated gene choline trimethylamine lyase *(cutC* in subjects gut microbiome before and 24 h after the choline-rich egg challenge. Bacterial RNA extracted from stool samples at the same time as the DNA used in figure (A) was assayed for *cutC* gene expression using 1 step qRT-PCR and degenerated primers specific for the *cutC* gene.

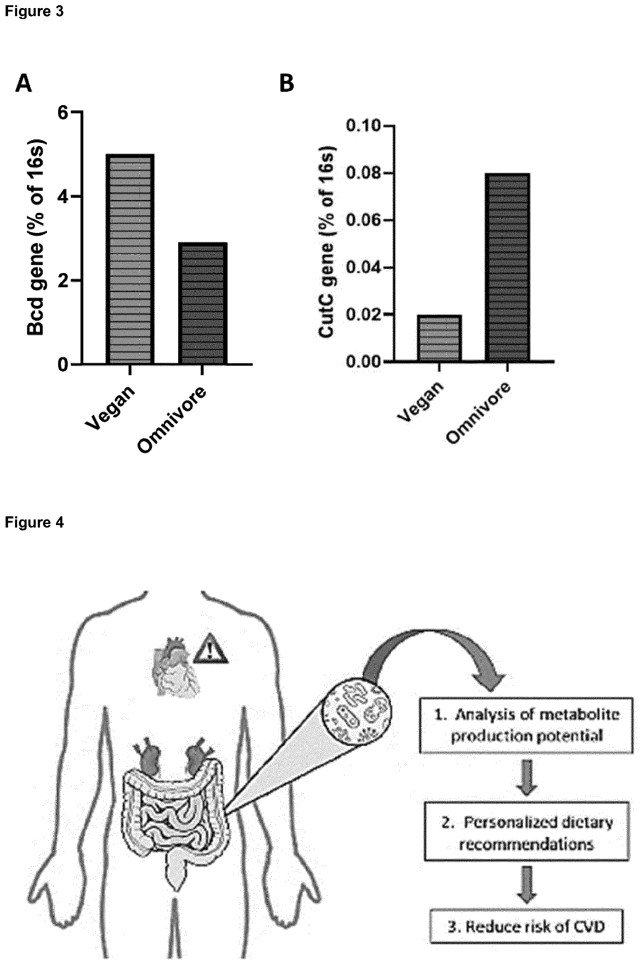

**Figure 3:** (A) Abundancy of the SCFA associated bacterial *Bcd* gene in the gut microbiome of subjects following a vegan or omnivorous diets. Bacterial gDNA was extracted from stool samples provided by healthy subjects following either a vegan diet or an omnivorous diet. The abundancy of the Bed gene in the gDNA samples was measured using degenerate primers specific for the Bed gene and normalized to the 16s rRNA gene as a readout of the total copies of bacterial genomes in the sample. (B) Abundancy of the TMAO associated *cutC* gene in the microbiome of subjects following a vegan or omnivorous diet. As described in figure 3A using degenerate primers specific for the bacterial *cutC* gene.

**Figure 4****:** Schematic representation of decision making to guide nutritional recommendations based on a patients determined production capacity of bacterial metabolites as a component in the clinical management of chronic kidney disease (CKD)

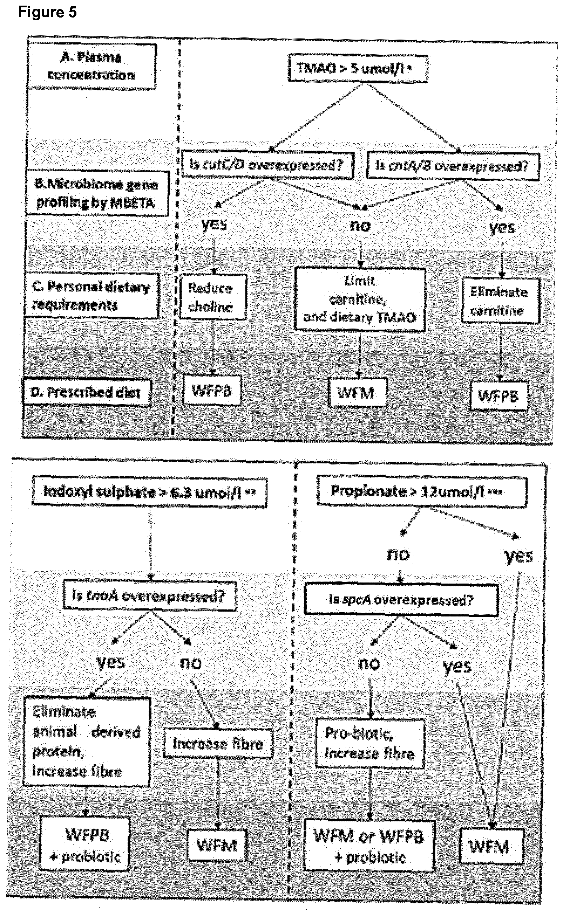

**Figure 5****:** Exemplary application scheme of the method of the present invention for individualized nutritional adjustment in a subject. Levels A to D represent the sequential steps involved in using the method of the present invention in a study or a clinical setting. (A) First plasma concentrations of TMAO, indoxyl sulphate and propionate are determined. (B) Then expression of target genes in the microbiome are analyzed by qRT-PCR assay. (C) Specific dietary requirements are then determined. (D) Individualized diets to optimize microbiome metabolite production can be prescribed. Abbreviations: WFPB=Whole Food Plant Based. WFM=Whole Food Mixed. *upper limit of normal plasma concentration of TMAO in healthy humans (11). ** upper limit of normal plasma concentration of total IS in healthy humans (12). *** mean - 2 S.D measured in healthy humans.

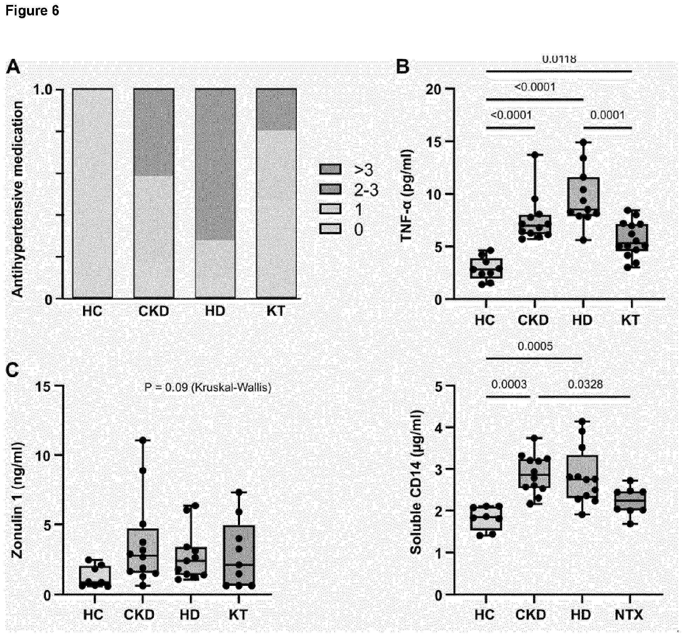

**Figure 6****:** Arterial hypertension and systemic inflammation are linked to impaired intestinal barrier function in pediatric chronic kidney disease. The number of antihypertensive drugs per individual (A, n= 48 patients) is shown in patients with chronic kidney disease (CKD G3-4), hemodialysis (HD), after kidney transplantation (KT) and healthy controls (HC). Plasma TNF-α (B, n=46 patients) was analyzed by chemiluminescence immunoassay. Gut barrier function was assessed using Zonulin 1 and soluble CD14 (C, n=40 patients) ELISA measurements in plasma. Data is shown as a box (median and interquartile range) and whiskers (min-max) with overlaid dot plot. P values ≤ 0.05 are shown, as measured by ordinary one-way ANOVA or Kruskal-Wallis test followed by Tukey's or Dunn's post-hoc correction for multiple comparisons, as appropriate.

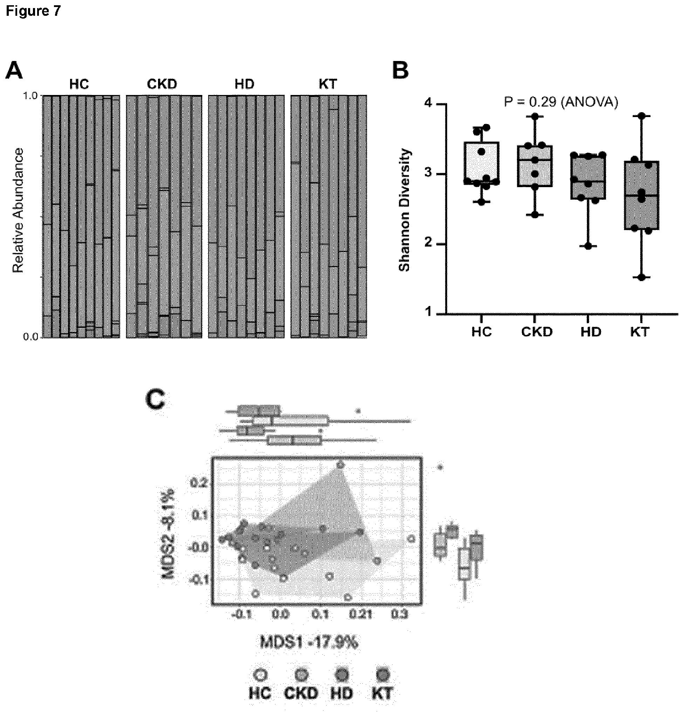

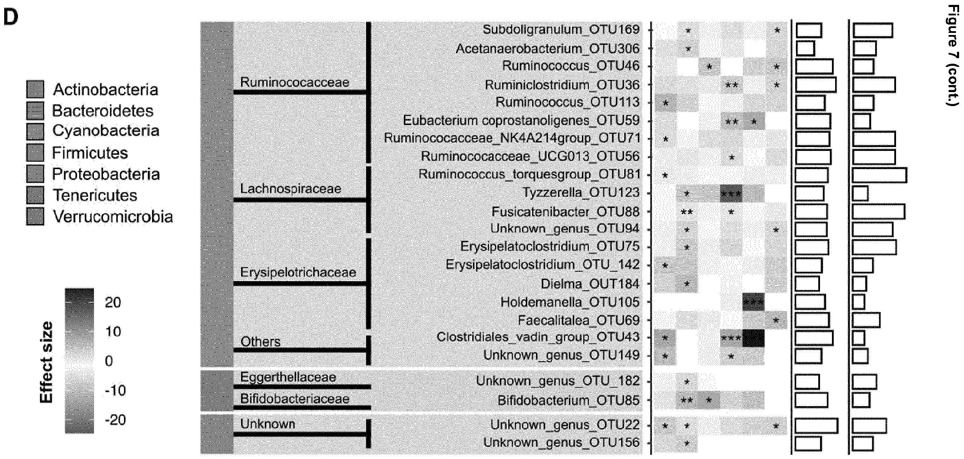

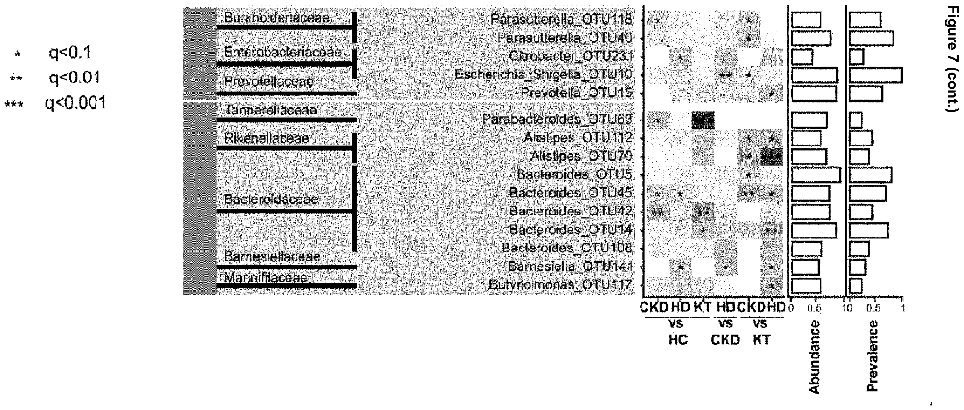

**Figure 7****:** Characteristics of gut microbiota in a cohort of pediatric patients with chronic kidney disease compared to healthy controls. Analysis of gut microbiota from 16S rRNA sequencing in children (n = 32) with chronic kidney disease (CKD G3-4), patients with hemodialysis (HD), patients after kidney transplantation (KT) and healthy controls (HC). A) Relative abundance on phylum level of individuals according to their respective group. B) Alpha diversity as measured by Shannon diversity. Data is shown as a box (median and interquartile range) and whiskers (min-max) with overlaid dot plot. C) Beta diversity assessment by Principal Coordinate Analysis (PCoA) based on Canberra distance (p = 0.01 by PERMANOVA). D) Analyses of group differences on OTU level are shown as a heatmap and their phylogenetic origin is visualized on genus, family and phylum level. Patient groups were tested against each other (pairwise). The heatmap shows significant changes in abundance using DESeq2 v1.30.1 package. Multiple groups of the same genus reported by Lotus due to lacking coverage in available phylogenetic databases are marked by numbers. Bar charts (right) show abundance and prevalence; abundance is calculated as log(genus count)/log(max(genus count)); prevalence for each genus is calculated across the whole data set. All significance estimates were adjusted for multiple tests using Benjamini-Hochberg FDR correction.

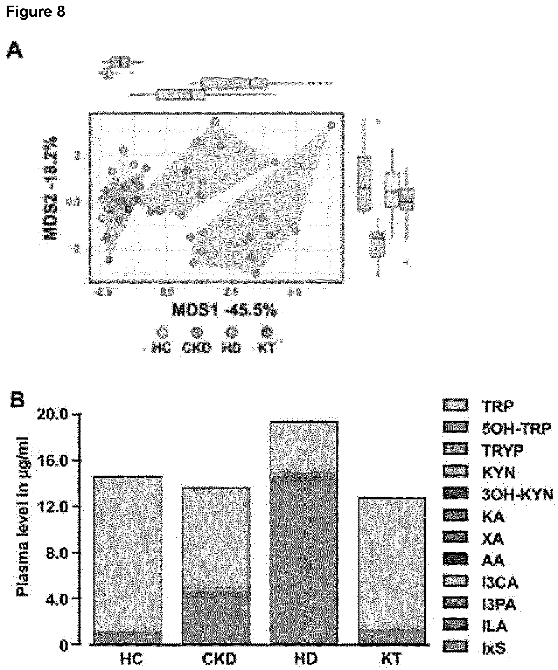

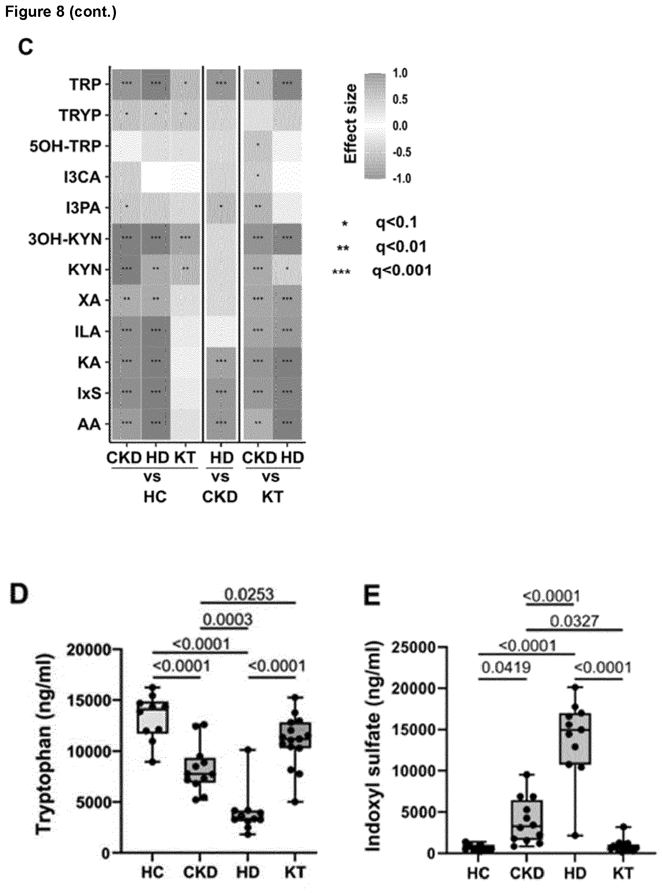

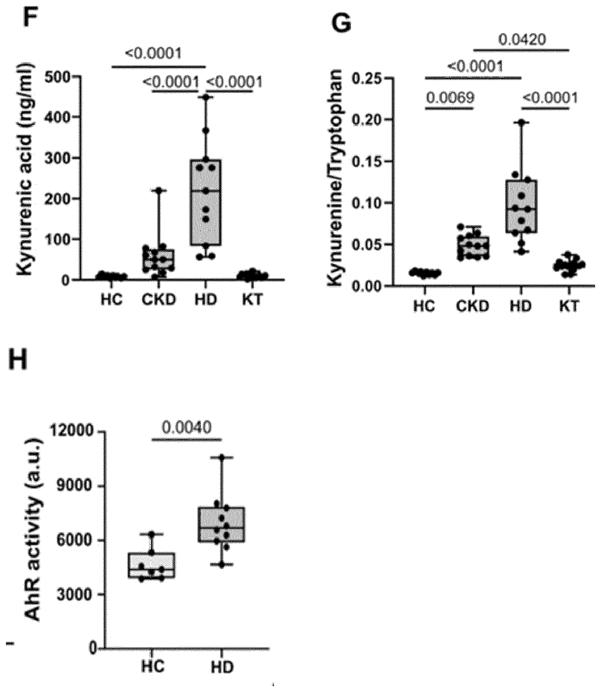

**Figure 8****:** Tryptophan (TRP) and its metabolites were measured in plasma of children at different stages of chronic kidney disease (CKD) compared to healthy controls (n = 48). (A) Multivariate analysis (Principal Coordinate Analysis) of all measured metabolites discriminates between patients with CKD G3-4, patients with hemodialysis (HD), patients after kidney transplantation (KT) and healthy controls (HC). (B) Cumulative load of TRP and its metabolites. (C) Univariate analysis depicted as heatmap shows effect sizes (Cliff's delta) for each pair of patient groups. Colours denote the effect directions (blue-positive and red-negative) and magnitudes (the darker the colour, the stronger the magnitude); asterisks represent the association significance. Statistical significance was assessed by Mann-Whitney U-test and Benjamini-Hochberg false discovery rate correction. Group differences of TRP (D), indoxyl sulfate (IxS, E) and kynurenin acid (KA, F) were further visualized in box plots. The Kynurenine/Tryptophan ratio (G) indicates the activity of tryptophan degradation to kynurenine metabolites. The activity of the Aryl-hydrocarbon receptor (AhR, H) was analyzed using a transfected reporter cell line after 48 h incubation with serum of HC (n=7) and HD (n=10) patients. P values ≤ 0.05 are shown, as measured by ordinary one-way ANOVA or Kruskal-Wallis test and adjusted by post-hoc Tukey's or Dunn's correction for multiple testing (D-G) or by t test (H). Data is shown as a box (median and interquartile range) and whiskers (min-max) with overlaid dot plot.
Abbreviations: TRP= tryptophan, 5OH-TRP= 5-hydroxy-tryptophan, TRYP= 50ryptamine, KYN= kynurenine, 3OH-KYN= 3-hydroxy-kynurenine, KA= kynurenic acid, AA= anthranilic acid, XA= xanthurenic acid, IxS= indoxyl sulfate; I3CA= indole-3-carboxyaldehyde, I3PA= indole-3-propionic acid, ILA= indole lactate, AhR= aryl hydrocarbon receptor.

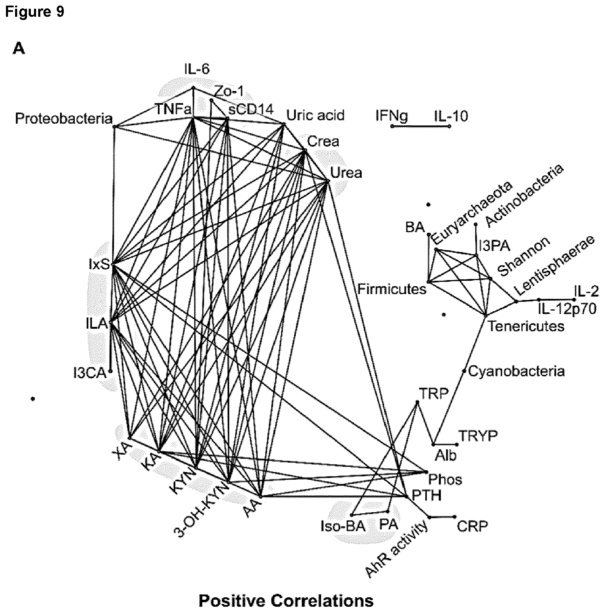

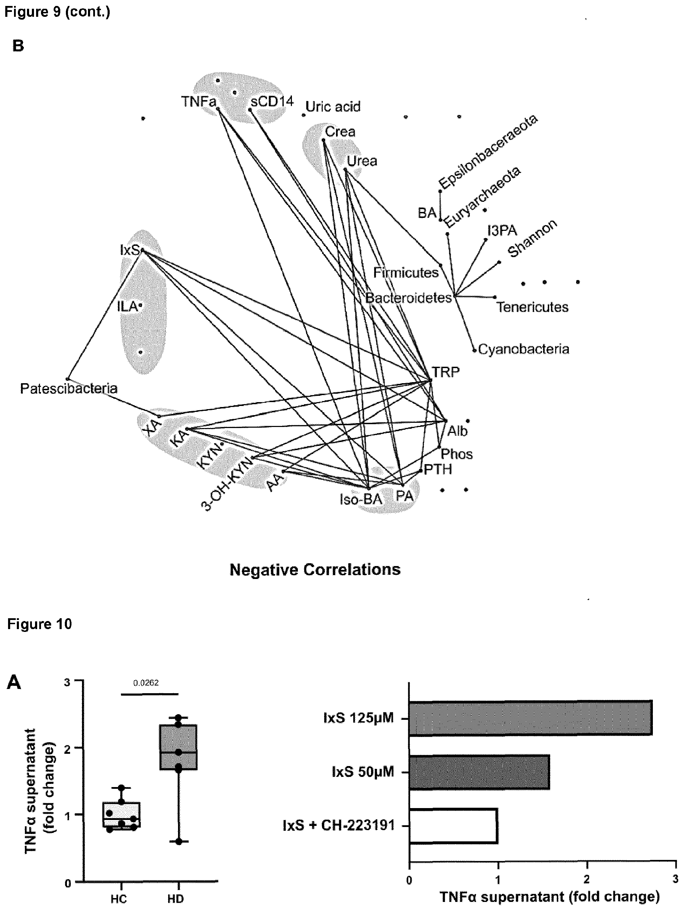

**Figure 9****:** Correlation network of gut microbiome, clinical parameters, plasma tryptophan metabolites and cytokines. Laboratory parameters, tryptophan metabolites (n = 48), cytokines (n=46) and taxonomic data (n = 32) were associated using pairwise Spearman correlations and adjusted for multiple testing using the Benjamini-Hochberg FDR correction. Edges for which absolute rho > 0.3 and Q < 0.1 are visualized. For better visualization eGFR was removed as creatinine and urea convey similar information. A) Positive correlations. B) Negative correlations.
Abbreviations: TRYP= tryptamin, TRP= tryptophan, KYN= kynurenine, KA= kynurenic acid, 3OH-KYN= 3-hydroxy-kynurenine, AA= anthranilic acid, XA= xanthurenic acid, PA= propionic acid, BA= butyric acid, Iso-BA= isobutyric acid, Zo-1= zonulin-1 , sCD14= soluble CD14, IxS= indoxyl sulfate, ILA= indole lactate, I3CA= indole-3-carboxyaldehyde, I3PA= indole-3-propionic acid, CrP= C-reactive protein. Crea= creatinine, phos= phosphate, Alb= albumin.

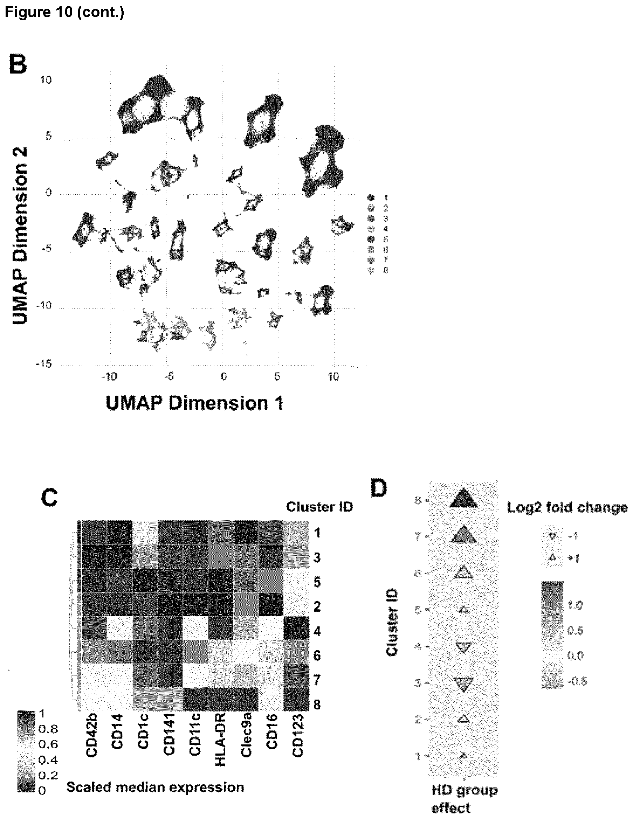

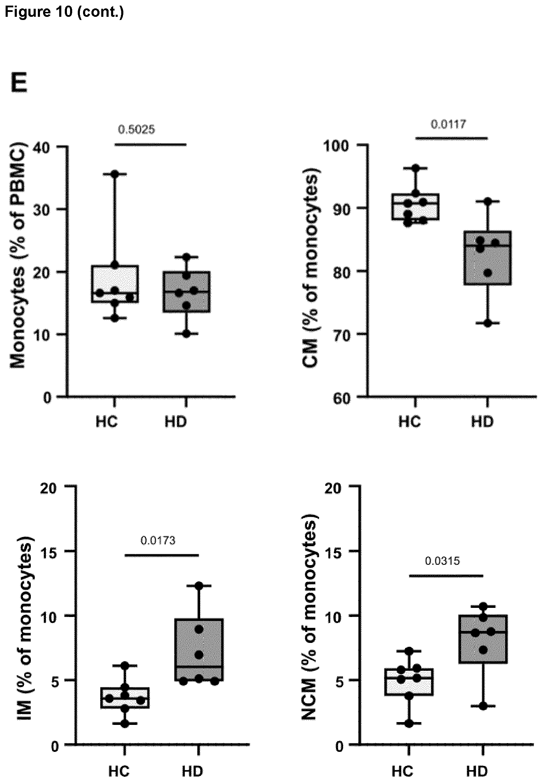

**Figure 10****:** Monocyte subtypes promote inflammation in chronic kidney disease. Monocytes isolated from healthy donors were incubated with serum from hemodialysis patients (HD, n=7) and healthy controls (HC, n=7). Monocytes were incubated with indoxyl sulfate (IxS) in presence or absence of the AhR antagonist CH-223191 (10µM). TNF-α was measured in the culture supernatant after 24 hrs incubation using ELISA (A). Peripheral blood mononuclear cells (PBMC) were isolated from HD (n=6) and HC (n=7) individuals for surface staining and multi-color flow cytometry was performed. Unsupervised clustering by FlowSOM revealed 8 different cell clusters (B) characterized by the differential expression of 9 surface marker describing myeloid and dendritic cells (C). Cuneiform plots depict the log2-fold changes for these clusters between HD and HC (indicated by color, size and directionality of the triangles, D). Classical hierarchical gating of total monocytes, classical monocytes (CD16+), non-classical (CD14+), and intermediate (CD14+CD16+) monocytes is shown in e).
Abbreviations: AhR= aryl hydrocarbon receptor.

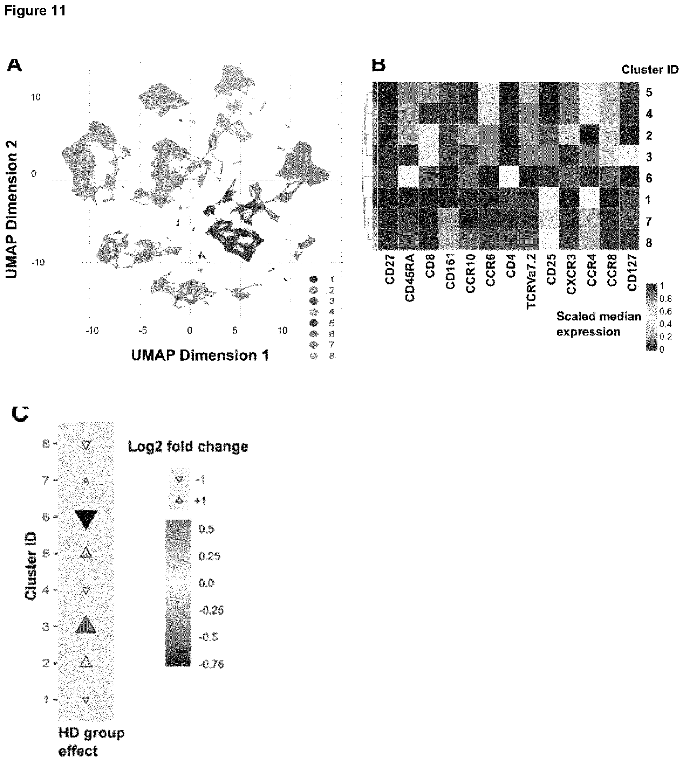

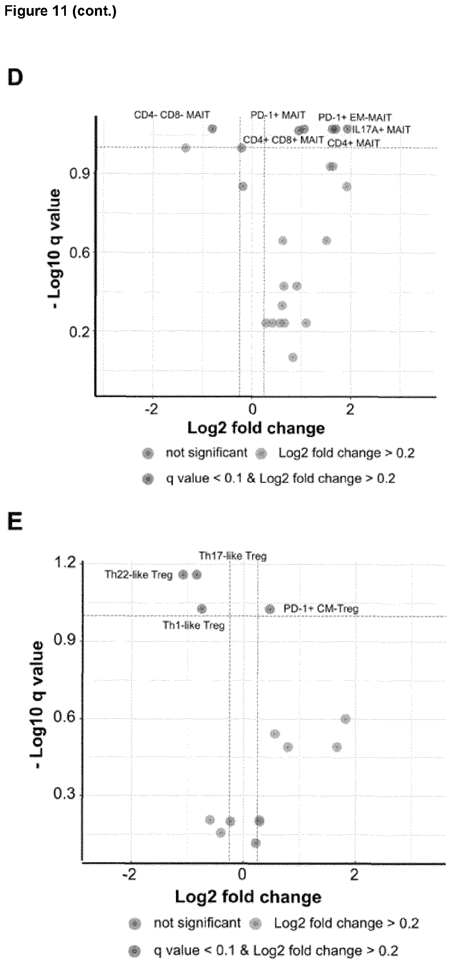

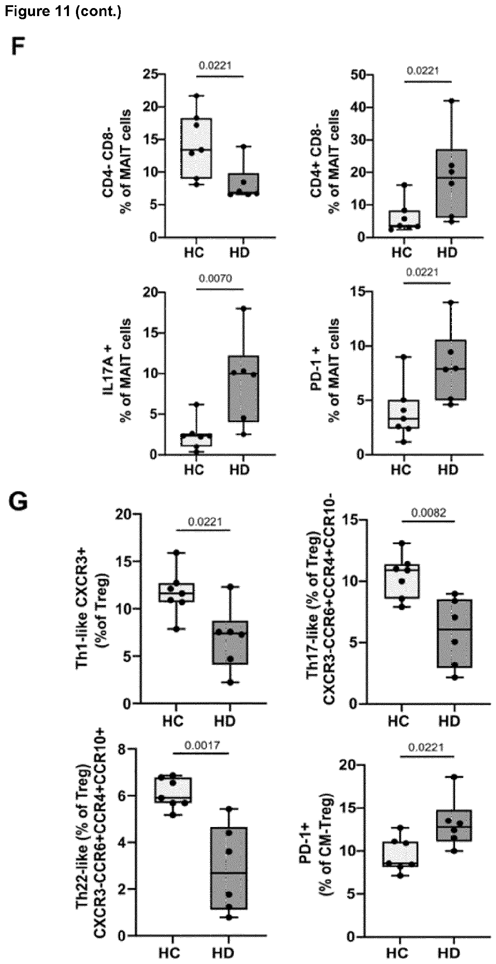

**Figure 11****:** Pro-inflammatory T cell subtypes in chronic kidney disease. Unsupervised clustering of HD (n=6) and HC (n=7) individuals by FlowSOM revealed 8 T cell clusters (A) based on the differential expression of surface marker (B). C) Cuneiform plots showing the log2-fold changes for these clusters between HD and HC (indicated by color, size and directionality of the triangles). Volcano plot of MAIT (D) and Treg (E) subpopulations by hierarchical gating, y-axis indicates Q value by Mann-Whitney U-test and Benjamini-Hochberg false discovery rate correction, x-axis log2-fold change between HD and HC. Significantly altered subpopulations are depicted as box (median and interquartile range) and whiskers (min-max) with overlaid dot plots for MAlT and Treg in F) and G), respectively. For f and g, *P* values ≤ 0.05 are shown, as measured by t test or Mann-Whitney-U test as appropriate.
Abbreviations: MAIT= mucosa-associated invariant T cells; Treg= regulatory T cells, HD= hemodialysis, HC= healthy controls.

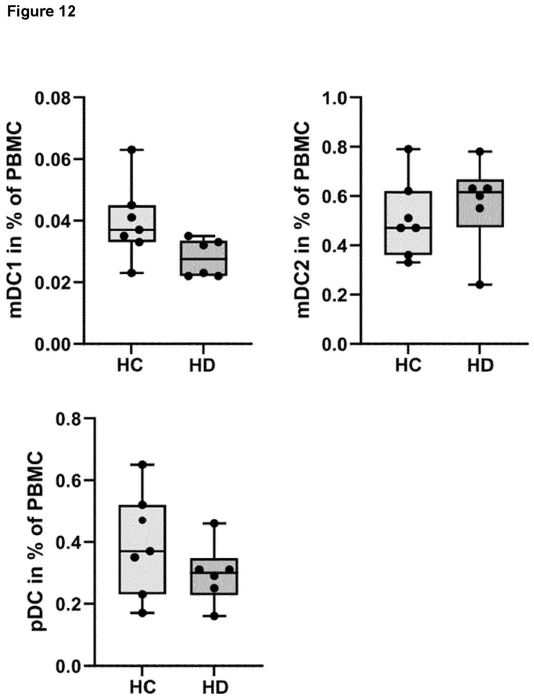

**Figure 12****:** Dendritic cell differentiation in human peripheral blood mononuclear cells. The relative abundance (in % of peripheral blood mononuclear cells (PBMC)) is shown for myeloid dendritic cells type 1 (mDC1: HLA-DR+CD141+CleC9a+), mDC2 (HLA-DR+, CD1c+, CD11c+) and plasmacytoid DC (pDC: HLA-DR+, CD123+) in HD (n=6) and HC (n=7) individuals. P values ≤ 0.05 are shown as analyzed by t test or Mann-Whitney-U test. Abbreviations: CKD= chronic kidney disease, HD= hemodialysis, KT= kidney transplantation, HC= healthy controls.

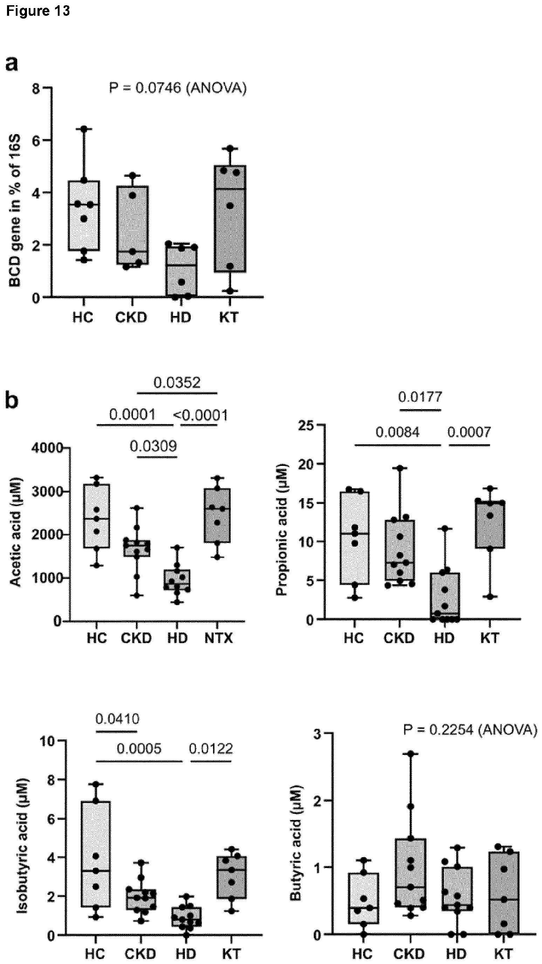

**Figure 13****:** Potential for short chain fatty acid (SCFA) production and its serum levels in children with chronic kidney disease. The abundance of the butyrate associated gene Butyryl-CoA-Dehydrogenase (BCD) was analyzed in fecal samples (n=24) showing a lower abundance in patients of the HD group (a). Systemic levels of the SCFA acetate, propionate, isobutyrate and butyrate were analyzed in serum samples (n=36, b). P values ≤ 0.05 are shown, as measured by ordinary one-way ANOVA or Kruskal-Wallis test and adjusted by post-hoc Tukey's or Dunn's correction for multiple testing. Data is shown as a box (median and interquartile range) and whiskers (min-max) with overlaid dot plot. Abbreviations: CKD= chronic kidney disease, HD= hemodialysis, KT= kidney transplantation, HC= healthy controls.

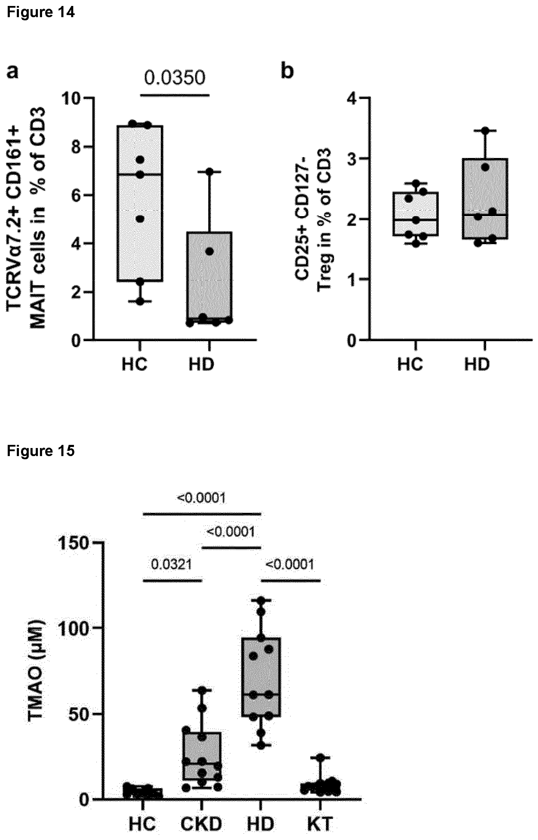

**Figure 14****:** Hierarchical gating of mucosa-associated invariant T cells and regulatory T cells. The relative abundance (in % of CD3+ T cells) is shown for mucosa-associated invariant T (MAIT) cells, defined as CD161+TCRVa7.2+ (a) and regulatory T cells (Treg), defined as CD25+CD127-in HD (n=6) and HC (n=7) individuals. P values ≤ 0.05 are shown as analyzed by t test or Mann-Whitney-U test.

**Figure 15****:** Systemic levels of TMAO in pediatric CKD. Trimethylamone-N-oxide (TMAO) was measured in plasma samples (n=46) of children at different stages of chronic kidney disease (CKD G3-4), hemodialysis (HD) and patients after kidney transplantation (KT) compared to healthy controls (HC). P values ≤ 0.05 are shown, as measured by ordinary one-way ANOVA and adjusted by post-hoc Tukey's correction for multiple testing. Data is shown as a box (median and interquartile range) and whiskers (min-max) with overlaid dot plot.

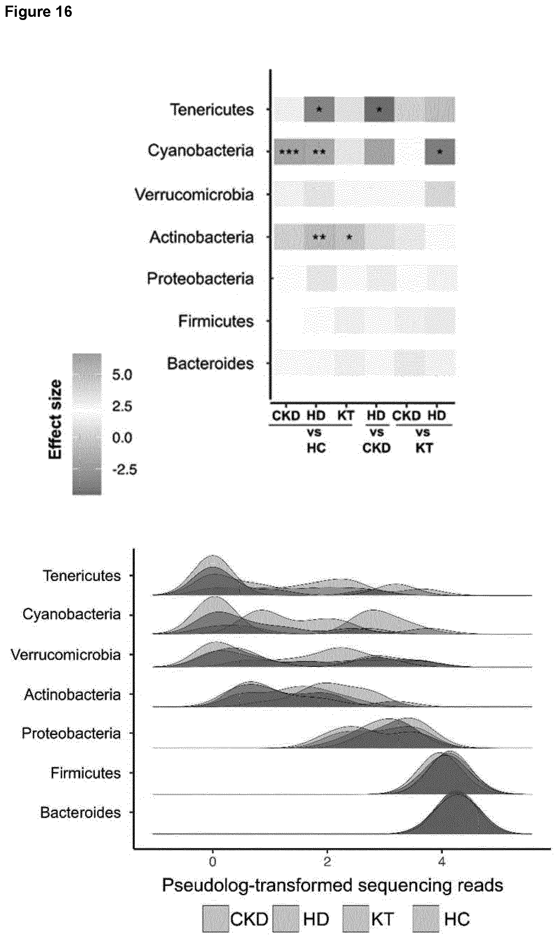

**Figure 16****:** Microbiome analysis on phylogenetic phylum level. Analysis of gut microbiota from 16S rRNA sequencing in children (n = 32) with chronic kidney disease (CKD G3-4), patients with hemodialysis (HD), patients after kidney transplantation (KT) and healthy controls (HC). Analyses of group differences on phylum level are shown as a heatmap. Patient groups were tested against each other (pairwise). The heatmap shows significant changes in abundance using DESeq2 v1.30.1 package. All significance estimates were adjusted for multiple tests using Benjamini-Hochberg FDR correction. In addition, phylum level abundance is visualized as density plots, stratified by disease status. x-axis shows Iog10 (relative abundance) of each phylum, y-axis the phylum annotation provided by LotuS.

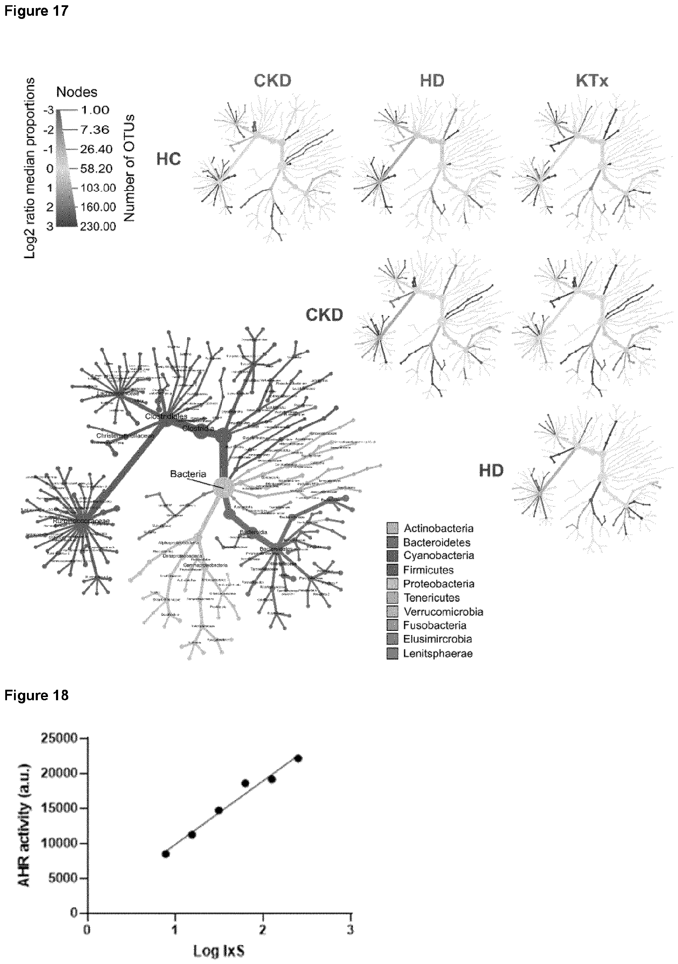

**Figure 17****:** Phylogenetic tree analysis of 16S amplicon sequencing. Phylogenetic analysis from 16S rRNA sequencing in children (n = 32) with chronic kidney disease (CKD G3-4), patients with hemodialysis (HD), patients after kidney transplantation (KT) and healthy controls (HC). Phylogenetic tree was constructed using the metacoder R package. Differences between groups are shown as log2 median ratio. Top right panels show group to group comparisons, bottom right tree indicates taxonomic annotations, node size indicates number of OTU per node. Not annotated nodes were not labeled.

**Figure 18****:** Activity of the aryl hydrocarbon receptor in response to different concentrations of indoxyl sulfate. Transfected HT29 reporter cells were incubated with different concentrations of indoxyl sulfate (IxS) for 24 hours. Subsequently, the luciferase activity was measured indicating the activity of the aryl hydrocarbon receptor (AhR).

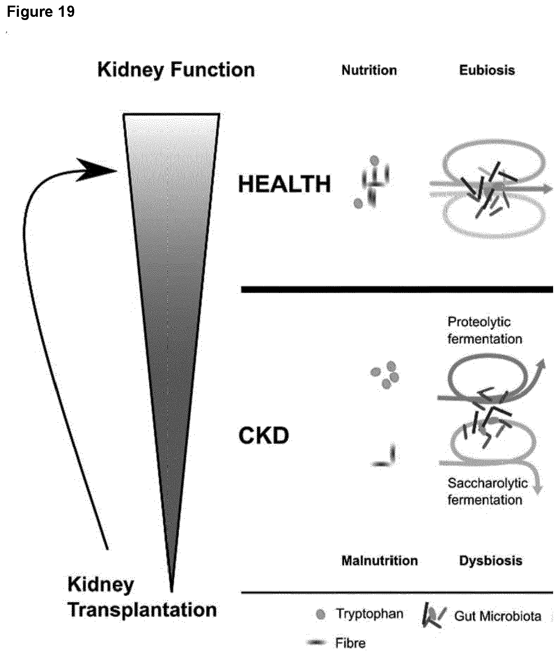

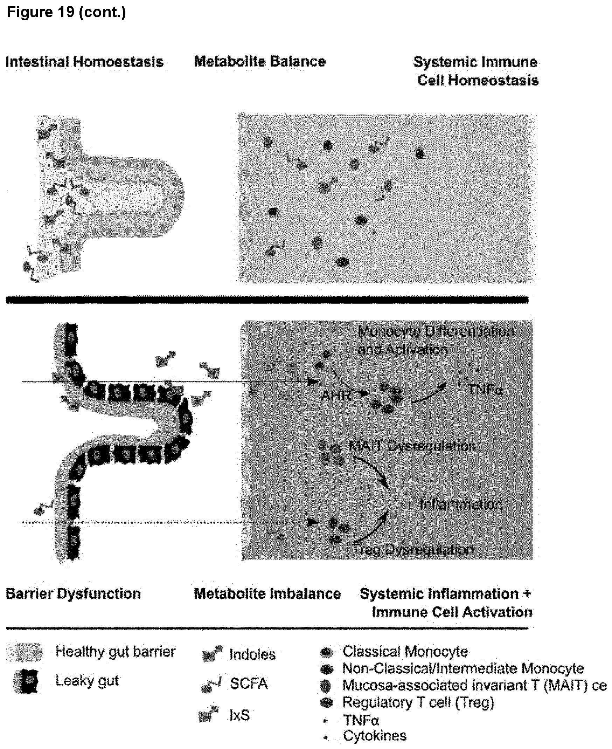

**Figure 19****:** Schematic description of CKD-associated alterations of the microbiome, serum metabolome and blood immunome of children with CKD. Pediatric patients with CKD stage-dependently develop a leaky gut barrier and systemic imbalance of microbiome-derived metabolites. CKD patients exhibit a reduction in SCFA and an increase of indole metabolites of bacterial origin due to alterations of the taxonomic composition of the gut microbiome and nutritional alterations. These changes are associated with increased serum TNF-α levels, AhR-dependent secretion of TNF-α from monocytes and a shift from classical to intermediate and non-classical monocytes. Additionally, CKD patients show a dysregulation of MAIT and Treg subsets. Kidney transplantation leads to a partial normalization of these dysregulated features.

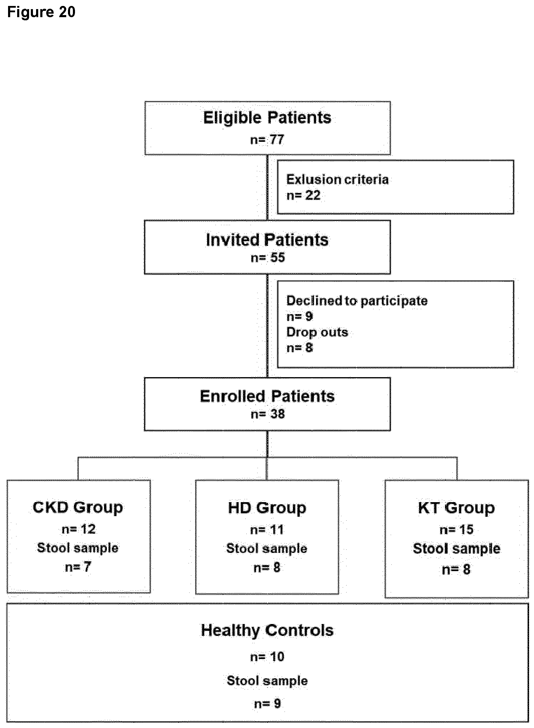

**Figure 20****:** Description of the study analyzing metabolite production capacity in patients with CKD. From 77 patients eligible for this study, 55 patients were invited to participate (22 were excluded as they met exclusion criteria). 9 patients or their parents declined to participate and 8 patients were excluded during the recruitment for other reasons (antibiotic treatment, lost to follow-up, difficulty in blood drawing). Moreover, 10 healthy individuals with normal kidney function, treated at the hospital for reasons other than kidney disease, were enrolled to this study. Abbreviations: CKD = chronic kidney disease; HD = hemodialysis; KT = kidney transplantation.

### EXAMPLES

### Example 1:

### Gene target selection and identification of bacteria:

The gene butyryl-CoA-dehydronase *(Bcd)* encoding for an essential enzyme of bacterial butyrate production was identified from scientific literature searches. The bacteria containing Bed and which are responsible for the majority of butyrate production in the microbiota were identified by curation of the baseline 109 bacterial species of the human gut microbiota with genome data from the databases of the National Center for Biotechnology Information (NCBI). If a bacterium was found to be over 1% represented in the baseline microbiota and previously shown to produce butyrate it was included as a candidate. Fourteen Bed containing bacteria were identified fitting these criteria. The specific gene and protein sequences were retrieved from the NCBI database. Partial sequences, unclassified strains, theoretical protein sequences and those with insufficient annotation were disregarded. In total 8 *Bcd* bacterial genomes were selected for assay development. A similar approach was used to identify target genes and key bacteria for propionate production. Previous studies have shown that the succinate pathway is the dominant pathway for propionate production in the gut microbiome. Therefore, genes for methylmalonyl-CoA decarboxylase (*mmdA*) and methylmalonyl-CoA mutase (*spcA*) were selected as potential targets for propionate production. *mmdA* was subsequently de-selected due to insufficient annotation in the NCBI database

### Example 2:

### Influence of targeted dietary changes over 24 h on the abundance and expression of the TMAO-associated gene choline trimethylamine lyase (cutC)

In a small cohort of healthy volunteers, the influence of a targeted dietary meal challenge on the abundance and expression of the TMAO-associated gene (*cutC*) was investigated and compared with the TMAO serum levels (Figures 1 to 2). In brief, fasted blood and stool samples were collected from subjects prior to (pre-choline)- and 24 hours after (post-choline) a choline-rich meal comprising 4 eggs (choline-rich egg challenge). The concentration of serum TMAO was measured in the pre- and 24h post-choline blood samples (Figure 1). Bacterial DNA and RNA were isolated form the subjects pre- and post-choline stool samples and the *cutC* gene abundancy and expression was determined using qPCR (DNA) and RT-qPCR (RNA (Figure 2), respectively. The experiments clearly showed that the choline-rich egg meal challenge led to a higher TMAO serum level in the blood. The analysis of the gene targeting qPCR data show that a single choline rich meal does not effect the abundancy of the *cutC* gene (Figure 2A) but does induce a significant increase in the expression of the *cutC* gene (Figure 2B). These data can help to unveil the mechanisms of microbial metabolite production in response to dietary precursors. An area which is to date not fully understood.

### Example 3:

### Influence of vegan and omnivorous diets on the abundance of the butyrate- and TMAO-associated genes butyrate-CoA-dehydrogenase (Bcd) and choline trimethylamine-lyase (cutC).

The gene abundancy of the butyrate associated gene *(Bcd)* and the TMAO associated gene (*CutC*) were compared in subjects following a vegan diet and an omnivorous diet using primers SEQ ID 10, 13, 33 and 34. The quantitative PCR analysis of bacterial DNA extracted from the subjects stool samples showed that a vegan diet is associated with higher *Bdc* and lower *cutC* whereas the opposite gene profile is observed in the omnivorous subject (low *Bcd* and high *cutC*)*.* See figures 3A and 3B.

### Example 4:

### Metabolite production and gene abundancy in patients with chronic kidney disease (CKD)

### Background:

Chronic kidney disease (CKD) is characterized by a sustained pro-inflammatory response of the immune system, promoting hypertension and cardiovascular disease. To analyze the underlying mechanisms linked to gut dysbiosis the fecal microbiome in terms of abundancy of bacterial genes, serum metabolite levels and immune phenotypes in children (normal kidney function, CKD stage G3-G4, G5 treated by hemodialysis (HD) or kidney transplantation) with a mean age of 10.6 ± 3.8 years was analyzed.

### Patients:

Patients were recruited from the Department of Pediatric Gastroenterology, Nephrology, and Metabolic Diseases at Charité University hospital in Berlin, Germany. Written informed consent was obtained from all participants and/or their parents prior to study entry. The study was approved by the local Ethical Review Board (EA2/162/17). All procedures performed were in accordance with the ethical standards of the institutional and national research committees and the 1964 Helsinki declaration and its later amendments or comparable standards.

Patients (age 3-18 years) were enrolled in the following groups:
- CKD group: CKD stage G3-G4, estimated GFR (eGFR) 15-60 ml/min*1.73m²
- HD group: CKD stage G5D, patients on maintenance hemodialysis, enrolled earliest four weeks after initiation of HD
- KT group: patients after successful KT, earliest four weeks after KT, without a history of rejection or chronic graft failure, eGFR > 60ml/min*1.73m²
- HC group: normal kidney function, admitted to the hospital or outpatient department for reasons other than kidney disease, not meeting the exclusion criteria

All individuals with a body weight below 15kg, acute or chronic inflammatory diseases, fever, diabetes, chronic liver disease, inflammatory bowel disease, or other gastrointestinal disorders (constipation, diarrhea, short bowel syndrome) were excluded from the study. Patients with antibiotic prophylaxis or treatment within the four weeks prior to recruitment were excluded.

At the time of enrolment, baseline demographic (age, gender, diagnosis, body weight and height) and clinical data from all patients were obtained. eGFR was calculated according to the bedside formula of Schwartz based on serum creatinine (Schwartz *et al.,* 2009), percentiles of weight and BMI were determined according to national references (Rosario *et al.,* 2010). Office systolic and diastolic blood pressures were documented as an average of three oscillometric measurements using local devices and normalized to national references (Lurbe *et al.,* 2016). Arterial hypertension was defined as blood pressure values above the 95^{th} percentile.

### Clinical assessment, biobanking and routine laboratory measurements:

Heparinized blood specimens were collected as part of routine laboratory sampling and used for the measurement of creatinine, urea, uric acid, phosphate, albumin, C-reactive protein (CrP), parathyroid hormone (PTH), and triglyceride levels. Serum, EDTA and Heparin plasma were stored at -80°C until further use. For the measurement of TNF-α, IL-2, IL-4, IL-6, IL-8, IL-1β, IL-10, IL-13, IL-12p70, and IFN-y, plasma was analyzed using the Meso Scale Discovery (MSD) V-PLEX Plus Proinflammatory Panel 1 (human) according to the manufacturer's protocol. All samples were run on a single plate on an MSD plate reader (model 1250). Zonulin-1 (Zo-1) and soluble CD14 (sCD14) were analyzed in patients serum using the human Zo-1 ELISA kit (Biomatik Corporation, Canada) and sCD14 Quantikine ELISA kit (R&D Systems, USA) according to manufacturer's protocol. Peripheral blood mononuclear cells (PBMC) were isolated by density gradation using Pancoll (Pan Biotech, Germany) using standard protocols and stored in liquid nitrogen until usage.

Stool specimens (2-4g) were collected (Sarstedt, Germany; #80.623.022) and stored for 24 hours at 4 - 8°C max. and transferred to the study center for freezing at -80°C until further use. Patients and parents were provided with detailed information about collection, storage and transport of stool specimens.

### Generation of 16S rRNA Amplicon Libraries and Sequencing:

Stool DNA was isolated using QIAamp^{®} Fast DNA Stool Mini Kit (QIAGEN, Hilden, Germany) according to manufacturer's protocol. The extracted DNA was used as a template to amplify the V3-V4 region of the bacterial 16S rRNA gene using fusion primers
TCGTCGGCAGCGTCAGATGTGTATAAGAGACAG-CCTACGGGNGGCWGCAG (MiSeq_overhang-D-Bact-0341-b-S-17) and
GTCTCGTGGGCTCGGAGATGTGTATAAGAGACAG-GACTACHVGGGTATCTAATCC (MiSeq_overhang-S-D-Bact-0785-a-A-21) including bacteria targeting primers (Klindworth *et al.,* 2013). The PCR reaction mixture with a total volume of 25 µl contained 12,5 µl KAPA HiFi HotStart ReadyMix (Roche), 1,25 µl of each primer (10 nM) and 3 ng/µl of isolated DNA. Thermal cycling scheme for bacterial amplicons was as follows: initial denaturation for 3 min at 95°C, 25 cycles at 95°C for 30 s, 30 s at 55°C, and 30 s at 72°C and a final extension at 72°C for 5 min. For each sample, three independent 16S amplicons were generated. The resulting PCR products were purified with Agencourt AMPure XP magnetic beads (Beckman coulter, Krefeld, Germany) and then quantified with Quant-iT dsDNA HS assay kit and a Qubit fluorometer (Invitrogen GmbH, Karlsruhe, Germany) following the manufacturer's instructions. The three individual, purified V3-V4 PCR products per sample were pooled and used to attach indices and Illumina sequencing adapters using the Nextera XT Index kit (Illumina, CA, USA). Index PCR was performed using 5 µl of template PCR product (each 2 ng/µl), 2.5 µl of each index primer, 12.5 µl of 2× KAPA HiFi HotStart ReadyMix and 2.5 µl PCR grade water. Thermal cycling scheme was as follows: 95°C for 3 min, 8 cycles of 30 s at 95°C, 30 s at 55°C and 30 s at 72°C, and a final extension at 72°C for 5 min. Bacterial 16S Amplicon libraries were sequenced using the dual index paired-end (v3, 2×300 bp) approach for the Illumina MiSeq platform as recommended by the manufacturer.16S amplicon sequences were filtered, quality controlled and taxonomically assigned using the LotuS pipeline (Hildebrand *et al.,* 2014) with default parameters using the

SILVA database (v138). Resulting abundance tables were normalized using the rarefaction toolkit (RTK) using default settings (Saary *et al.,* 2017). The data retrieved from the Lotus analysis pipeline were further processed using the R package metacoder (v. 0.3.5.001). OTUs with less than 50 reads were discarded and samples were rarefied using the function calc_obs_props. Differences between groups were calculated using the compare_groups function and the log2 median ratio was used to visualize trending differences in abundance between groups in taxonomic trees.

### Real Time PCR (qPCR):

qPCRs were carried out using a Quantstudio 3 (Applied Biosciences, CA, USA) thermal cycler in a reaction volume of 20µl Taqman fast universal master mix (Applied Biosciences) with Taqman gene expression assay for the butyrate associated bacterial gene Butyryl-CoA-Dehydrogenase (BCD). Primers SEQ-ID 10 and 13 and probe SEQ-ID 16 were used. 50ng of bacterial genomic DNA was used as template and all qPCR was carried out at 60°C in fast mode and primer and probe concentrations were set to 750nM and 250nM, respectively. Gene abundancy was determined relative to bacterial 16s rRNA gene.

### Targeted metabolomics - Determination of serum metabolite levels:

The analysis of plasma metabolites was focused on tryptophan (TRP) metabolites and short chain fatty acids (SCFA). For the TRP analysis, liquid chromatography - mass spectrometry (LC-MS) analysis was performed, SCFA were analyzed by gas chromatography (GC-MS).

### Aryl hydrocarbon receptor (AhR) activity assay:

As a method to detect AhR agonists we used a human cell line stably transfected with a reporter plasmid containing an AhR responsive promoter element (HT-29-AhR (InvivoGen, CA, USA)). The XRE is activated upon ligand binding to the AhR and drives the expression of the detectable reporter lucia luciferase. HT-29-AhR cell line (InvivoGen, CA, USA) was grown and maintained in Dulbecco's Modified Eagle Medium (DMEM, Sigma Aldrich) supplemented with 4.5g/l glucose (Sigma Aldrich), 2mM L-glutamine (Sigma Aldrich), 10% (v/v) heat-inactivated fetal bovine serum (Biochrom, Germany), 100 µg/ml Zeocin (Sigma Aldrich) and 100 ug/ml Penicillin-Streptomycin (Sigma Aldrich). For the AhR reporter assay cells at 80% confluency were harvested and resuspended at 2.8 × 10^5 cells/ml. 20µl of patient serum was mixed with 180µl of cell suspension in a white 96 well plate (Corning, NY, USA). The plate was incubated at 37°C with 5% CO2 for 48h. After 48h, 20µl of cell supernatant was taken from each well and the lucia luciferase was quantified by incubating with Quanti-luc reagent (InvivoGen) according to manufacturer's instructions. Assays were performed in triplicates and luciferase activity was quantified as relative luminescence units (RLU) using a microplate reader (Infinite 200 plate reader, Tecan, Switzerland). The luminescent signal representing ligand induced AhR activity from sera was normalized to the signal of non-inoculated media.

### Monocyte isolation and serum incubation:

PBMC were isolated from a healthy donors and monocytes were enriched using CD14 MicroBeads (Milteny Biotec, USA) according to manufacturer's protocol. On a 96 well plate 1x106 cells were plated and incubated for 24 hours at 37°C in standard culture medium containing 20% patient serum, IxS (IxS potassium salt, Sigma Aldrich, USA) at concentrations of 50µM and 125µM or the synthetic AhR antagonist (CH-223191 , Sigma Aldrich, USA) respectively. Afterwards, TNF-α was measured in the supernatant using a human TNF-α ELISA kit (Thermo Fisher, USA) according to manufacturer's protocol.

### Flow cytometry analysis:

PBMC were analysed by multi-colour flow cytometry. To avoid batch effects, all collected cells were thawed and measured at the same time. After thawing, cells were either stained immediately in FACS buffer or restimulated in a final volume of 200 µl RPMI 1640 (Sigma-Aldrich, USA)) supplemented with 10% FBS (Merck), 100 U/ml penicillin (Sigma-Aldrich, USA), 100 mg/ml streptomycin (Sigma-Aldrich, USA), 50 ng/ml PMA (Sigma-Aldrich, USA), 250 ng/ml ionomycin (Sigma-Aldrich, USA) and 1.3 µl/ml Golgistop (BD, USA). Cells were fixed and permeabilized (eBioscience^{™} Foxp3/Transcription Factor Staining Buffer Set, Thermo Fisher, USA) and labelled using monoclonal antibodies. Cells were analysed using a LSRFortessa flow cytometer and FACSDiva software (BD, USA). Data analysis was performed with FlowJo (LLC, USA). For FlowSOM analysis cells were manually gated (live single cells) on T cells (CD3+) and monocytic cells (CD3- CD4+) and exported for the respective analysis. This resulted in 1.1 - 6.2 x105 monocytic cells per sample and 1.4 - 6.6 x105 T cells per sample for further analysis. Markers used for FlowSOM analysis of the respective panel are shown in the heatmap (Figure 5c and 6b). Data were clustered onto a 10 node × 10 node square SOM as implemented in the CATALYST package v.1.14.1 (wrapper for FlowSOM). Lower resolution ConsensusClusterPlus metaclustering was then performed to reduce them to 8 metaclusters. Phenotypic relationship between individual clusters were explored using the UMAP algorithm for dimension reduction of 105 gated cells from all samples acquired, as implemented in the runDR, runUMAP functions from the CATALYST package (R version 4.0.3). Resource intensive computation has been performed on the HPC for Research cluster of the Berlin Institute of Health.

### Statistical analysis of microbiome and metabolome analysis:

Alpha diversities of microbial communities (Shannon diversity as computed by the RTK (0.93.1) tool, defined at the operational taxonomic unit (OTU) level) were compared between groups using Kruskal-Wallis (KW) test. Beta diversity was assessed using Euclidean (metabolome) and Canberra (microbiome) dissimilarity index between samples computed using vegan package v2.5-7. Principal Coordinates Analysis (PCoA) was performed using the vegan package v2.5-7 (employing Euclidean and Canberra distance metrics as above). PERMANOVA was performed using the adonis function from the vegan package v2.5-7. We estimate differential abundance on phylum and genus level between groups using the package DESeq2 v1.30.1. The DESeq2 pipeline uses negative binomial distribution models to test for differential abundance between testing conditions. We ran the pipeline with normalized counts under default settings. P values were adjusted according to Benjamini-Hochberg (BH) false discovery rates (FDR) correction. A q-value of <0.1 was considered statistical significant. Heatmaps visualization was performed in ggplot2 v3.3.5 and abundance plots were visualized using cowplot v1.1.1.

For each pair of patient groups, features from the tryptophan (TRP) analysis were compared using two-sided Mann-Whitney-U (MWU) tests with effect sizes calculated as Cliff's delta metric per the R ordom package v3.1. Effect sizes were taken as Spearman's rho. All significance estimates were adjusted for multiple tests using BH-FDR correction. To assess the effect of patient groups on tryptophan pathway metabolites (concentrations), multifactorANOVAs were calculated per metabolite to account for multiple groups as well as potential confounders (including age, sex, ethnical background, underlying disease category, BMI, and eGFR).

The co-abundance network of host, microbiome and metabolome features was calculated from the dataset as a whole by assessing pairwise Spearman correlations and adjusted for multiple testing using BH-FDR correction as implemented in the R psych package v1.9.12. Edges for which absolute rho > 0.3 and Q < 0.1 were visualized using the iGraph R package. Correlations were separately also assessed stratifying for group effects using the R coin package v1.3.1.

### Results and conclusion:

### Childhood CKD marked by arterial hypertension, inflammation and leaky aut:

Ten healthy individuals and 38 patients were enrolled in the study (Figure 20). Participant mean age was 10.6 ± 3.8 years. CAKUT was the most prevalent disease. Patients treated with HD had a median time on dialysis of six months (range 3-29 months) with a median residual diuresis of 50 ml per day (range 0 - 1800 ml per day) and a mean Kt/V of 1.56 ± 0.27 (SD), indicating adequacy of HD treatment. Patients after KT had a stable graft function with a mean eGFR of 78.8 ± 19.4 ml/min*1.73m2. The median time from transplantation was 48 months (range 10 - 125 months).

Both the CKD group (CKD stage G3-G4) and the HD group (CKD stage G5D) were hypertensive (83% and 91%, respectively) and received antihypertensive treatment (Figure 6A). Six HD patients (55%) exhibited hypertensive blood pressures despite treatment (Table 1). In the HD and CKD groups serum levels of the pro-inflammatory cytokine TNF-α were increased (Figure 6B), while differences in other cytokines did not reach significance. Serum levels of the tight junction protein Zo-1 and the lipopolysaccharide (LPS) binding protein sCD14 were investigated, which were elevated in CKD and HD compared to control and KT (Figure 6C), indicating stage-dependent intestinal barrier dysfunction and CKD-associated endotoxemia.

Thus, in the absence of classical cardiovascular risk factors other than hypertension, CKD in children is characterized by elevated serum markers of inflammation and leaky gut.

### Microbiome alterations in CKD:

Since intestinal barrier dysfunction may be associated with dysbiosis, next the taxonomic composition of the gut microbiome was analyzed by 16S sequencing. A high compositional variability at phylum level was observed (Figure 7A). Microbiome taxonomic richness and diversity showed no statistically significant changes (Figure 7B). However, analysis of beta diversity (Canberra distance) indicated significant differences in microbiome composition between groups, with CKD and HD groups separating most clearly from the HC (Figure 7C, p = 0.01). Analysis of bacterial composition on OTU level revealed significant alterations predominantly in HD patients (Figure 7D). First, relative abundances of Firmicutes and Actinobacteria such as OTU88 in the genus of Fusicatenibacter (belonging to the family of Lachnospiraceae), Subdoligranulum_OTU169 (Ruminococcaceae), and Bifidobacterium_OTU85 (Bifidobacteriaceae) were significantly diminished in HD patients compared to HC. Second, we found an increase in relative abundance of Proteobacteria, such as Citrobacter_OTU231 (Enterobacteriaceae) in HD and Parasutterella_OTU118 (Parasutterellaceae) in CKD patients, as well as several genera of Bacteroides (Bacteroidaceae) in CKD and HD groups compared to HC and KT. Analysis on phylum level showed significant changes in Tenericutes, Cyanobacteria and Actinobacteria, as visualized in Figure 16. In addition, HD patients tend towards higher abundances of Proteobacteria and lower Firmicutes abundances. We constructed a phylogenetic tree to better visualize phylogenetic classification and changes among the groups (Figure 17), again emphazising the CKD and HD-related reduction of OTUs from Lachnospiraceae and Ruminococcaceae and an increase in Enterobactericae in HD patients.

Taken together, the taxonomic microbiome changes in CKD are stage-dependent, most pronounced in the HD group and less pronounced after KT. The abundance of proteolytic bacteria (such as Citrobacter) increases in HD whereas saccharolytic bacteria (such as Bifidobacterium) decrease.

### CKD stage-dependent imbalance of bacterial metabolites:

To investigate the functional effects of the alterations in microbiome composition on host physiology, plasma metabolite analysis on TRP metabolites and SCFA was performed. Dietary TRP is a substrate for both cellular metabolism and bacterial proteolytic fermentation, the latter being a source of microbially-produced uremic toxins. We found significant differences in the abundance of TRP metabolites in CKD and HD compared to the HC and KT groups (Figure 8A). CKD and HD patients showed a large-scale shift from TRP to its indole and kynurenine (KYN) metabolites, which was predominantly driven by an increase of IxS (Figure 8). We found a significant decrease in plasma TRP concentrations in patients with CKD and HD, while IxS and 5 KYN metabolites (KYN, kynurenic acid (KA), 3-OH-kynurenine (3OH-KYN), anthranilic acid (AA), xanthurenic acid (XA)) were significantly elevated (Figure 8C). Individual values of TRP, IxS and, KA are shown across the different groups in Figure 8D-F. The activation of the cellular KYN pathway is also indicated by the ratio of KYN to TRP, which was significantly elevated in CKD and HD (Figure 8G). In a multivariate ANOVA only group and eGFR had an impact on several of the compounds, confirming their accumulation with declining eGFR. Because indole and KYN metabolites have been shown to activate the AhR, we measured the AhR activating potential of serum from the HD and HC group in vitro. Serum from HD patients induced a significantly higher AhR activity as compared to HC (Figure 8H). Similarly, IxS activates the AhR in a dose-dependent manner (Figure 18).

To further functionally validate the findings of decreased abundancies of saccharolytic microbes, the abundance of a central enzyme for bacterial SCFA production by qPCR in feces was measured. The abundance of Butyryl-CoA-Dehydrogenase was lower in HD patients relative to the other patient groups (Figure 13A). Thus, the observed stage-dependent taxonomic microbiome changes result in an altered production potential of certain bacterial metabolites. Next, serum SCFA levels were analyzed showing a reduction of acetate, propionate and isobutyrate, but not butyrate, in HD patients compared to HC (Figure 13B). In addition, trimethylamine-N-oxide (TMAO) as a well described toxin of microbial origin was measured accumulating in CKD and potentially driving CVD progression. TMAO was stage-dependently increased with highest levels in HD patients. Again, TMAO levels were almost at normal levels in the KT group. (Figure 15).

In order to comprehensively visualize the relationship of clinical, microbial and metabolomic parameters, we performed a correlation network analysis (Figure 9). Here, TNF-α correlated positively with sCD14, Proteobacteria, IxS, several KYN metabolites and biomarkers of kidney function (Figure 9A, positive correlations). Conversely, the SCFA propionate and isobutyrate correlated inversely with TNF-α, IxS, KYN metabolites and kidney function (Figure 9B, negative correlations). Firmicutes correlated positively with microbial diversity, butyrate and indole-3-propionic acid (I3PA). These associations support our assumption of kidney function as a catalyst of gut bacteria-driven inflammation.

### Monocyte subsets contribute to the pro-inflammatory phenotype in CKD:

The AhR is expressed in various immune cells including myeloid cells and is known to modulate their function. The accumulation of AhR ligands in CKD contributes to immune cell activation. Therefore, monocytes from healthy donors were isolated and incubated them with serum from HD patients or HC. Monocytes incubated with HD serum showed a significantly higher TNF-α production (Figure 10A). Similarly, incubation of isolated monocytes with IxS dose-dependently increased TNF-α production which could be reversed by co-incubation with the synthetic AhR-antagonist CH-223191, highlighting the importance of AhR-mediated immune activation in CKD (Figure 10A). In order to get a broader overview of changes in relevant immune cell populations in CKD, PBMC from seven HC and six HD patients were recollected for immunophenotyping by flow cytometry. Unsupervised FlowSOM analysis17 of monocyte and dendritic cell targeting flow panel (Figure 5B), showed phenotypic alterations of monocytes subtypes (cluster 3, Figure 10C) being decreased (Figure 5D) and dendritic cell (cluster 7, Figure 10C) being increased (Figure 10D) in HD patients. Using classical hierarchical gating, similar abundances of total monocytes (identified by HLA-DR, CD14, and CD1618) were observed, but a significant shift from classical (CD14+CD16-) towards non-classical (CD14-CD16+) and intermediate (CD14+CD16+) monocytes in HD (Figure 10E), the latter two being known for their potent production of TNF-α19. Hierarchical gating within the dendritic cell population revealed no significant differences of dendritic cell subtypes (Figure 12).

Thus, HD patients are characterized by alterations within myeloid cells, showing a shift from classical to pro-inflammatory intermediate and non-classical monocytes.

### Pro-inflammatory T cell subsets in CKD:

Since SCFA and TRP metabolites are known to modulate T cell differentiation and function, FlowSOM analysis with a T cell targeting panel was performed (Figure 11A). MAIT cells and a subpopulation of Treg (Clusters 6 and 3, respectively, Figure 11B) exhibited the largest effects between HD and HC patients (Figure 11C). This finding was confirmed by classical hierarchical gating, with circulating MAlT cells (CD3+CD161+TCRVa7.2+) being decreased in HD (Figure 14A). Next, a more detailed analysis of MAIT cell subpopulations was performed. After adjusting for multiple testing, a decrease of CD4-CD8- MAIT cells was found, while CD4+CD8- and CD4+CD8+ cells were enriched in HD (Figure 11D, F). Moreover, MAlT cells of HD patients displayed an effector memory phenotype (CD45RA-, CD62L-) and expressed more PD-1, indicative of increased activation. Importantly, upon in vitro restimulation, MAIT cells of HD patients secreted significantly more interleukin-17A (IL-17A) (Figure 11 D, F).

In addition, changes within a CD4+CD25+CD127- cell cluster (Cluster 3, Figure 11 A-C) were identified, indicating alterations in Treg subpopulations. Total Treg proportions were not altered between HC and HD (Figure 14B). However, since different subpopulations with partly different functions are known to exist within the total Treg population, the Treg characterization was extended. Adjusted for multiple testing, several Treg subpopulations were significantly changed in HD patients (Figure 11E). In detail, Treg from HD patients with a central-memory phenotype (CD45RA-, CD62L+) showed a higher expression of PD-1 indicating activation state. Th1-like (CXCR3+), Th17-like (CXCR3-CCR6+CCR4+CCR10-) and Th22-like (CXCR3-CCR6+CCR4+CCR10+) Treg were significantly diminished in HD patients compared to HC (Figure 11E, G).

Taken together, significant alterations in T cell subtypes, namely MAIT cells and Treg were observed, which play an important role in mucosal immunity and inflammation.

### Conclusion:

It is shown in children with CKD, that intestinal barrier dysfunction and dysbiosis are associated with a systemic bacterial metabolite imbalance that contributes to the pro-inflammatory phenotype of several immune cells including monocytes and T cells. Further insight is provided into the microbiota-immune crosstalk mediated by TRP metabolites and SCFA. Thus, a stage-dependent aberration of the microbiome-immune axis is demonstrated, which is both a contributor to inflammation and a potential target for anti-inflammatory therapeutic strategies.

Serum TNF-α and sCD14 were stage-dependently elevated, indicating inflammation, gut barrier dysfunction and endotoxemia. Compositional and functional alterations of the microbiome, including a diminished production of short chain fatty acids was observed in the patients. Plasma metabolite analysis revealed a stage-dependent increase of tryptophan metabolites of bacterial origin. Further, serum from HD patients activated the aryl hydrocarbon receptor and stimulated TNF-α production in monocytes, corresponding to a pro-inflammatory shift from classical to non-classical and intermediate monocytes. Unsupervised analysis of T cells revealed a loss of mucosa-associated invariant T (MAIT) cells and regulatory T cell subtypes in HD patients. The CKD specific dataset of gut microbiome composition, altered microbial metabolism of nutrients, and the corresponding impact on inflammation and immune cell dysregulation in children suffering from CKD shows dysbiosis-driven immunological changes highlighting the potential of microbiota-targeted therapies to improve prognosis of CKD patients across all ages. Overall, gut barrier dysfunction and microbial metabolite imbalance apparently mediate the pro-inflammatory immune phenotype, thereby driving the susceptibility to cardiovascular disease. Thus, the data highlight the importance of the microbiota-immune axis in CKD irrespective of confounding comorbidities.

In conclusion, the present invention shows for the first time CKD stage-dependent alterations of the microbiota-metabolite-immune axis in children with CKD (Figure 19). The data demonstrate alterations at all levels of this pivotal axis. In this context, TRP metabolites act as a mechanistic link between the microbiota and the immune system, contributing to a pro-inflammatory phenotype in an AhR-dependent manner- even at a young age. SCFA deficiency may further exacerbate CKD-associated chronic inflammation and intestinal barrier dysfunction. These data provide strong evidence that the microbiota is an important stimulus for persistent inflammation. Restoring intestinal eubiosis could favorably influence the pro-inflammatory sequelae. Therefore, the microbiota appears a promising target of future therapeutic strategies aimed at sustained containment of inflammation to prevent chronic sequelae such as CVD and premature mortality in CKD.

### References:

1. Gansevoort RT, Correa-Rotter R, Hemmelgarn BR, Jafar TH, Heerspink HJ, Mann JF, et al.: Chronic kidney disease and cardiovascular risk: epidemiology, mechanisms, and prevention. Lancet, 382: 339-352, 2013 10.1016/S0140-6736(13)60595-4
2. Claro LM, Moreno-Amaral AN, Gadotti AC, Dolenga CJ, Nakao LS, Azevedo MLV, et al.: The Impact of Uremic Toxicity Induced Inflammatory Response on the Cardiovascular Burden in Chronic Kidney Disease. Toxins (Basel), 10, 2018 1 0.3390/toxins1 01 00384
3. Onal EM, Afsar B, Covic A, Vaziri ND, Kanbay M: Gut microbiota and inflammation in chronic kidney disease and their roles in the development of cardiovascular disease. Hypertens Res, 42: 123-140, 2019 10.1038/s41440-018-0144-z
4. Vanholder R, Schepers E, Pletinck A, Nagler EV, Glorieux G: The uremic toxicity of indoxyl sulfate and p-cresyl sulfate: a systematic review. J Am Soc Nephrol, 25: 1897-1907, 2014 10.1681/ASN.2013101062
5. Vaziri ND, Wong J, Pahl M, Piceno YM, Yuan J, DeSantis TZ, et al.: Chronic kidney disease alters intestinal microbial flora. Kidney Int, 83: 308-315, 2013 10.1038/ki.2012.345
6. Poesen R, Ramezani A, Claes K, Augustijns P, Kuypers D, Barrows IR, et al.: Associations of Soluble CD14 and Endotoxin with Mortality, Cardiovascular Disease, and Progression of Kidney Disease among Patients with CKD. Clin J Am Soc Nephrol, 10: 1525-1533, 2015 10.2215/CJN.03100315
7. Ramezani A, Massy ZA, Meijers B, Evenepoel P, Vanholder R, Raj DS: Role of the Gut Microbiome in Uremia: A Potential Therapeutic Target. Am J Kidney Dis, 67: 483-498, 2016 10.1053/j.ajkd.2015.09.027
8. Wang X, Yang S, Li S, Zhao L, Hao Y, Qin J, et al.: Aberrant gut microbiota alters host metabolome and impacts renal failure in humans and rodents. Gut, 69: 2131-2142, 2020 10.1136/gutjnl-2019-319766
9. Holle J, Querfeld U, Kirchner M, Anninos A, Okun J, Thurn-Valsassina D, et al.: Indoxyl sulfate associates with cardiovascular phenotype in children with chronic kidney disease. Pediatr Nephrol, 2019 10.1007/s00467-019-04331-6
10. Harambat J, van Stralen KJ, Kim JJ, Tizard EJ: Epidemiology of chronic kidney disease in children. Pediatr Nephrol, 27: 363-373, 2012 10.1007/s00467-011-1939-1
11. Schwartz GJ, Munoz A, Schneider MF, Mak RH, Kaskel F, Warady BA, et al.: New equations to estimate GFR in children with CKD. J Am Soc Nephrol, 20: 629-637, 2009 10.1681/ASN.2008030287
12. Rosario AS, Kurth BM, Stolzenberg H, Ellert U, Neuhauser H: Body mass index percentiles for children and adolescents in Germany based on a nationally representative sample (KiGGS 2003-2006). Eur J Clin Nutr, 64: 341-349, 2010 10.1038/ejcn.2010.8
13. Lurbe E, Agabiti-Rosei E, Cruickshank JK, Dominiczak A, Erdine S, Hirth A, et al.: 2016 European Society of Hypertension guidelines for the management of high blood pressure in children and adolescents. J Hypertens, 34: 1887-1920, 2016 10.1097/HJH.0000000000001039
14. Tang WH, Wang Z, Kennedy DJ, Wu Y, Buffa JA, Agatisa-Boyle B, et al.: Gut microbiota-dependent trimethylamine N-oxide (TMAO) pathway contributes to both development of renal insufficiency and mortality risk in chronic kidney disease. Circ Res, 116: 448-455, 2015 10.1161/CIRCRESAHA.116.305360
15. Witkowski M, Weeks TL, Hazen SL: Gut Microbiota and Cardiovascular Disease. Circ Res, 127: 553-570, 2020 10.1161/CIRCRESAHA.120.316242
16. RothhammerV, Quintana FJ: The aryl hydrocarbon receptor: an environmental sensor integrating immune responses in health and disease. Nat Rev Immunol, 19: 184-197, 2019 10.1038/s41577-019-0125-8
17. Quintelier K, Couckuyt A, Emmaneel A, Aerts J, Saeys Y, Van Gassen S: Analyzing high-dimensional cytometry data using FlowSOM. Nat Protoc, 16: 3775-3801, 2021 10.1038/s41596-021-00550-0
18. Abeles RD, McPhail MJ, Sovnter D, Antoniades CG, Vergis N, Vijay GK, et al.: CD14, CD16 and HLA-DR reliably identifies human monocytes and their subsets in the context of pathologically reduced HLA-DR expression by CD14(hi) /CD16(neg) monocytes: Expansion of CD14(hi) /CD16(pos) and contraction of CD14(lo) /CD16(pos) monocytes in acute liver failure. Cytometry A, 81: 823-834, 2012 10.1002/cyto.a.22104
19. Wong KL, Tai JJ, Wong WC, Han H, Sem X, Yeap WH, et al.: Gene expression profiling reveals the defining features of the classical, intermediate, and nonclassical human monocyte subsets. Blood, 118: e16-31, 2011 10.1182/blood-2010-12-326355
20. Duhen T, Duhen R, Lanzavecchia A, Sallusto F, Campbell DJ: Functionally distinct subsets of human FOXP3+ Treg cells that phenotypically mirror effector Th cells. Blood, 119: 4430-4440, 2012 10.1182/blood-2011-11-392324
21. Mitsnefes MM: Cardiovascular disease in children with chronic kidney disease. J Am Soc Nephrol, 23: 578-585, 2012 10.1681/ASN.2011111115
22. Schaefer F, Doyon A, Azukaitis K, Bayazit A, Canpolat N, Duzova A, et al.: Cardiovascular Phenotypes in Children with CKD: The 4C Study. Clin J Am Soc Nephrol, 12: 19-28, 2017 10.2215/CJN.01090216
23. Jankowski J, Floege J, Fliser D, Bohm M, Marx N: Cardiovascular Disease in Chronic Kidney Disease: Pathophysiological Insights and Therapeutic Options. Circulation, 143: 1157-1172, 2021 10.1161/CIRCULATIONAHA.120.050686
24. Fasano A: Zonulin and its regulation of intestinal barrier function: the biological door to inflammation, autoimmunity, and cancer. Physiol Rev, 91: 151-175, 2011 10.1152/physrev.00003.2008
25. Yuan JH, Xie QS, Chen GC, Huang CL, Yu T, Chen QK, et al.: Impaired intestinal barrier function in type 2 diabetic patients measured by serum LPS, Zonulin, and IFABP. J Diabetes Complications, 35: 107766, 2021 10.1016/j.jdiacomp.2020.107766
26. Tajik N, Frech M, Schulz O, Schalter F, Lucas S, Azizov V, et al.: Targeting zonulin and intestinal epithelial barrier function to prevent onset of arthritis. Nat Commun, 11: 1995, 2020 10.1038/s41467-020-15831-7
27. Schutt C, Schilling T, Grunwald U, Schonfeld W, Kruger C: Endotoxin-neutralizing capacity of soluble CD14. Res Immunol, 143: 71-78, 1992 10.1016/0923-2494(92)80082-v
28. Zanoni I, Granucci F: Role of CD14 in host protection against infections and in metabolism regulation. Front Cell Infect Microbiol, 3: 32, 2013 10.3389/fcimb.2013.00032
29. Stanislawski MA, Lange LA, Raffield LM, Zakai NA, Meyer M, Ferrier K, et al.: Soluble CD14 Levels in the Jackson Heart Study: Associations With Cardiovascular Disease Risk and Genetic Variants. Arterioscler Thromb Vasc Biol, 41: e369-e378, 2021 10.1161/ATVBAHA.121.316035
30. Olson NC, Koh I, Reiner AP, Judd SE, Irvin MR, Howard G, et al.: Soluble CD14, Ischemic Stroke, and Coronary Heart Disease Risk in a Prospective Study: The REGARDS Cohort. J Am Heart Assoc, 9: e014241, 2020 10.1161/JAHA.119.014241
31. Hiippala K, Jouhten H, Ronkainen A, Hartikainen A, Kainulainen V, Jalanka J, et al.: The Potential of Gut Commensals in Reinforcing Intestinal Barrier Function and Alleviating Inflammation. Nutrients, 10, 2018 10.3390/nu10080988
32. Geirnaert A, Calatayud M, Grootaert C, Laukens D, Devriese S, Smagghe G, et al.: Butyrate-producing bacteria supplemented in vitro to Crohn's disease patient microbiota increased butyrate production and enhanced intestinal epithelial barrier integrity. Sci Rep, 7: 11450, 2017 10.1038/s41598-017-11734-8
33. Hobby GP, Karaduta O, Dusio GF, Singh M, Zybailov BL, Arthur JM: Chronic kidney disease and the gut microbiome. Am J Physiol Renal Physiol, 316: F1211-F1217, 2019 10.1152/ajprenal.00298.2018
34. Gryp T, Huys GRB, Joossens M, Van Biesen W, Glorieux G, Vaneechoutte M: Isolation and Quantification of Uremic Toxin Precursor-Generating Gut Bacteria in Chronic Kidney Disease Patients. Int J Mol Sci, 21, 2020 10.3390/ijms21061986
35. Crespo-Salgado J, Vehaskari VM, Stewart T, Ferris M, Zhang Q, Wang GD, et al.: Intestinal microbiota in pediatric patients with end stage renal disease: a Midwest Pediatric Nephrology Consortium study. Microbiome, 4, 2016 ARTN 50 10.1186/s40168-016-0195-9
36. Forslund K, Hildebrand F, Nielsen T, Falony G, Le Chatelier E, Sunagawa S, et al.: Disentangling type 2 diabetes and metformin treatment signatures in the human gut microbiota. Nature, 528: 262-266, 2015 10.1038/nature15766
37. Bouter KE, van Raalte DH, Groen AK, Nieuwdorp M: Role of the Gut Microbiome in the Pathogenesis of Obesity and Obesity-Related Metabolic Dysfunction. Gastroenterology, 152: 1671-1678, 2017 10.1053/j.gastro.2016.12.048
38. Chu H, Williams B, Schnabl B: Gut microbiota, fatty liver disease, and hepatocellular carcinoma. Liver Res, 2: 43-51, 2018 10.1016/j.livres.2017.11.005
39. Roager HM, Licht TR: Microbial tryptophan catabolites in health and disease. Nat Commun, 9: 3294, 2018 10.1038/s41467-018-05470-4
40. Dodd D, Spitzer MH, Van Treuren W, Merrill BD, Hryckowian AJ, Higginbottom SK, et al.: A gut bacterial pathway metabolizes aromatic amino acids into nine circulating metabolites. Nature, 551: 648-652, 2017 10.1038/nature24661
41. Vaziri ND, Yuan J, Norris K: Role of urea in intestinal barrier dysfunction and disruption of epithelial tight junction in chronic kidney disease. Am J Nephrol, 37: 1-6, 2013 10.1159/000345969
42. Poesen R, Viaene L, Verbeke K, Claes K, Bammens B, Sprangers B, et al.: Renal clearance and intestinal generation of p-cresyl sulfate and indoxyl sulfate in CKD. Clin J Am Soc Nephrol, 8: 1508-1514, 2013 10.2215/CJN.00300113
43. Dou L, Poitevin S, Sallee M, Addi T, Gondouin B, McKay N, et al.: Aryl hydrocarbon receptor is activated in patients and mice with chronic kidney disease. Kidney Int, 93: 986-999, 2018 10.1016/j.kint.2017.11.010
44. Duni A, Vartholomatos G, Balafa O, Ikonomou M, Tseke P, Lakkas L, et al.: The Association of Circulating CD14++CD16+ Monocytes, Natural Killer Cells and Regulatory T Cells Subpopulations With Phenotypes of Cardiovascular Disease in a Cohort of Peritoneal Dialysis Patients. Front Med (Lausanne), 8: 724316, 2021 10.3389/fmed.2021.724316
45. Heine GH, Ortiz A, Massy ZA, Lindholm B, Wiecek A, Martinez-Castelao A, et al.: Monocyte subpopulations and cardiovascular risk in chronic kidney disease. Nat Rev Nephrol, 8: 362-369, 2012 10.1038/nrneph.2012.41
46. Felizardo RJF, Watanabe IKM, Dardi P, Rossoni LV, Camara NOS: The interplay among gut microbiota, hypertension and kidney diseases: The role of short-chain fatty acids. Pharmacol Res, 141: 366-377, 2019 10.1016/j.phrs.2019.01.019
47. Bartolomaeus H, Balogh A, Yakoub M, Homann S, Marko L, Hoges S, et al.: Short-Chain Fatty Acid Propionate Protects From Hypertensive Cardiovascular Damage. Circulation, 139: 1407-1421, 2019 10.1161/CIRCULATIONAHA.118.036652
48. Hsu CN, Lu PC, Hou CY, Tain YL: Blood Pressure Abnormalities Associated with Gut Microbiota-Derived Short Chain Fatty Acids in Children with Congenital Anomalies of the Kidney and Urinary Tract. J Clin Med, 8, 2019 10.3390/jcm8081090
49. Arpaia N, Campbell C, Fan X, Dikiy S, van der Veeken J, deRoos P, et al.: Metabolites produced by commensal bacteria promote peripheral regulatory T-cell generation. Nature, 504: 451-455, 2013 10.1038/nature12726
50. Haghikia A, Zimmermann F, Schumann P, Jasina A, RoesslerJ, Schmidt D, et al.: Propionate attenuates atherosclerosis by immune-dependent regulation of intestinal cholesterol metabolism. Eur Heart J, 2021 10.1093/eurheartj/ehab644
51. Halim L, Romano M, McGregor R, Correa I, Pavlidis P, Grageda N, et al.: An Atlas of Human Regulatory T Helper-like Cells Reveals Features of Th2-like Tregs that Support a Tumorigenic Environment. Cell Rep, 20: 757-770, 2017 10.1016/j.celrep.2017.06.079
52. Saigusa R, Winkels H, Ley K: T cell subsets and functions in atherosclerosis. Nat Rev Cardiol, 17: 387-401, 2020 10.1038/s41569-020-0352-5
53. Lowther DE, Goods BA, Lucca LE, Lerner BA, Raddassi K, van Dijk D, et al.: PD-1 marks dysfunctional regulatory T cells in malignant gliomas. JCI Insight, 1, 2016 10.1172/jci.insight.85935
54. Toubal A, Kiaf B, Beaudoin L, Cagninacci L, Rhimi M, Fruchet B, et al.: Mucosal-associated invariant T cells promote inflammation and intestinal dysbiosis leading to metabolic dysfunction during obesity. Nat Commun, 11: 3755, 2020 10.1038/s41467-020-17307-0
55. Magalhaes I, Pingris K, Poitou C, Bessoles S, Venteclef N, Kiaf B, et al.: Mucosal-associated invariant T cell alterations in obese and type 2 diabetic patients. J Clin Invest, 125: 1752-1762, 2015 10.1172/JCI78941
56. Touch S, Assmann KE, Aron-Wisnewsky J, Marquet F, Rouault C, Fradet M, et al.: Mucosal-associated invariant T (MAIT) cells are depleted and prone to apoptosis in cardiometabolic disorders. FASEB J: fj201800052RR, 2018 10.1096/fj.201800052RR
57. Juno JA, Waruk JLM, Wragg KM, Mesa C, Lopez C, Bueti J, et al.: Mucosal-Associated Invariant T Cells Are Depleted and Exhibit Altered Chemokine Receptor Expression and Elevated Granulocyte Macrophage-Colony Stimulating Factor Production During End-Stage Renal Disease. Front Immunol, 9: 1076, 2018 10.3389/fimmu.2018.01076
58. Swarte JC, Douwes RM, Hu S, Vich Vila A, Eisenga MF, van Londen M, et al.: Characteristics and Dysbiosis of the Gut Microbiome in Renal Transplant Recipients. J Clin Med, 9, 2020 10.3390/jcm9020386
59. Wu H, Singer J, Kwan TK, Loh YW, Wang C, Tan J, et al.: Gut Microbial Metabolites Induce Donor-Specific Tolerance of Kidney Allografts through Induction of T Regulatory Cells by Short-Chain Fatty Acids. J Am Soc Nephrol, 2020 10.1681/ASN.2019080852
60. Hill NR, Fatoba ST, Oke JL, Hirst JA, O'Callaghan CA, Lasserson DS, et al. Global Prevalence of Chronic Kidney Disease - A Systematic Review and Meta-Analysis. PloS one. 2016;11(7):e0158765.
61. Jha V, Modi GK. Getting to know the enemy better-the global burden of chronic kidney disease. Kidney Int. 2018;94(3):462-4.
62. Victoria Pfann's dissertation "Characterization of a novel murine model of chronic kidney disease and its complications" (available online)
63. Velasquez MT, Centron P, Barrows I, et al. Gut Microbiota and Cardiovascular Uremic Toxicities. Toxins (Basel) 2018; 10.
64. Castillo-Rodriguez E, Fernandez-Prado R, Esteras R, et al. Impact of Altered Intestinal Microbiota on Chronic Kidney Disease Progression. Toxins (Basel) 2018; 10.
65. Beto JA, Schury KA, Bansal VK. Strategies to promote adherence to nutritional advice in patients with chronic kidney disease: a narrative review and commentary. Int J Nephrol Renovasc Dis. 2016;9:21-33.
66. Zeevi D, Korem T, Zmora N, Israeli D, Rothschild D, Weinberger A, et al. Personalized Nutrition by Prediction of Glycemic Responses. Cell. 2015;163(5):1079-94.
67. Zhao L, Zhang F, Ding X, Wu G, Lam YY, Wang X, et al. Gut bacteria selectively promoted by dietary fibers alleviate type 2 diabetes. Science. 2018;359(6380):1151-6.
68. Schlender J, Behrens F, McParland V, et al. Bacterial metabolites and cardiovascular risk in children with chronic kidney disease. Mol Cell Pediatr 2021; 8: 17.
69. McEntyre CJ, Lever M, Chambers ST, George PM, Slow S, Elmslie JL, et al. Variation of betaine, N,Ndimethylglycine, choline, glycerophosphorylcholine, taurine and trimethylamine-N-oxide in the plasma and urine of overweight people with type 2 diabetes over a two-year period. Ann Clin Biochem. 2015;52(Pt 3):352-60.
70. Daskova N, Heczkova M, Modos I, Videnska P, Splichalova P, Pelantova H, Kuzma M, Gojda J, Cahova M. Determination of Butyrate Synthesis Capacity in Gut Microbiota: Quantification of but Gene Abundance by qPCR in Fecal Samples. Biomolecules. 2021 Sep 2;11(9):1303. doi: 10.3390/biom11091303. PMID: 34572516; PMCID: PMC8469203
71. Schirmer, M., et al., Dynamics of metatranscription in the inflammatory bowel disease gut microbiome. Nat Microbiol, 2018. 3(3): p. 337-346.
72. Smith PM, Howitt MR, Panikov N, Michaud M, Gallini CA, Bohlooly YM, et al. The microbial metabolites, short-chain fatty acids, regulate colonic Treg cell homeostasis. Science. 2013;341 (6145):569-73.
73. Brown AJ, Goldsworthy SM, Barnes AA, Eilert MM, Tcheang L, Daniels D, et al. The Orphan G protein-coupled receptors GPR41 and GPR43 are activated by propionate and other short chain carboxylic acids. J Biol Chem. 2003;278(13):11312-9.
74. Baxter NT, Schmidt AW, Venkataraman A, Kim KS, Waldron C, Schmidt TM. Dynamics of Human Gut Microbiota and Short-Chain Fatty Acids in Response to Dietary Interventions with Three Fermentable Fibers. mBio. 2019;10(1).
75. Vaziri ND, Liu SM, Lau WL, Khazaeli M, Nazertehrani S, Farzaneh SH, et al. High amylose resistant starch diet ameliorates oxidative stress, inflammation, and progression of chronic kidney disease. PLoS One. 2014;9(12):e114881.
76. Jakobsdottir G, Xu J, Molin G, Ahrne S, Nyman M. High-fat diet reduces the formation of butyrate, but increases succinate, inflammation, liver fat and cholesterol in rats, while dietary fibre counteracts these effects. PLoS One. 2013;8(11):e80476.
77. Tang WH, Wang Z, Fan Y, Levison B, Hazen JE, Donahue LM, et al. Prognostic value of elevated levels of intestinal microbe-generated metabolite trimethylamine-N-oxide in patients with heart failure: refining the gut hypothesis. J Am Coll Cardiol. 2014;64(18):1908-14.
78. Koeth RA, Wang Z, Levison BS, Buffa JA, Org E, Sheehy BT, et al. Intestinal microbiota metabolism of Icarnitine, a nutrient in red meat, promotes atherosclerosis. Nature medicine. 2013;19(5):576-85.
79. Nakano T, Katsuki S, Chen M, Decano JL, Halu A, Lee LH, et al. Uremic Toxin Indoxyl Sulfate Promotes Proinflammatory Macrophage Activation Via the Interplay of OATP2B1 and DII4-Notch Signaling. Circulation. 2019;139(1):78-96.
80. Liang H, Dai Z, Liu N, Ji Y, Chen J, Zhang Y, et al. Dietary L-Tryptophan Modulates the Structural and Functional Composition of the Intestinal Microbiome in Weaned Piglets. Front Microbiol. 2018;9:1736.
81. Hai X, Landeras V, Dobre MA, DeOreo P, Meyer TW, Hostetter TH. Mechanism of Prominent Trimethylamine Oxide (TMAO) Accumulation in Hemodialysis Patients. PloS one. 2015;10(12):e0143731.
82. Lin CN, Wu IW, Huang YF, Peng SY, Huang YC, Ning HC. Measuring serum total and free indoxyl sulfate and pcresyl sulfate in chronic kidney disease using UPLC-MS/MS. J Food Drug Anal. 2019;27(2):502-9.
83. Marzocco S, Fazeli G, Di Micco L, Autore G, Adesso S, Dal Piaz F, et al. Supplementation of Short-Chain Fatty Acid, Sodium Propionate, in Patients on Maintenance Hemodialysis: Beneficial Effects on Inflammatory Parameters and Gut-Derived Uremic Toxins, A Pilot Study (PLAN Study). J Clin Med. 2018;7(10).
84. Anders HJ, Andersen K, Stecher B. The intestinal microbiota, a leaky gut, and abnormal immunity in kidney disease. Kidney Int. 2013;83(6):1010-6.
85. Reichardt N, Duncan SH, Young P, Belenguer A, McWilliam Leitch C, Scott KP, et al. Phylogenetic distribution of three pathways for propionate production within the human gut microbiota. ISME J. 2014;8(6):1323-35.
86. Devlin AS, Marcobal A, Dodd D, Nayfach S, Plummer N, Meyer T, et al. Modulation of a Circulating Uremic
87. Solute via Rational Genetic Manipulation of the Gut Microbiota. Cell host & microbe. 2016;20(6):709-15.
88. Altschul, S.F., Gish, W., Miller, W., Myers, E.W. & Lipman, D.J. (1990) "Basic local alignment search tool." J. Mol. Biol. 215:403-410
89. Klindworth A, Pruesse E, Schweer T, et al. Evaluation of general 16S ribosomal RNA gene PCR primers for classical and next-generation sequencing-based diversity studies. Nucleic Acids Res 2013; 41: e1.
90. Hildebrand F, Tadeo R, Voigt AY, et al. LotuS: an efficient and user-friendly OTU processing pipeline. Microbiome 2014; 2: 30.
91. Saary P, Forslund K, Bork P, et al. RTK: efficient rarefaction analysis of large datasets. Bioinformatics 2017; 33: 2594-2595.

## Claims

1. A method for determining a production capacity of multiple bacterial metabolites in a subject by quantifying an expression level and an abundance of each of multiple bacterial genes, said genes encoding enzymes associated with the production of said bacterial metabolites, comprising:
- Performing a quantitative polymerase chain reaction (qPCR) on a sample comprising commensal bacteria obtained from the subject, with primers that hybridize to nucleic acid molecules encoding said bacterial genes, and determining an abundancy of said bacterial genes, and
- Performing a quantitative reverse transcription polymerase chain reaction (qRT-PCR) on said sample, with primers that hybridize to said nucleic acid molecules encoding said bacterial genes, and determining expression levels of said bacterial genes.

2. The method according to the preceding claim, for use in indicating an individualized nutritional adjustment and/or therapy of the subject, for example when said expression and/or abundance levels deviate from a control value, for example a healthy subject and/or a population average.

3. The method according to the preceding claim, wherein deviation of gene expression and/or abundance from a control value indicates the presence and/or risk of a medical condition, for example an inflammatory and/or chronic disease, such as a cardiovascular disease including atherosclerosis, arterial hypertension, peripheral arterial occlusive disease (pAVK) and coronary heart disease (CHD), a chronic kidney disease, irritable bowel syndrome, an inflammatory bowel disease including Crohn's disease or ulcerative colitis, obesity, insulin resistance, obesity, diabetes type 2, an autoimmune disease including a rheumatic disease, multiple sclerosis and diabetes type 1, allergic asthma, non-alcoholic fatty liver disease (NAFLD/NASH), cancer, a mental health disorder including Alzheimer's disease, dementia, Parkinson, autism spectrum disorders, generalized anxiety disorder, depression and posttraumatic stress disorder (PTSD), and/or cancer and/or a skin disease including eczema and psoriasis.

4. The method according to claim 2, wherein the indicated nutritional adjustment comprises an adjustment in diet and/or administration of commensal bacteria.

5. The method according to any of the preceding claims, wherein the sample is a stool sample, fecal water, a biopsy sample (e.g., of the gastrointestinal tract and/or skin), interstitial fluid sample of an oral, respiratory and/or intestinal compartment, a mucosal swab and/or skin swab of the subject.

6. The method according to any of the preceding claims, comprising additionally a quantitative nucleic acid amplification reaction of a standard for gene expression and/or for gene abundancy, wherein the standard comprises amplification of a gene specific copy number standard and/or amplifying a 16s ribosomal RNA (16s rRNA) sequence as a quantitative control for the bacterial gene abundancy.

7. The method according to any of the preceding claims, wherein the primers are degenerate and hybridize to nucleic acid molecules from multiple bacteria with distinct coding sequences for the bacterial gene encoding an enzyme associated with the production of said bacterial metabolite.

8. The method according to any of the preceding claims, wherein the bacterial metabolite is a short chain fatty acid (SCFA) or bacterial indole derivative.

9. The method according to any of the preceding claims, wherein the bacterial metabolite is butyrate, propionate, trimethylamine-N-oxide (TMAO) and/or indoxyl sulphate (IS).

10. The method according to the preceding claim, wherein the bacterial gene comprises, butyrate-CoA-dehydrogenase *(Bcd),* methylmalonyl-CoA-mutase (*spcA*)*,* choline trimethylamine lyase (*cutC*)*,* carnitine monooxygenase (*cntA*)*,* tryptophanase enzyme (*tnaA*)*,* bile acid inducible operon (*baiCD*) and/or sulphite reductase (*dsrA*)*.*

11. The method according to any of the preceding claims, wherein:
- the primers hybridize with a region of a bacterial butyrate-CoA-dehydrogenase gene *(Bcd)* according to SEQ ID NO 1 to 7, and/or
- the method comprises primers according to SEQ ID NO 8 to 13, or sequences with an 80%, preferably 90% identity thereto, and optionally comprises a probe according to SEQ ID NO 14 to 16, or sequences with an 80%, preferably 90% identity thereto.

12. The method according to any of the preceding claims, wherein:
- the primers hybridize with a region of a methyl-malonyl-CoA-mutase (scpA) gene according to SEQ ID NO 17 to 23 and/or 26 to 28, and/or
- the method comprises primers according to SEQ ID NO 24, 25, 29 and/or 30 and SEQ ID NO 31, or sequences with an 80%, preferably 90% identity thereto, and optionally comprises a probe according to SEQ ID NO 31, or sequences with an 80%, preferably 90% identity thereto.

13. The method according to any of the preceding claims, wherein:
- the primers hybridize with a region of a bacterial choline trimethylamine lyase (*cutC*) gene according to SEQ ID NO 32, 35 to 39 and/or 51 to 54 and/or,
- the method comprises primers according to SEQ ID NO 33, 34, 40 to 50, and/or 55 to 61, or sequences with an 80%, preferably 90% identity thereto.

14. The method according to any of the preceding claims, wherein:
- the primers hybridize with a region of a bacterial carnitine monooxygenase (*cntA*) gene according to SEQ ID NO 62 to 64, and/or
- the method comprises primers according to SEQ ID NO 65 and/or 66, or sequences with an 80%, preferably 90% identity thereto.

15. The method according to any of the preceding claims, wherein:
- the primers hybridize with a region of a bacterial tryptophanase enzyme (*tnaA*) gene according to SEQ ID NO 67, 68 and/or 71 to 77, and/or
- the method comprises primers according to SEQ ID NO 69, 70, 78 and/or 79, or sequences with an 80%, preferably 90% identity thereto.

16. A kit for quantifying an expression level and an abundancy of one or more bacterial genes associated with the production of one or more bacterial metabolites according to any one of the preceding claims, comprising
- degenerate primers that hybridize to nucleic acid molecules encoding said bacterial genes associated with the production of said bacterial metabolites,
- one or more standards for said bacterial gene, for example for generating one or more copy number standard curves, and
- optionally one or more probes that hybridize with said nucleic acid molecule encoding said bacterial gene, and
- optionally one or more primers that hybridize with a nucleic acid molecule encoding a bacterial 16s RNA sequence
- optionally one or more reagents for preparing a sample and isolating nucleic acids from the sample, and
- optionally one or more reagents for performing a quantitative polymerase chain reaction (qPCR) and/or quantitative reverse transcription polymerase chain reaction (qRT-PCR),
wherein preferably the degenerated primers, probes and standards are provided in a dried form within a multi-well plate, preferably a 96-, 192 or 384-well plate.
